# NEUE EUROPÄISCHE PATENTSCHRIFT

(11) **EP 0 475 160 B2**
(45) Veröffentlichungstag und Bekanntmachung des Hinweises auf die Entscheidung über den Einspruch: **14.07.2004**
(45) Hinweis auf die Patenterteilung: 14.02.1996
(21) Anmeldenummer: 91114163.8
(22) Anmeldetag: 23.08.1991
(51) Int. Cl.: A61K 9/127

(54) **Präparat zur Wirkstoffapplikation in Kleinsttröpfchenform**
Preparation for drug application in minute droplet form
Préparation pour l'application d'un principe actif sous forme de gouttelettes miniscules

(30) Priorität: 24.08.1990 DE 4026833; 24.08.1990 DE 4026834; 06.03.1991 DE 4107153; 06.03.1991 DE 4107152
(43) Veröffentlichungstag der Anmeldung: 18.03.1992
(73) Patentinhaber: IDEA AG, 80807 Munich (DE)
(72) Erfinder: IDEA AG, 80807 Munich (DE)
(74) Vertreter: Maiwald, Walter, Dr. Dipl.-Chem.

(56) Entgegenhaltungen:
- EP-A- 0 102 324
- EP-A- 0 211 647
- EP-A- 0 220 797
- EP-A- 0 280 492
- WO-A-88/07362
- US-A- 4 937 078
- Journal of Controlled Release, Bd. 12, Nr. 1, März 1990, Amsterdam NL, Seiten 25-50; V.M. Knepp et al.: "Controlled drug release from a novel liposomal delivery system. ii. transdermal delivery characteristics"

## Beschreibung

Die Erfindung betrifft die Verwendung von Präparaten zum Transport von Wirkstoffen durch Permeabilitätsbarrieren in Form von, mit einer membranartigen Hülle aus einer oder wenigen Lagen amphiphiler Moleküle bzw. mit einer amphiphilen Trägersubstanz versehenen, Flüssigkeitströpfchen. Außerdem betrifft die Erfindung ein Verfahren zur Herstellung solcher Präparate, insbesondere zur nichtinvasiven Verabreichung von antidiabetischen Wirkstoffen, speziell von Insulin.

Die Anwendung von Wirkstoffen wird häufig durch Barrieren eingeschränkt, die zuwenig durchlässig für diese Wirkstoffe sind. Bedingt durch die Undurchdringlichkeit der Haut müssen zum Beispiel die meisten gängigen Therapeutika entweder peroral oder parenteral (i.v., i.m., i.p.) verabreicht werden. Intrapulmonale und intranasale Anwendung von Aerosolen, der Einsatz von Rektalzäpfchen, die Applikation von Schleimhautgelen, occularen Präparaten usw. lassen sich nur an bestimmten Stellen und nicht mit allen Wirkstoffen realisieren. Das Einbringen von Wirkstoffen in das pflanzliche Gewebe unterliegt aufgrund der kuticulären Wachsschichten noch stärkeren Beschränkungen.

Nichtinvasive Wirkstoffapplikationen durch Permeabilitätsbarrieren wären in vielen Fällen vorteilhaft. Bei Mensch und Tier würde beispielsweise eine perkutane Applikation der Agentien die verabreichten Wirkstoffe vor der Zersetzung im Gastrointestinaltrakt schützen und ggf. eine modifizierte Agensverteilung im Körper zur Folge haben; sie würde die Pharmakokinetik der Droge beeinflussen und sowohl häufige, als auch einfache, nichtinvasive Behandlung erlauben (Karzel K., Liedtke, R.K. (1989) Arzneim. Forsch./Drug Res. 39, 1487-1491). Bei Pflanzen könnte eine verbesserte Penetration durch oder in die Kuticula die erforderliche Wirkstoffkonzentration senken und die Umweltbelastung signifikant herabsetzen (Price, C.E. (1981) In: The plant cuticle (D.F. Cutler, K.L Alvin, C.E. Price, Hrsgb.), Academic, New York, pp 237-252).

Bestrebungen, die Hautdurchlässigkeit durch geeignete Maßnahmen zu beeinflussen, sind vielfach besprochen worden (siehe z.B. Karzel und Liedtke, op. cit.). Besonders erwähnenswert sind z.B. Jetinjektion (Siddiqui & Chien (1987) Crit. Rev. Ther. Drug. Carrier. Syst. 3, 195-208.), der Einsatz von elektrischen Feldern (Burnette & Ongpipattanakul (1987) J. Pharm. Sci. 76, 765-773 ) oder die Verwendung von chemischen Additiva, wie z.B. von Lösungsmitteln oder Tensiden. Eine lange Liste von Hilfsstoffen, die zwecks der Erhöhung von Penetration eines wasserlöslichen Wirkstoffs (Nolaxon) in die Haut getestet wurden, ist z.B. in der Arbeit von Aungst et al. (1986, Int. J. Pharm. 33, 225-234) enthalten. Diese Liste umfaßt nichtionische Substanzen (darunter langkettige Alkohole, Tenside, zwitterionische Phospholipide, usw), anionische Stoffe (besonders Fettsäuren), kationische langkettige Amine, Sulfoxide, sowie diverse Aminoderivate; auch amphothere Glycinate und Betaine sind angeführt. Trotz allem ist jedoch das Problem der Wirkstoffpenetration in die Haut bisher nicht - oder nicht befriedigend - gelöst worden.

Eine Übersicht der Maßnahmen, die zwecks Erhöhung der Wirkstoffpenetration durch die pflanzliche Kuticula eingesetzt werden, ist in der Arbeit von Price (1981, op.cit.) zusammengefaßt. Wenn chemische Penetrationsverstärker verwendet wurden, ist es bisher üblich gewesen, diese dem wirkstoffhaltigen Gemisch einfach hinzuzufügen; lediglich im Falle von menschlicher Haut wurden Additiva manchmal auch vorab, in Form einer organischen Lösung, aufgetragen. Diese Darbringungsform hing mit den bisher untersuchten und diskutierten Wirkungsprinzipien von Additiven zusammen: Im allgemeinen ging man davon aus, daß die verstärkte Agenspenetration einerseits auf der Aufweichung (Fluidisierung) der Haut basiert (Golden et al., (1987) J. Pharm. Sci. 76, 25-28). (Diese geht in der Regel mit einer Zerstörung der Hautoberfläche und ihren schützenden Barriereeigenschaften einher und ist folglich unerwünscht.) Andererseits wurde gezeigt, daß manche Wirkstoffe durch die Haut in Form von niedrigmolekularen Komplexen mit den Zusatzmolekülen permeiren (Green et al., (1988) Int. J. Pharm. 48, 103-111).

Von diesen Konzepten abweichende Vorschläge brachten bisher wenig Verbesserung. Der von mehreren Autoren theoretisch diskutierte perkutane Einsatz von Tragern auf Lipidbasis, den Liposomen (Patel, Bioch. Soc. Trans., 609th Meeting, 13, 513-517, 1985, Mezei, M. Top. Pharm. Sci. (Proc. 45th Int. Congr. Pharm. Sci.F.I.P.,) 345-58 Elsevier, Amsterdam, 1985) zielte hauptsächlich auf die Beeinflussung der Wirkstoffkinetik. Es war vom Einsatz von herkömmlichem Lipidvesikeln die Rede, die die Haut nicht oder extrem unvollkommen passieren, wie in dieser Anmeldung gezeigt ist. JP 61/271204 A2 [86/271204] griff die Verwendung von Liposomen im ähnlichen Sinne auf, durch Verwendung von Hydrochinon-Glucosidal als wirkstoffstabilitätserhöhende Maßnahme.

Die bisherigen Präparate für perkutane Applikation wurden zumeist occlusiv angewandt; im Falle von liposomenhaltigen Präparationen war das sogar die Regel. Solche Präparate enthielten dabei ausschließlich kleine oder lipophile Wirkstoffe, sowie einige hautfluidisierende Additiva.

So betrifft das Journal of Control Release, Band 12, Nr. 1, März 1990, Amsterdam NL, S. 25-30, die Freisetzung des Wirkstoffes Progesteron mittels Liposomen. In der EP-A 0 102 324 wird ein Verfahren zur Herstellung von unilameltaren Liposomen in wässriger Phase beschrieben, worin eine homogene Mischung eines ionischen Tensids und eines Lipids dispergiert werden. Die erhaltenen Liposomen können als Träger von Wirkstoffen unterschiedlichster Art zu therapeutischen Zwecken verwendet werden. Ferner wird in der WO-A 88/07362 eine lokale Applikation von Minoxidil in Spray- oder Salbenform beschrieben. Als Trägersysteme für diesen Wirkstoff lassen sich u.a. auch Liposomen verwenden. Die EP-A 0 280 492 beschreibt Liposomenzusammensetzungen, die einen Wirkstoff enthalten. Die beschriebenen Liposomen bestehen aus einer liposomalen Membran aus Phosphorlipiden und anionischen oberflächenaktiven Stoffen mit einem hohen Kraft-Punkt, d.h. in einer Konzentrationen oberhalb ihrer kritischen Mizellenbildungskonzentration. Die EP-A 0 220 797 beschreibt ein Verfahren zur Herstellung von Liposomen, die ein Phosphorlipid und ein hydrophiles nicht-ionisches oberflächenaktives Mittel umfassen. Die nach diesem Verfahren hergestellten Liposomen lassen sich beispielsweise in der Kosmetik zur Verbesserung der percutanen Absorption von Wirkstoffen verwenden. Die EP-A0 211 647 beschreibt ein Verfahren zur Herstellung von Liposomen. Die Liposomen enthalten Hydratisierungsmittel wie Arginin- oder Glutaminsäure und liposomenbildende Materialien. Die dabei erhaltenen Liposomen-Gele können durch Zusatz einer wässrigen Lösung hochstabile Liposomen mit einer hohen Trägerkapazität bilden. Ferner beschreibt die US-A 4 937 078 ein Verfahren zur Herstellung von Liposomen, die lokale anästhetische oder analgetische Wirkstoffe enthalten. Die erhaltenen Liposomen können auf die Haut aufgetragen werden und weisen gegenüber herkömmlichen Trägern, wie Salben, Cremes oder Lotionen, eine verbesserte anästhetische oder analgetische Wirkung auf. Alle diese vorgenannten Präparate weisen aber den Nachteil einer unzureichenden Permeationsfähigkeit für den Transport von Wirkstoffen durch Permeabilitätsbarrieren auf.

Sie gewährleisteten daher nur eine begrenzte Kontrolle über die pharmakokinetischen Eigenschaften der Formulierung. Als Verbesserung wurde in WO 87/1938 A1 vorgeschlagen, die wirkstoffbeladenen Lipidvesikel zusammen mit einem Gelbildner in Form von 'transdermal patches' zu verwenden. Die Wirkzeit konnte auf diese Weise verlänget die Penetrationsfähigket des Wirkstoffs jedoch kaum erhöht werden. Durch massiven Einsatz von penetrationförderndem Polyethylenglycol und Fettsäuren zusammen mit Lipidvesikeln gelang es Gesztes und Mezei (1988, Anesth. Analg. 67, 1079-1081) eine lokale Analgesie mit lidocainhaltigen Trägern zu erreichen, allerdings erst nach mehreren Stunden occlusiver Applikation und in geringem Maßstab.

Mit einer Spezialformulierung konnten wir die Ergebnisse von Gesztes und Mezei erstmalig dramatisch übertreffen. Diese Trägerformulierung enthielt filtrierte, detergenshaltige Lipidvesikel (Liposomen) mit einem deklarierten optimalen Lipid/Tensid Gehalt von 1-40/1, in der Praxis zumeist um 4/1.

Diese Ergebnisse waren die Grundlage der deutschen Patentanmeldung P 40 26 834.9-41, die auf die Patentanmeldung P 40 26 833.0-43 über die Liposomenherstellung Bezug nimmt.

Nun wurde überraschenderweise gefunden, daß alle solche Träger für eine Penetration in und durch die Permeabilitatsbarrieren geeignet sind, die sich durch besondere, in dieser Anmeldung beschriebene Eigenschaften auszeichnen. Die Hauptanforderung an solche Träger, im folgenden als Transfersomen bezeichnet, ist, daß sie genügend elastisch sind, um durch die Konstriktionen in der Barriere, z.B. in der Haut, durchdringen zu können. Für Transfersomen aus Phosphatidylcholin und Natriumcholat wird diese Bedingung erfüllt, wenn die Randspannung unterhalb von 10 Piconewton ist; ähnliche Werte gelten auch für andere verwandte Systeme. Wenn die Träger nach der Applikation selbst einen Gradienten aufbauen, werden sie besonders nützlich, da sie in diesem Fall zur spontanen Penetration der Permeabilitätsbarriere tendieren.

Es ist daher eine Aufgabe der Erfindung, die Verwendung von Präparationen für verschiedenste Wirkstoffe und andere Substanzen anzugeben, die deren schnellen und wirksamen Transport durch Barrieren und Konstriktionen gestatten.

Eine weitere Aufgabe besteht in der Verwendung von Präparationen zum Wirkstofftransport durch menschliche, tierische und pflanzliche Hautschichten, die eine verbesserte Verfügbarkeit des Wirkstoffes am Wirkungsort ergeben.

Es ist eine weitere Aufgabe der Erfindung, die Verwendung von Präparaten zur nichtinvasiven Verabreichung von antidiabetischen Wirkstoffen, besonders von Insulin, anzugeben, die eine verbesserte, therapeutisch ausreichende und reproduzierbare Wirkstoffapplikation ermöglichen.

Aufgabe der Erfindung ist es weiterhin, ein Verfahren zur Herstellung solcher Präparate anzugeben.

Zur Lösung dieser Aufgaben dienen die Merkmale der unabhängigen Ansprüche.

Vorteilhafte Ausgestaltungen sind in den Unteransprüchen angegeben.

Die erfindungsgemäß verwendbaren Transfersomen unterscheiden sich in mindestens drei Grundeigenschaften von den bisher beschriebenen Liposomen für die topische Anwendung und von sonstigen verwandten Trägern. Erstens können sie aus beliebigen Amphiphilen bestehen, einschließlich Ölen. Zweitens können sie auf beliebige Weise hergestellt werden: ihre Penetrationsfähigkeit ist nicht von der Präparationsmethode abhängig. Drittens: Die Penetrationsfähigkeit von bisher beschriebenen, für die Hautapplikationen optimierten Liposomen (cf. Patentanmeldung P 40 26 834.9-41), basiert auf einem optimalen Lipid/Tensid-Verhältnis im Bereich L/T= 1-40/1 . Von Transfersomen wird jedoch hauptsächlich eine bestimmte Elastizität verlangt, die eine ausreichende Permeationsfähihkeit vermittelt. Wenn diese Charakteristik der Träger durch den Einsatz von randaktiven Substanzen gewährleistet wird, kann die erforderliche Gesamtmenge des randaktiven Stoffes im System L/T-Werten unterhalb von 1/500 (im Falle von klassischen Tensiden unterhalb von 1/50 bis 1/100) entsprechen. Der Wirkungsbereich von Transfersomen sprengt somit die bisher bekannten Grenzen um mehrere Zehntausend Prozent

Transfersomen unterscheiden sich in mindestens zwei Grundsätzen von mizellenartigen Trägerformulierungen. Erstens sind sie in der Regel viel größer als die Mizellen und unterliegen daher anderen Diffusionsgesetzen. Zweitens - und noch viel wichtiger - enthalten die vergleichbaren Transfersomen typischerweise einen hydrophilen Kern (das Innere von Vesikeln), in den fast beliebige wasserlösliche Substanzen eingeschlossen und somit über die Permeationsbarriere transportiert werden können. Gleichzeitig sind die Transfersomen auch für den Transport von amphiphilen und lipophilen Substanzen geeignet.

Wenn die Träger nicht von sich aus ausreichend deformierbar sind und ihre Permeationsfähigkeit durch den Zusatz von randaktiven Stoffen erreicht werden soll, entspricht die Konzentration dieser Stoffe vorzugsweise 0.1 % bis 99 % der Menge, die für eine Solubilisierung der Träger erforderlich wäre. Häufig liegt das Optimum zweckmäßig und wirkstoffabhängig in einem Bereich zwischen 1 und 80 %, besonders häufig zwischen 10 und 60 % und ganz bevorzugt zwischen 20 und 50 Mol.-%.

Die verwendeten Transfersomen sind zum Wirkstofftransport durch fast beliebige Permeationshindernisse tauglich, z.B. für eine perkutane Medikamentenapplikation. Sie können wasserlösliche oder fettlösliche Agentien transportieren und erreichen je nach ihrer Zusammensetzung, Applikationsmenge und Form unterschiedliche Penetrationstiefen. Die Spezialeigenschaften, die einen Träger zum Transfersom machen, können sowohl von phospholipidhaltigen Vesikeln, als auch von anderen Amphiphilaggregaten erreicht werden.

In dieser Anmeldung wird erstmalig gezeigt, daß mittels Transfersomen ein Großteil von Wirkstoffmolekülen nich nur in die Barriere, z.B. in die Haut, sondern auch in die Tiefe getragen werden kann und dort systemisch aktiv ist. Transfersomen tragen z.B. Polypeptidmoleküle 1000-fach effizienter durch die Haut als das bisher mit Hilfe von permeationfördernden strukturfosen Stoffen möglich war. Mit Transfersomen eingebrachte Substanzen können im Menschen fast 100 % des maximal erreichbaren biologischen oder therapeutischen Potentials entfalten: ein Effekt, der bisher nur invasiv mit Injektionen erreicht wurde.

Überraschend wurde gefunden, daß durch den Einsatz dieser neuartigen Wirkstoffträger Antidiabetesmittel ohne Spritzen oder Begleitmaßnahmen durch die Haut in das Blut eingeschleust werden können. So erreichen z.B. regelmäßig mehr als 50%, häufig mehr als 90%, der perkutan applizierten Insulinmoleküle ihren Bestimmungsort im Körper, wenn sie mittels Transfersomen angebracht wurden. Insulinhaltige Transfersomen, die auf die Haut aufgetragen werden, können folglich erfolgreich das Spritzen von Insulinlösungen ersetzen.

Durch diese Erfindung wurde somit ein Weg gefunden für die einfache, nichtinvasive und vollkommen schmerzlose Therapie von Typ II Diabetes: Transfersomen können alleine oder in Kombination mit beliebigen Dosiergeräten zur problemlosen akuten und/oder chronischen Diabetesbehandlung eingesetzt werden.

Träger gemäß dieser Anmeldung können aus einer oder mehreren Substanzen bestehen. Am häufigsten verwendet man ein Gemisch von Grundsubstanz(en), einer oder mehreren randaktiven Substanzen und von Wirkstoffen. Die geeignetsten Grundsubstanzen sind Lipide und andere Amphiphile; bevorzugte randaktive Substanzen sind Tenside oder geeignete Lösungsmittel; diese können mit den Wirkstoffmoleküle in bestimmten Verhättnissen gemischt werden, die sowohl von der Wahl der Substanzen als auch von ihren absoluten Konzentrationen abhängig sind. Es kann vorkommen, daß eine oder mehrere Präparationskomponenten erst nachträglich (z.B. durch eine chemische oder biochemische Abwandlung ex tempore und/oder in situ) randaktiv werden.

Transfersomen öffnen somit einen eleganten, einheitlich und allgemein nützlichen Weg für den Transport von diversen Wirkstoffen über die Permeabilitätsbarrieren. Diese neuentdeckten Träger eignen sich für den Einsatz in Human- und Tiermedizin, Dermatologie, Kosmetik, Biologie, Biotechnologie, Agrartechnologie und anderen Gebieten.

Ein Transfersom umfaßt einen erfindungsgemäßen Träger, der sich durch seine Fähigkeit auszeichnet, unter der Wirkung eines Gradienten durch und/oder in Permeabilitätsbarrieren kommen bzw. diffundieren zu können und dabei Stoff zu transportieren.

Ein solcher (Wirkstoff)Träger entspricht vorzugsweise einem molekularen Homo- oder Heteroaggregat oder einem Polymer. Das Trägeraggregat setzt sich erfindungsgemäß aus mehreren bis vielen, gleichen oder unterschiedlichen Molekülen zusammen, diephysiko-chemisch, physikalisch, thermodynamisch, und häufig funktionell, eine Einheitbilden. Einige Beispiele solcher Aggregate sind Mizellen, Diskmizellen, Öltröpfchen (Nanoemulsionen), Nanopartikel, Vesikel oder 'partikuläre Emulsionen'. Aggregatteile können miteinander auch nichtkovalent verknüpft sein. Die optimale Tragergröße ist eine Funktion der Barrierecharakteristika. Sie hängt auch von der Polarität (Hydrophilie), Mobilität (Dynamik), und Ladung sowie von der Elastizität der Träger(oberfläche) ab. Ein Transfersom ist vorteilhaft zwischen 10 und 10 000 nm groß.

Für die dermatologischen Applikationen werden z.B. vorzugsweise als Träger Partikel oder Vesikel in der Größenordnung von 100-10000 nm, häufig von 100 bis 400 nm, besonders häufig von 100 bis 200 nm verwendet.

Für die Applikationen an Pflanzen werden zweckmäßig zumeist relativ kleine Träger, vorwiegend mit einem Durchmesser unter 500 nm eingesetzt.

### DEFINITIONEN

### LIPIDE

Ein Lipid im Sinne dieser Erfindung ist jede Substanz, die fettartige oder fettähnliche Eigenschaften besitzt. In der Regel besitzt es einen ausgedehnten apolaren Rest (die Kette, X) und zumeist auch einen wasserlöslichen, polaren, hydrophilen Teil, die Kopfgruppe (Y) und hat die Grundformel 1

X-Yₙ (1)

worin n größer oder gleich null ist. Lipide mit n=0 werden als apolare Lipide bezeichnet, Lipide mit n >= 1 polare Lipide genannt. In diesem Sinne können alle Amphiphile, wie zum Beispiel Glyceride, Glycerophospholipide, Glycerophosphinolipide, Glycerophosphonolipide, Sulfolipide, Sphingolipide, Isoprenoidlipide, Steroide, Sterine oder Sterole und kohlehydrathaltige Lipide, schlicht als Lipide bezeichnet werden.

Ein Phospholipid ist beispielsweise eine Verbindung der Formel 2 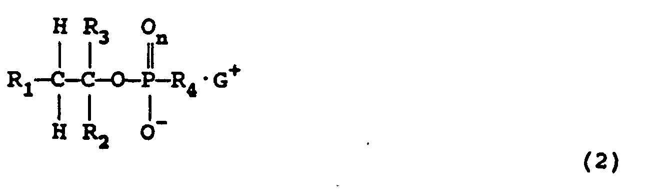 worin n und R₄ die unter Formel 8 genannten Bedeutungen haben, aber R₁, R₂ nicht Wasserstoff, OH oder kurzkettiger Alkylrest sein kann und R₃ meist Wasserstoff oder OH ist. R₄ ist außerdem durch Tri-kurzkettiges-Alkylammonio, z.B. Trimethylammonio, oder Amino substituiertes kurzkettiges Alkyl, z.B. 2-Trimethylammonioethyl (Cholinyl).

Ein Lipid ist vorzugsweise eine Substanz gemäß der Formel 2, worin n = eins, R₁ und R₂ Hydroxyacyl, R₃ Wasserstoff und R₄ 2-Trimethylammonioethyl (das letztere entspricht der Phosphatidylcholinkopfgruppe), 2-Dimethylammonioethyl, 2-Methylammonioethyl oder 2-Aminoethyl (entsprechend Phosphatidylethanolaminkopfgruppe) darstellen.

Ein solches Lipid ist z.B. ein natürliches Phosphatidylcholin - veraltet auch Lecithin genannt. Es kann z.B. gewonnen werden aus Ei (reich an Arachidonsäure), Sojabohne (reich an C-18 Ketten), Kokosnuß (reich an gesättigten Ketten), Oliven (reich an einfach ungesättigten Ketten), Safran (Saflor) und Sonnenblumen (reich an n-6 Linoleinsäure), Leinsamen (reich an n-3 Linolensäure), aus Walfett (reich an einfach ungesättigten n-3 Ketten), Nachtkerze oder Primel (reich an n-3 Ketten). Bevorzugte natürliche Phosphatidylethanolamine ( veraltet auch Kephaline genannt) stammen häufig aus Ei oder Sojabohnen.

Außerdem sind als Lipide synthetische Phosphatidylcholine (R₄ in der Formel 2 entspricht 2-Trimethylammonioethyl), synthetische Phosphatidylethanolamine (R₄ gleich 2-Aminoethyl), synthetische Phosphatidsäuren (R₄ ist ein Proton) oder ihre Ester (R₄ entspricht z.B. einem kurzkettigen Alkyl, wie Methyl oder Äthyl), synthetische Phosphatidylserine (R₄ gleich L- oder D-Serin), oder synthetische Phosphatidyl(poly)alkohole, wie z.B. Phosphatidylglycerol (R₄ gleicht L-oder D-Glycerol), bevorzugt, worin R₁ und R₂ identische Acyloxyreste, z.B. Lauroyl, Oleoyl, Linoyl, Linoleoyl oder Arachinoyl bedeuten, z.B. Dilauroyl-, Dimyristoyl-, Dipalmitoyl-, Distearoyl-, Diarachinoyl-, Dioleoyl-, Dilinoyl-, Dilinoleoyl-, oder Diarachinoylphosphatidylcholin oder -ethanolamin, oder verschiedene Acylreste, z.B. R₁ = Palmitoyl und R₄ = Oleoyl, z.B. 1-Palmitoyl-2-oleoyl-3-glycerophosphocholin; oder verschiedene Hydroxyacylreste, z.B. R₁ = Hydroxypalmitoyl und R₄ = Hydroxyoleoyl; oder Gemische davon, z.B. R₁ = Hydroxypalmitoyl und R₄ = Oleoyl usw. sind. Ferner kann R₁ Alkenyl und R₂ identische Hydroxyalkylreste bedeuten, wie z.B. Tetradecylhydroxy oder Hexadecylhydroxy, z.B. in Ditetradecyl- oder Dihexadecylphosphatidylcholin oder -ethanolamin, R₁ kann Alkenyl und R₂ Hydroxyacyl, z.B. ein Plasmalogen (R₄ Trimethylammonioethyl), oder R₁ ein Acyl z.B. Myristoyl oder Palmitoyl, und R₂ Hydroxy sein; so z.B. in natürlichen oder synthetischen Lysophosphatidylcholinen oder Lysophosphatidylglycerolen oder Lysophosphatidylethanolaminen, z.B. 1-Myristoyl- oder 1-Palmitoyllysophosphatidylcholin oder - phosphatidylethanolamin sein; R₃ stellt häufig Wasserstoff dar.

Ein geeignetes Lipid im Sinne dieser Erfindung ist auch ein Lipid der Formel 2, worin n = 1 ist, R₁ einen Alkenylrest, R2 einen Acylamidorest, R₃ Wasserstoff und R₄ 2-Trimethylammonioethyl (Cholinrest) darstellen. Ein solches Lipid ist unter dem Namen Sphingomyelin bekannt.

Ein geeignetes Lipid ist außerdem ein Lysophosphatidylcholin-Analog, z.B. 1-Lauroyl-1,3-propandiol-3-phosphorylcholin, ein Monoglycerid, z.B. Monoolein oder Monomyristin, ein Cerebrosid, ein Gangliosid oder ein Glycerid, welches keine freie oder veresterte Phosphoryl- oder Phosphonogruppe oder Phosphinogruppe in 3-Stellung enthält. Ein solches Glycerid ist beispielsweise ein Diacylglycerid oder 1-Alkenyl-1-hydroxy-2-acylglycerid mit beliebigen Acyl- bzw. Alkenylgruppen, worin die 3-Hydroxygruppe durch einen der genannten Kohlenhydratreste, z.B. einen Galactosylrest, verethert ist, wie z.B. in einem Monogalactosylglycerin.

Lipide mit erwünschten Kopf- oder Kettengruppen-Eigenschaften können auch auf biochemischem Wege, z.B. mittels Phospholipasen (wie Phospholipase A1, A2, B, C, und besonders D), Desaturasen, Elongasen, Acyl-Transferasen, usw. aus natürlichen oder synthetischen Prekursoren gebildet werden.

Ein geeignetes Lipid ist ferner ein jedes Lipid, welches in biologischen Membranen enthalten und mit Hilfe von apolaren organischen Lösungsmitteln, z.B. Chloroform, extrahierbar ist. Zu solchen Lipiden gehören außer der bereits erwähnten Lipide beispielsweise auch Steroide, z.B. Oestradiol, oder Sterine, z.B. Cholesterin, beta-Sitosterin, Desmosterin, 7-Keto-Cholesterin oder beta-Cholestanol, fettlösliche Vitamine, z.B. Retinoide, Vitamine, z.B. Vitamin A1 oder A2, Vitamin E, Vitmin K, z.B. Vitamin K1 oder K2 oder Vitamin D1 oder D3, usw.

### RANDAKTIVE SUBSTANZEN

Randaktive Substanz im Sinne dieser Anmeldung ist ein Stoff, der dem Trägersystem die Fähigkeit verleiht, oder diese Fähigkeit erhöht, Ränder, Ausläufer, oder relativ stark gekrümmte Flächen zu bilden; diese Eigenschaft manifestiert sich auch in der Fähigkeit, in einem höheren Konzentrationsbereich Poren in Lipidphasen, z.B. Membranen, zu bilden oder gar Solubilisierung (Lyse) zu bewirken. Im engeren Sinne handelt es sich dabei um Stoffe, die sich dadurch auszeichnen, daß sie sich an den Rändern zwischen den polaren und apolaren Molekülteilen und/oder an den Rändern zwischen den polaren und apolaren Teilen der supramolekularen Aggregate bevorzugt ansammeln und dadurch die freie Energie für die Bildung von Rändern oder stark gekrümmten Flächen herabsetzen. Alle Tenside ebenso wie viele Lösungsmittel sowie asymmetrische, und daher amphiphile, Moleküle oder Polymere, wie z.B. manche Oligo- und Poly-Kohlenhydrate, Oligo-und Polypeptide, Oligo- und Polynukleotide oder ihre Derivate gehören in diese Kategorie.

Die Randaktivität der verwendeten 'Lösungsmittel', Tenside, Lipide, oder Wirkstoffe hängt von der effektiven, relativen Hydrophilie/Hydrophobie des jeweiligen Moleküls ab, ist aber auch von der Wahl der sonstigen Systemkomponenten und Randbedingungen im System (Temperatur, Salzgehalt, pH-Wert, usw.) abhängig. Funktionelle Gruppen, z.B. Doppelbindungen im hydrophoben Rest, welche den hydrophoben Charakter dieses Restes abschwächen, erhöhen die Randaktivität; Verlängerung oder raumbeanspruchende Substituenten im hydrophoben Rest, z.B. in aromatischem Rest, erniedrigen die Randaktivität einer Substanz. Geladene oder stark polare Gruppen in der Kopfgruppe, bei gleichbleibender hydrophoben Kette, tragen normalerweise zu einer höheren Randaktivität der Moleküle bei. Direkte Bindungen zwischen den lipophilen und/oder amphiphilen Systemkomponenten haben eine entgegengesetzte Wirkung.

Zu den Lösungsmitteln, die lediglich in bestimmten Konzentrationsbereichen eine gewisse Randaktivität besitzen, gehören einfache, besonders kurzkettige, Alkohole wie z.B. Methanol, Ethanol, n-Propanol, 2-Propen-1-ol (Allylalkohol), n-Butanol, 2-Buten-1-ol, n-Pentanol (Amylalkohol), n-Hexanol, n-Heptanol, n-Octanol und n-Decanol, ferner iso-Propanol, iso-Butanol oder iso-Pentanol. Noch tauglicher sind die höheren Alkohole, wie z.B. Ethandiol (Ethylenglycol), 1,2-Propandiol (Propylenglycol), 1,3-Propandiol, 1,3-Butandiol, 2,3-Butandiol, Propantriol (Glycerol), 2-Buten-1,4-diol, 1,2,4-Butantriol, 1,3,4-Butantriol, 1,2,3-Butantriol, Butantetraol (Erythritol), 2,2-bis(Hydroxymethyl)1,3-propandiol (Pentaerythritol), 2,4-Pentadiol und andere Pentadiole oder Pentendiole, 1,2,5-Pentantriol und andere Pentantriole oder Pententriole, Pentantetraol, 1,2,6-Hexantriol und andere Hexantriole, Hexantetraole und -pentaole, Heptandiol, - triol, -tetraol, -pentaol und -hexaol, 1,4-Butandiol-diglycidyl-ether, usw. Auch kurzkettige, Di-, Tri-, Tetra-, Penta- und Hexa-Oxyethylenglycole und -Ethylenglycole gehören in diese Kategorie; außerdem cyclische Alkohole, wie z.B. Benzylalkohol, Cyclopentanol, Cyclohexanol, 3-, 4-, 5-Cyclohexanol, Cyclohexylalkohol, Aryl-alkohole, wie z.B. Phenyl-Ethanol, usw.

Randaktive Lösungsmittel, die erfindungsgemäß eingesetzt werden können, umfassen ferner Lösungen von kurzkettigen Acyl-,Alkyl-, Alkenyl, Hydroxyacyl-, Alkenyloxy- sowie Arylderivate von diversen Säuren und Basen, z.B. von Essig-, Ameisen- oder Propionsäure, Butensäure, Pentensäure, usw., von manchen Aminosäuren, von Benzoesäure, Phosphor- und Schwefelsäure, von Ammoniak, Purin, Pyrimidin, usw., insofern sie die chemische Integrität der Träger und Wirkstoffmoleküle nicht unannehmbar beeinträchtigen.

Eine nichtionische randaktive Substanz ist ein Stoff, der mindestens eine, zumeist jedoch mehrere, stark hydrophile Gruppe(n) enthält und mindestens einen, manchmals auch mehrere relativ hydrophobe(n), wasserunlösliche(n) Rest(e). 'Nichtionische' randaktive Substanzen können zwitterionisch oder nichtionisch sein.

Ladungsfrei und randaktiv sind z.B. die lipidähnlichen Stoffe mit der Grundformel 3

R₁ - ((Xᵢ - Yⱼ)ₖ - Zₗ)ₘ - R₂ (2)

worin X, Y und Z unterschiedliche polare (hydrophile) oder apolare (hydrophobe) Gruppen sind, die dem Gesamtmolekül einen amphiphilen Charakter verleihen. Z ist zumeist ein wasserlöslicher Rest und i, j, k, l und m sind größer oder gleich Null. R₁ und R₂ sind zwei beliebige Reste, der erste jedoch zumeist polar oder sehr kurzkettig, der zweite apolar.

Die Reste R₂ oder X in solchen Lipiden sind häufig eine Acyl-, Alkyl-, Alkenyl-, Hydroxyalkyl-, Hydroxyalkenyl- oder Hydroxyacyl-Kette mit 8-24 Kohlenstoffatomen. Besonders häufig werden n-Hexyl, n-Heptyl, n-Octyl, n-Nonyl, n-Decyl, n-Undecyl, n-Dodecyl, n-Tetradecyl oder n-Tetradecenoyl, n-Hexadecyl, n-Hexadecenoyl, n-Octadecyl, n-Octadecenoyl und n-Octadecendienyl, n-Octadecentrienyl, usw, verwendet.

Sorbitol ist ein möglicher Beispiel für den Rest Z. (Xᵢ - Yⱼ) kann z.B. ein Polyen, Polyoxyalken, wie z.B. Polyoxyethylen, Polyalkohol, z.B. Polyglycol, oder Polyether sein. (Xᵢ - Yⱼ) enthält vorzugsweise 1-20, besonders häufig 2-10 Einheiten, wie z.B. in Ethylenglycol, Di- und Triglycol (Oligoglycol) oder Polyethylenglycol.

Bei einfachen Substanzen gemäß Formel 3 ist der Rest R₁ oder R₂ häufig eine Alkyl-, Alkenyl-, Hydroxyalkyl-, Alkenylhydroxy- oder Hydroxyacyl-Kette mit 1-24 Kohlenstoffatomen. Sehr gut geeignet sind z.B. n-Dodecyl (Laurylether), n-Tetradecyl (Myristoyl-ether), n-Pentadecyl (Cetyl-ether), n-Hexadecyl (Palmitoyl-ether), n-Octadecyl (Stearoylether), n-Tetradecenoyl (Myristoleoyl-ether), n-Hexadecenoyl (Palmitoleoyl-ether) oder n-Octadecenoyl (Oleoyl-ether). Aufgrund ihrer guten Zugänglichkeit werden beispielsweise häufig verwendet: 4-Lauryl-Ether (Brij 30), 9-Lauryl-Ether, 10-Lauryl-Ether, 23-Lauryl-Ether (Brij 35), 2-Cetyl-Ether (Brij 52), 10-Cetyl-Ether (Brij 56), 20-Cetyl-Ether (Brij 58), 2-Stearyl-Ether (Brij 72), 10-Stearyl-Ether (Brij 76), 20-Stearyl-Ether (Brij 78), 21-Stearyl-Ether (Brij 721), 2-Oleoyl-Ether (Brij 92), 10-Oleoyl-Ether (Brij 96) und 20-Oleoyl-Ether (Brij 78), worin die steigende Anfangszahl auf die zunehmende Kopfgruppengröße hindeutet. Geeignete Substanzen sind unter den Bezeichnungen GENAPOL, THESIT und LUBROL im Handel erhältlich.

Zu den bekanntesten entsprechenden veresterten nichtionischen Tensiden gehören Substanzen mit dem Handelsnamen Myrj, wie z. B. Polyoxyethylen(8)-Stearat (Myrj45), Polyoxyethylen(20)-Stearat (Myrj49), Polyoxyethylen(30)-Stearat (Myrj51), Polyoxyethylen(40)-Stearat (Myrj52), Polyoxyethylen(50)-Stearat (Myrj53), Polyoxyethylen(100)-Stearat (Myrj59), usw. Weitere Produkte dieser Substanzklassen werden z.B. unter dem Handelsnamen Cirrasol ALN vertrieben; übliche Polyoxyethylen-Alkylamide sind z.B. Tenside mit dem Handelsnamen Atplus.

Bei einer weiteren wichtige Spezialform der nichtionischen randaktiven Substanz gemäß Strukturformel 3 ist der Rest R₁ zumeist eine Hydroxylgruppe, der Rest R₂ zumeist ein Wasserstoffatom. Die Reste X und Z sind häufig eine Alkoxy- oder Alkenoxy-, im Prinzip auch Hydroxyalkyl-, Hydroxyalkenyl- oder Hydroxyacyl-Kette mit 4-100 Kohlenstoffatomen. Auch der Rest Y ist häufig eine Alkoxy-, Alkenoxy-, Hydroxyalkyl-, Hydroxyalkenyl- oder Hydroxyacyl-Kette, die allerdings zumeist verzweigt ist und eine Methyl- bzw. Ethyl-Seitenkette trägt. Zu den verbreitesten randaktiven Substanzen dieser Klasse gehören die Tenside, die unter der Bezeichnung "Pluronic" im Handel erhältlich sind.

Weitere, häufig verwendete Spezialformen von nichtionischen randaktiven Substanzen sind unter der Bezeichnung "TWEEN" erhältlich. Sie haben als zyclischen Teil häufig einen Sorbitolring. Die Reste R₁, R₂, R₃ und R₄ sind häufig vom Alkoxy- oder Alkenoxy-, noch häufiger vom Polyen-, Polyoxyalken-, wie z.B. Polyoxyethylen-, Polyalkohol-, wie z.B. Polyglycol-, oder Polyether-Typ. Manche dieser Ketten können apolar sein, z.B. eine Acyl-, Alkyl-, Alkenyl-, Hydroxyalkyl, Hydroxyalkenyl- oder Hydroxyacyl-Kette mit 8-24 Kohlenstoffatomen. Wenn keiner der Reste R₁, R₂, R₃ oder und R₄ apolar ist, liegt ein hydrophober Rest als Seitenkette an einer verzweigten Kette oder als Terminalrest vor.

Besonders häufig treten in Substanzen vom TWEEN-Typus Polyoxyethylen-Ketten auf. Diese enthalten zumeist einen terminalen Wasserstoff, seltener eine Methoxy-Gruppe. Eine der Polyoxyethylen-Ketten ist jedoch mit einem hydrophoben Rest versehen, der vorzugsweise eine Acyl-, Alkyl-, Alkenyl-, Hydroxyalkyl-, Hydroxyalkenyl- oder Hydroxyacyl-Kette mit 4-24, insbesondere 12-18 Kohlenstoffatomen ist.

Auch randaktive Substanzen, die unter der Bezeichnung "TRITON" erhältlich sind, sind erfindungsgemäß verwendbar.

Polyalkoholreste R₂ sind vorzugsweise verestert oder verethert; sie können jedoch auch über ein Stickstoffatom an die hydrophobe Kette geknüpft sein. Sie sind sehr häufig Ethylenglycol-, Glycerol-, Erythritol- Pentaerythritol-Addukte, wie z.B. 1-Alkyl-, 1-Alkenoyl-, 1-Hydroxyalken-Glycerol, oder entsprechende 1,2-, oder 1,3-Diglyceride (z.B. 1-Alkyl,2-Alkyl-, 1-Alkyl,2-Alkenyl-, 1-Alkenyl,2-Alkyl-, 1-Alkenyl,2-Alkenyl-, 1-Alkenyl,2-Hydroxyalkyl-, 1-Hydroxyalkyl,2-Alkenyl-, 1-Alkyl,2-Hydroxyalkyl-, 1-Hydroxyalkyl,2-Alkyl-, 1-Alkenyl,2-Hydroxyalken-, 1-Hydroxyalken,3-Alkenyl-, 1-Alkyl,3-Alkyl, 1-Alkyl,3-Alkenyl-, 1-Alkenyl,3-Alkyl-, 1-Alkenyl,3-Alkenyl-, 1-Alkenyl,3-Hydroxyalkyl-, 1-Hydroxyalkyl,3-Alkenyl-, 1-Alkyl,3-Hydroxyalkyl-, 1-Hydroxyalkyl,3-Alkyl-, 1-Alkenyl,3-Hydroxyalken- oder 1-Hydroxyalken,3-Alkenyl-). An Stelle des Glycerols kann auch ein anderer höherwertigen Alkohol, z.B. Erythritol, Pentantriol, Hexantriol, -tetraol oder -pentaol, usw. auftreten, woraus sich eine Vielfalt an Verknüpfungsmöglichkeiten ergibt.

Z oder R₂ können ferner aus einem oder mehreren 1-10, vorzugsweise 1-6, ganz besonders häufig 1-3 Kohlenhydratresten oder ihren Derivaten bestehen. Die Bezeichnung Kohlenhydratrest hat dabei die bereits beschriebene Bedeutung und steht vorzugsweise für alpha oder beta und L- oder D-Allosid, -Altrosid, -Fucosid, -Furanosid, -Galactosid, -Galactopyranosid, -Glucosid, -Glucopyranosid, - Lactopyranosid, -Mannosid, -Mannopyranosid, -Psicosid, Sorbosid, -Tagatosid, -Talosid; häufig verwendete Derivate von Disacchariden sind L- oder D-Maltopyranosid, -Maltosid, Lactosid, - Malto-oder -Lactobionamid; auch entsprechende Derivate von Maltotriose oder -tetraose sind nützlich.

Der Kohlenhydrat-Rest kann außerdem schwefelhaltig sein, wie z.B. in beta-L- oder D-Thioglucopyranosid oder -Thioglycosid.

Zwitterionische Tenside sind z.B. sulfonathaltige Substanzen wie (3-((3-cholamidopropyl)-dimethylyammonio)-1-propansulfonat (CHAPS) und (3-((3-cholamidopropyl)-dimethylyammonio)-2-hydroxy-1-propansulfonat (CHAPSO) oder N-octyl-N,N-dimethyl-3-ammonio-1-propansulfonat, N-dodecyl-N,N-dimethyl-3-ammonio-1-propansulfonat (Lauryl-sulfobetain), N-tetradecyl-N,N-dimethyl-3-ammonio-1-propansulfonat (Myristyl-sulfobetain), N-hexadecyl-N,N-dimethyl-3-ammonio-1-propansulfonat (Palmityl-sulfobetain), N-octadecyl-N,N-dimethyl-3-ammonio-1-propansulfonat(Stearyl-sulfobetain), 'N-octadecenoyl-N,N,-dimethyl-3-ammonio-1-propansulfonat (Oleoyl-Sulfobetain) usw.

Zwitterionische Tenside sind ferner Substanzen mit der Formel 4 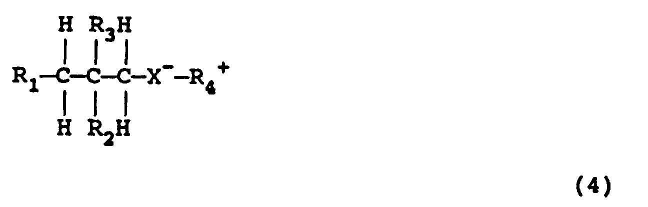 worin n eins oder null ist. Eine von beiden Seitenketten R₁ und R₂ enthält eine Acyl-, Alkyl-, Alkenyl-, Alkenoyl-, Hydroxyalkyl-, Hydroxyalkenyl- oder Hydroxyacyl-, bzw. Alkoxy-Kette mit je 8-24 Kohlenstoffatomen; die andere besteht aus Wasserstoff, Hydroxygruppe oder kurzkettigem Alkylrest. R₃ stellt normalerweise ein Wasserstoffatom oder eine kurze Alkylkette dar. X ist zumeist anionisch, z.B. ein Phosphat- oder Sulfat-Rest. Der Rest R₄ ist dann kationisch, um den zwitterionischen Charakter zu gewährleisten. Am häufigsten handelt es sich hierbei um gegebenenfalls substituierte Ammonio-alkylderivate, z.B. Ethanol-, Propanol-, Butanol-, Pentanolamin, Hexanolamin, Heptanolamin oder Octanolamin, N-Methyl-, N,N-Dimethyl, oder N,N,N-Trimethyl-ammonio-alkyl, N-Ethyl-, N,N-Diethyl, oder N,N,N-Triethyl-aminoalkyl, ungleiche N-Alkyle, z.B. N,N-Methyl-ethyl-ammonio-alkyl oder entsprechende Hydroxyalkylsubstanzen. (Einkettige (Lyso)-Derivate sämtlicher biologischer zwitterionischen Phospholipide sowie ihre Abwandlungen (z.B. Platelet-Activating-Factor und seine Analoga) gehören in diese Kategorie.). R₄ kann auch ein positiv geladener Kohlenhydratrest sein, z.B. ein Aminozucker oder seine Derivate. Die Positionen von R₄ und X können vertauscht sein.

Eine ionische randaktive Substanz ist ein Stoff, der zumindest eine positive oder negative Ladung trägt sowie mindestens einen wenig wasserlöslichen Rest. Eine anionische Substanz dieser Art kann auch mehrere Ladungen tragen, besitzt jedoch eine negative Gesamtladung; die Gesamtladung einer kationischen Substanz ist positiv.

Zu anionischen randaktiven Substanzen gehören Stoffe mit der Grundformel 5: 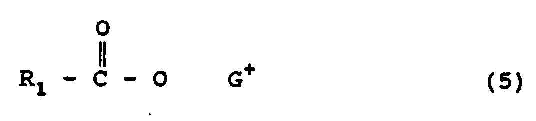 worin R₁ ein gegebenenfalls substituierter Kohlenwasserstoffrest ist und G⁺ ein einwertiges Gegenion darstellt, vorwiegend ein Alkalimetallkation (z.B. Lithium, Natrium, Kalium, Rubidium, oder Cäsium), ein Ammoniumion bzw. ein niedermolekulares Tetraalkylammonium-Ion, z.B. Tetramethylammonium oder Tetraethylammonium.

Der Kohlenwasserstoffrest R₁ in einem anionischen Tensid der Formel 5 ist zumeist ein geradkettiges oder verzweigtes Acyl, Alkyl oder Alkenoyl, bzw. oxidierte oder hydroxygenierte Derivate davon; der Rest R₁ kann auch cyclische Teile haben.

Die Kette R₁ enthält 6-24, sehr häufig 10-20, besonders häufig 12-18, Kohlenstoffatome; falls ungesättigt, enthält sie 1-6, besonders häufig 1-3, Doppelbindungen in n-3- oder n-6-Position.

Bevorzugte Hydroxyalkylketten sind in diesem Fall: n-Dodecylhydroxy (Hydroxylauryl), n-Tetradecylhydroxy (Hydroxymyristyl),n-Hexadecylhydroxy (Hydroxycetyl), n-Octadecylhydroxy (Hydroxystearyl), n-Eicosylhydroxy oder n-Docosyloxy. Von Hydroxyacylketten seien genannt Hydroxylauroyl, Hydroxymyristoyl, Hydroxypalmitoyl, Hydroxystearoyl, Eicosoylhydroxy oder Docosoyloxy-Ketten; von Hydroxyalken-Resten die Hydroxydodecen, Hydroxytetradecen, Hydroxyhexadecen, Hydroxyoctadecen, Hydroxyeicosen, Hydroxydocosen, ganz besonders häufig 9-cis,12-hydroxy-Octadecenyl (Ricinolenyl) oder 9-trans,12-hydroxy-Octadecenyl (Ricinelaidyl), 5-cis,8-cis,11-cis,14-cis,15-hydroxy-Eicosatetraenyl (15-hydroxy-Arachidonyl), 5-cis,8-cis,11-cis,14-cis,15-hydroxy,17-cis-Eicosapentaenyl, 4-cis,7-cis,10-cis,13-cis,15-hydroxy,16-cis-Docosapentaenyl und 4-cis,7-cis,10-cis,13-cis,15-hydroxy,16-cis,19-cis-Docosahexaenyl.

Ein weitere Klasse anionischer, randaktiven Substanz entspricht der Formel 6

(R₁ - (O - X) - Y)⁻ G⁺ ( 6 )

R₁ bedeutet hier einen gegebenenfalls substituierten Kohlenwasserstoffrest; X steht für einen kurzkettigen Alkylrest und Y kennzeichnet eine Sulfonat-, Sulfat-, Phosphat-, Phosphonat oder Phosphinatgruppe. G⁺ ist ein zumeist einwertiges Gegenion (Kation).

Durch eine Etherbindung verknüpft, und zu diesem Grundtypus gehörend, sind Alkalimetall-alkyl- oder -alkenylethersulfonate oder -phosphate. Beispiele dafür sind Natrium- oder Kalium-n-dodecyloxyethylsulfat, -n-tetradecyloxyethylsulfat, -n-hexadecyl-oxyethylsulfat oder -n-octadecyloxyethylsulfat oder ein Alkalimetall-alkansulfonat, z.B. Natrium-oder Kalium-n-hexansulfonat, n-octansulfonat, n-decansulfonat, n-dodecansulfonat, -n-tetradecansulfonat, -n-hexadecansulfonat oder -n-octadecan-sulfonat.

Verwandt mit den Verbindungen des Typs 6 sind die Substanzen der allgemeinen Formel 7

(R₁ - Y)⁻ G⁺ (7)

die analog zu den Substanzen der Formel 6 gebildet werden, jedoch durch direkte Bindung der geladenen Kopfgruppe an die Kette.

Besonders geeignete anionische, randaktive Substanzen der obigen Formel 6 sind Alkalimetall-alkylsulfate. Einige Beispiele solcher Substanzen sind: Natrium oder Kalium-n-Dodecyl (Lauryl)-sulfat, -n-Tetradecyl (Myristyl)-sulfat, -n-Hexadecyl (Palmityl)-sulfat, -n-Octadecyl (Stearyl)-sulfat, n-Hexadecylen(Palmitolein)-sulfat und n-Octadecylen(Olein)sulfat. Anstelle der Sulfatgruppe können z.B. auch Sulfonat, n-Methyl- oder n-Ethylglycin verwendet werden.

Ferner kommen die Salze der Bis-(2-alkyl-alkyl)-sulfosuccinate für eine Anwendung im Sinne dieser Anmeldung in Frage. Sie werden vorzugsweise als Lithium-, Natrium-, Kalium-, oder Tetramethylammonium-bis-(2-ethyl-hexyl)-sulfosuccinat verwendet.

Weitere geeignete Substanzen sind Sarkoside, Alkyl- oder Alkenoyl-Sulfochloridderivate der Eisweiskondensate, Sulfonamidseifen, sulfatierte oder phosphorylierte Alkoholester, sulfatierte oder phosphorylierte Amide bzw. Monoglyceride. Fettsäurenalkylamide, Sulfo- oder Phosphobernsteinsäureester, Tauride, Alkylphenol-, Alkylbenzol-, Alkylnapthalin-ethersulfonate usw.

Eine wichtige Gruppe anionischer randaktiven Substanzen sind die Derivate von Cholsäure. Ihre Grundformel ist 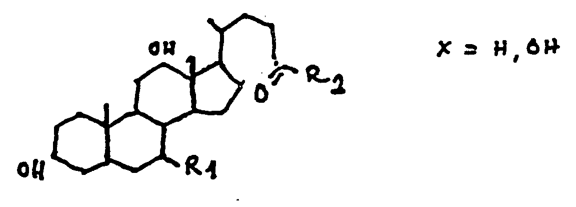 worin R₁ einem Proton, einer OH- oder oder einer Carbonylgruppe entspricht und R₂ beispielsweise Derivate von Taurin und Glycokoll kennzeichnet. Vorzugsweise werden Salze der Cholsäure (Gallensäure, 3alpha, 7alpha,12alpha-trihydroxy-5beta-Cholan-24-oin-säure), Deoxycholsäure (3alpha, 12alpha-dihydroxy-5beta-Cholan-24-oin-säure), Chenodeoxycholsäure, Glycocholsäure (N-(3alpha,7alpha,12alpha-trihydroxy-24-oxycholan-24-yl-)glycin), Deoxycholsäure, Glycodeoxycholsäure (N-(3alpha,12alpha-dihydroxy-24-oxycholan-24-yl-)glycin), Glycochenodeoxycholsäure, Glycolitocholsäure, Glycoursodeoxycholsäure, Litocholsäure, Taurodeoxycholsäure, Taurocholsäure (3alpha,7alpha,12alphatrihydroxy-5beta-Cholan-24-oin-säure-N-(sulfoethyl)amid), Taurochenodeoxycholsäure, Tauroglycocholsäure, Taurolitocholsäure, Taurolitocholsäure-3-Sulfat, Tauroursodeoxycholsäure, Ursocholansäure, Ursodeoxycholsäure (3alpha,7beta-dihydroxy-5beta-cholansäure), verwendet, wobei als Ion zumeist Natrium oder Kalium fungiert.

Des weiteren besitzen diverse Cholsäureester, wie z.B. Cholesteryl-Alkyl-, -Alkenyl-, -Hydroxyalkyl-, -Hydroxyalkenester oder Cholesterylsulfate und -sulfonate eine gewisse Randaktivität im Sinne dieser Erfindung.

Auch verwandte synthetische Addukte der CHAPS-Klasse sind verwendbar; hier ist R₂ häufig NH-(CH2)₃-N',N'-(CH₂)₂(CH₂)₂-R3-CH₂-SO₃, während R₃ ein Proton oder Carbonylgruppe sein kann. Am häufigsten treten auch hier Natrium oder Kalium als Gegenionen auf.

Digitonine sowie Saponine, z.B. Quillajasäure, haben im Kern eine ähnliche Struktur wie die Cholsäure-Derivate und kommen ebenfalls für eine Verwendung im Sinne dieser Erfindung in Frage.

Die summarische Formel für phosphorhaltige anionische randaktive Substanzen ist 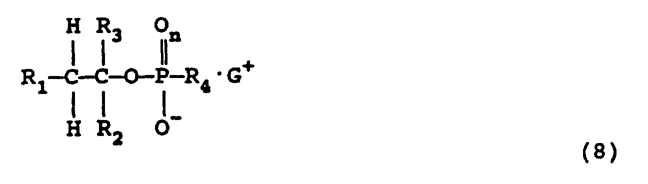 Der Wert von n ist null oder eins. Eine von beiden Seitenketten R₁ und R₂ besteht aus Wasserstoff, Hydroxygruppe oder kurzkettigem Alkylrest; die andere enthält eine Alkyl-, Alkenyl-, Hydroxyalkyl-, Hydroxyalkenyl- oder Hydroxyacyl-Kette (bzw. einen Alkenyl-, Alkoxy-, Alkenyloxy- oder Acyloxy-Rest) mit 8-24 Kohlenstoffatomen. Der Rest R₃ entspricht in der Regel Wasserstoff oder einer Alkyl-Kette mit weniger als 5 Kohlenstoffatomen. R₄ kann anionischer Sauerstoff oder eine Hydroxygruppe sein oder eine Alkylkette mit bis zu 8 C-Atomen; oder ein anderer Kohlenhydratrest mit bis zu 12 Kohlenstoffatomen; oder, wenn sowohl R₁ als auch R₂ Wasserstoff und/oder Hydroxygruppe sind, ein Steroidrest, ein Zuckerderivat, eine aminogruppenhaltige Kette, usw. Alkylreste können auch substituiert sein.

Zu den geeignetsten Tensiden dieser Substanzklassen gehören: n-Tetradecyl(=Myristoyl)-glycero-phosphatidsäure, n-Hexadecyl(=Plamityl)-glycero-phosphatidsäure, n-Octadecyl(=Stearyl)glycero-phosphatidsäure, n-Hexadecylen(=Palmitoleil)-glycero-phosphatidsäure, n-Octadecylen(=Oleil)-glycero-phosphatidsäure, n-Tetradecylglycero,phosphoglycerol, n-Hexadecyl-glycero-phosphoglycerol, n-Octadecylen-glycero-phosphoglycerol, n-Tetradecyl-glycero-phosphoserin, n-Hexadecyl-glycerophosphoserin, -n-Octadecyl-glycero-phosphoserin, n-Hexadecylen-glycero-phosphoserin und n-Octadecylen-glycero-phosphoserin.

Entsprechende Lyso-Sulfolipide, Phosphono- bzw. Phosphino-Lipide kommen auch für eine Anwendung im Sinne dieser Erfindung in Frage.

Als Gegenion tritt zumeist ein Alkalimetalkation (z.B. Lithium, Natrium, Kalium, Cäsium) oder ein wasserlösliches Tetraalkylammonium-Ion auf (z.B. Tetramethylammonium, Tetrathylammonium).

Für den Kohlenwasserstoffrest R1 gilt dasselbe, was bereits im Zusammenhang mit den Tensiden der Formel 3 gesagt wurde. Dieser Rest ist zumeist ein geradkettiges oder verzweigtes Alkyl oder Alkenoyl mit 6-24, sehr häufig 10-20, insbesondere 12-18, Kohlenstoffatomen und 1-6, besonders häufig 1-3, Doppelbindungen in n-3- oder n-6- Position.

Sehr gut geeignet als Alkyl-Reste R₁ oder R₂ sind zum Beispiel n-Dodecyl, n-Tetradecyl, n-Hexadecyl, n-Octadecyl, n-Eicosyl oder n-Docosyl-Ketten. In Frage kommen jedoch auch n-Nonyl, n-Undecyl, n-Tridecyl, n-Pentadecyl, n-Heptadecyl und n-Nonadecyl.

Alkenyl in Stellung R₁ oder R2 ist vorzugsweise ein 9-cis-Dodecenyl (Lauroleyl), 9-cis-Tetradecenyl (Myristoleyl), 9-cis-Hexadecenyl (Palmitoleoyl), 6-cis-Octadecenyl (Petroselinyl), 6-trans-Octadecenyl (Petroselaidinyl), 9-cis-Octadecenyl (Oleyl), 9-trans-Octadecenyl (Elaidinyl), 11-cis-Octadecenyl (Vaccenyl), 9-cis-Eicosenyl (Gadoleinyl), 13-cis-Docosenyl, 13-trans-Docosenyl oder 15-cis-Tetracosenyl.

Höhere, in Frage kommende ungesättigte Alkenyle sind: 9-cis,12-cis-Octadecendienyl, 9-trans,l2-trans-Octadecendienyl, 9-cis, 12-cis, 15-cis-Octadecentrienyl, 6-cis,9-cis, 12-cis-Octadecentrienyl, 11-cis, 14-cis, 17-cis-Eicosatrienyl, 6-cis,9-cis,12-cis,15-cis-Octadecentetraenyl, 5-cis,8-cis,1 1-cis, 14-cis-Eicosatetraenyl, 5-cis,8-cis, 11-cis, 14-cis, 17-cis-Eicosapentaenyl, 4-cis, 7-cis, 10-cis, 13-cis, 16-cis-Docosapentaenyl und 4-cis, 7-cis, 10-cis, 13-cis,16-cis, 19-cis-Docosahexaenyl.

Bevorzugte Beispiele für die Reste R₁ oder R₂ der Hydroxyalkyl-Klasse sind: n-Decylhydroxy, n-Dodecylhydroxy (Hydroxylauryl), n-Tetradecylhydroxy (Hydroxymyristyl), n-Hexadecylhydroxy (Hydroxycetyl), n-Octadecylhydroxy (Hydroxystearyl) und n-Eicosylhydroxy (Hydroxyarachinyl)-Ketten.

Alkenylhydroxy-R₁ oder R₂ ist vorzugsweise 9-cis-Dodecenylhydroxy (Hydroxylauroleyl), 9-cis-Tetradecenylhydroxy (Hydroxymyristoleyl), 9-cis-Hexa- decenylhydroxy (Hydroxypalmitoleinyl), 6-cis-Octadecenylhydroxy (Petroselinylhydroxy), 6-trans-Octadecenylhydroxy (Hydroxypetroselaidinyl), 9-cis-Octadecenylhydroxy (Hydroxyoleyl), 9-trans-Octadecenylhydroxy (Hydroxyelaidinyl) und 9-cis-Eicosenyl (Hydroxygadoleinyl).

Alkanoylhydroxy-R₁ oder R₂ ist vorzugsweise n-Decanoylhydroxy, n-Dodecanoythydroxy (Lauroylhydroxy), n-Tetradecanoylhydroxy (Myristoylhydroxy), n-Hexadecanoylhydroxy, n-Hexadecanoylhydroxy (Palmitoylhydroxy), n-Octadecanoylhydroxy (Stearoylhydroxy) und n-Eicosoylhydroxy (Arachinoylhydroxy).

Alkenoylhydroxy-R₁ oder R₂ ist vorzugsweise 9-cis-Dodecenylhydroxy (Lauroleoylhydroxy), 9-cis-Tetradecenoylhydroxy (Myristoleoylhydroxy), 9-cis-Hexadecenoylhydroxy (Palmitoleinoylhydroxy), 6-cis-Octadecenoylhydroxy (Peteroselinoylhydroxy), 6-trans-Octadecenoylhydroxy (Petroselaidinoylhydroxy), 9-cis-Octadecenoylhydroxy (Oleoylhydroxy), 9-trans-Octadecenoylhydroxy (Elaidinoylhydroxy) und 9-cis-Eicosenoyl (Gadoleinoylhydroxy).

Beispiele für den kurzkettigen Alkylrest, der meistens als Rest R₄ auftritt, sind Methylen-, Ethylen-, n-Propylen-, iso-Propylen-, n-Butylen- oder iso-Butylen- sowie n-Pentylen- oder n-Hexylen-Gruppen. Als Rest R₄ können auch z.B. Carboxy- oder Sulfo-Gruppen, saure und basische Gruppen, z.B. Carboxy- und Amino-Gruppen, fungieren; die Aminogruppe steht in einem solchen Fall stets in alpha-Stellung, bezogen auf die Carboxygruppe. Ein weiterer Beispiel für den R₄-Rest sind freie oder veretherte Hydroxygruppen (zwei veretherte Hydroxygruppen können dabei durch einen divalenten Kohlenwasserstoffrest, wie z.B. Methylen, Ethylen, Ethyliden, 1,2-Propylen oder 2,2- Propylen, miteinander verbunden sein). Der Rest R₄ kann ferner durch Halogen, z.B. Chlor oder Brom, Niederalkoxycarbonyl, z.B. Methoxy- oder Ethoxycarbonyl, oder durch Niederalkansulfonyl, z.B. Methansulfonyl, substituiert sein.

Substituiertes kurzkettiges Alkyl-R₄ mit 1-7 C-Atomen ist vorzugsweise Carboxy-kurzkettiges Alkyl, z.B. Carboxymethyl, Carboxyethyl- oder 3-Carboxy-n-propyl, omega-Amino-m-carboxy-kurzkettiges Alkyl, z.B. 2-Amino-2- carboxyethyl oder 3-Amino-3-carboxy-n-propyl, Hydroxy-kurzkettiges Alkyl, z.B. 2-Hydroxyethyl oder 2,3-Dihydroxypropyl, Niederalkoxynieder- 3-Methoxy-n-propyl, kurzkettiges Alkylendioxy-kurzkettiges Alkyl, z.B. 2,3- Ethylendioxypropyl oder 2,3-(2,2-Propylen)-dioxypropyl, oder Halogen-kurzkettiges Alkyl, z.B. Chlor- oder Brommethyl, 2-Chlor- oder 2-Bromethyl, 2- oder 3-Chlor- oder 2-oder 3-Brom-n-propyl.

Ein Kohlenhydratrest-R₄ mit 5-12 C-Atomen ist beispielsweise ein natürlicher Monosaccharidrest, der sich von einer als Aldose oder Ketose vorliegenden Pentose oder Hexose ableitet.

Ein Kohlenhydratrest-R₄ ist ferner ein natürlicher Disaccharidrest, z.B. ein Disaccharidrest, der sich aus zwei Hexosen, wie bereits beschrieben, gebildet hat. Außerdem kann ein Kohlenhydratrest R₄ ein derivatisierter Mono-, Di- oder Oligosaccharidrest sein, in dem beispielsweise die Aldehydgruppe und/oder ein oder zwei endständige Hydroxygruppen zu Carboxygruppen oxydiert sind, z.B. ein D-Glucon-, D-Glucar- oder D-Glucoronsäurerest, welcher vorzugsweise als cyklischer Lactonreste vorliegt. Ebenso können in einem derivatisierten Mono- oder Disaccharidrest Aldehyd- oder Ketogruppen zu Hydroxygruppen reduziert sein, z.B. Inosit, Sorbit oder D-Mannit, oder Hydroxygruppen durch Wasserstoff, z.B. Desoxyzucker, z.B. 2-Desoxy-D-ribose, L-Rhamnose oder L-Fucose, oder durch Aminogruppen, z.B. Aminozucker, z.B. D-Glucosamin oder D-Galactosamin, ersetzt sein.

R₄ kann auch ein Steroidrest oder Sterinrest sein. Wenn R₄ einen Steroidrest darstellt, ist R₃ Wasserstoff, während R₁ und R₂ vorzugsweise einer Hydroxygruppe entsprechen.

Das Gegenion ist vorzugsweise Ammonium, Natrium oder Kalium.

In einem anionischen Tensid der Formel 8 ist vorzugsweise n = 1, R₁ Alkyl, z.B. n-Dodecyl (Lauryl), n-Tridecyl, n-Tetradecyl (Myristyl), n-Pentadecyl, n-Hexadecyl (Cetyl), n-Heptadecyl oder n-Octadecyl (Stearyl), Hydroxyalkyl, z.B. n-Dodecylhydroxy (Hydroxylauryl), n-Tetradecylhydroxy (Hydroxymyristyl), n-Hexadecylhydroxy (Hydroxycetyl), oder n-Octadecylhydroxy (Hydroxystearyl), Hydroxyacyl, z.B. Hydroxylauroyl, Hydroxymyristoyl, Hydroxypalmitoyl oder Hydroxystearoyl, R₂ Wasserstoff oder Hydroxy, R₃ Wasserstoff oder kurzkettiges Alkyl, z.B. Methyl, R₄ kurzkettiges Alkyl, z. B. Methyl oder Ethyl, kurzkettiges Alkyl substituiert durch saure und basische Gruppen, z.B. Carboxy und Amino, z.B. omega-Amino-omega-carboxy-kurzkettiges Alkyl, z.B. 2-Amino-2-carboxyethyl oder 3-Amino-3-carboxy-n-propyl, Hydroxy-kurzkettiges Alkyl, z.B. 2-Hydroxyethyl oder 2,3-Hydroxypropyl, kurzkettiges Alkylendioxy-kurzkettiges Alkyl, z.B. 2,3-Ethylendioxypropyl oder 2,3-(2,2-Propylen)-dioxypropyl, Halogen-kurzkettiges Alkyl, z.B. 2-Chlor- oder 2-Bromethyl, ein Kohlenhydratrest mit 5-12 C-Atomen, z.B. Inosit, oder ein Steroidrest, z.B. ein Sterin, z.B. Cholesterin, und G⁺ = Natrium-, Kalium- oder Ammonium-Ion.

Ein anionisches Tensid der Formel 8 ist in erster Linie das Natrium-oder Kaliumsalz des Lysophosphatidylserins, z.B. das Natrium- oder Kaliumsalz des Lysophosphatidylserins aus dem Rinderhirn oder das Natrium-oder Kaliumsalz eines synthetischen Lysophosphatidylserins, z.B. Natrium-oder Kalium-1-myristoyl- oder -1-palmitoyllysophosphatidylserin, oder das Natrium- oder Kaliumsalz des Lysophosphatidylglycerins. Das Wasserstoffatom an der Phosphatgruppe kann durch ein zweites Kation G⁺ oder das Calcium-, Magnesium-, Mangan-Ion, usw. ersetzt sein.

In einem anionischen Tensid der Formel 8 ist vorzugsweise R₁ Alkyl, z.B. n-Dodecyl (Lauryl), n-Tridecyl, n-Tetradecyl (Myristoyl), n-Pentacedyl, n-Hexadecyl (Cetyl), n-Heptadecyl oder n-Octadecyl (Stearyl), Hydroxyalkyl, z.B. n-Dodecylhydroxy (Hydroxylauryl), n-Tetradecylhydroxy (Hydroxymyristyl), n-Hexadecylhydroxy (Hydroxycetyl), oder n-Octadecylhydroxy (Hydroxystearyl), Hydroxyacyl, z.B. Hydroxylauroyl, Hydroxymyristoyl, Hydroxypalmitoyl oder Hydroxystearoyl, R₂ Wasserstoff oder Hydroxy und R₃ Wasserstoff oder kurzkettiges Alkyl, z.B. Methyl. G+ ist vorzugsweise Ammonium, Natrium, Kalium oder Tetramethylammonium.

Ein anionisches Tensid der Formel 8 ist ferner das Natrium- oder Kaliumsalz einer natürlichen Phosphatidsäure, z.B. Ei-Phosphatidsäure, das Natrium- oder Kaliumsalz einer natürlichen Lysophosphatidsäure, z.B. Ei-Lysophosphatidsäure, das Natrium- oder Kaliumsalz einer synthetischen Lysophosphatidsäure, z.B. 1-Lauroyl-, 1-Myristoyl-, 1-Palmitoyl- und 1-Oleoyl-Lysophosphatidsäure.

Zu den wichtigsten Klassen von kationischen Tensiden gehören: Ammoniumsalze, quartäre Ammoniumsalze, Salze von heterozyklischen Basen, wie z.B. Aklkylpyridium-, Imidazol-, oder Imidazolinium-Salze, Salze von Alkylamiden und Polyaminen, Salze von acylierten Diaminen und Polyaminen, Salze von acylierten Alkanolaminen, Salze der Ester und Ether von Alkanolaminen, usw.

Ein kationisches Tensid ist beispielsweise eine Verbindung der Formel 9 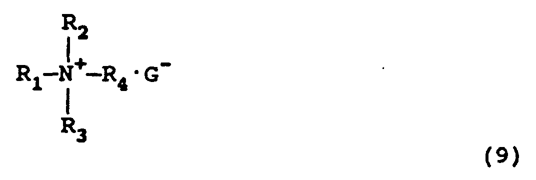 worin R₁ einen gegebenenfalls substituierten Kohlenwasserstoffrest kennzeichnet. R₂ steht für ein kurzkettiges Alkyl, Phenyl-kurzkettiges-Alkyl oder Wasserstoff. R₃ und R₄ bedeuten jeweils einen kurzkettigen Alkylrest. R₂ und R₃ zusammen mit dem Stickstoffatom stellen einen gegebenenfalls an einem Kohlenstoffatom substituierten, aliphatischen Heterocyclus und R₄ ein kurzkettiges Alkyl dar; R₂, R₃ und R₄ zusammen mit dem Stickstoffatom können auch einen gegebenenfalls an einem Kohlenstoffatom substituierten, aromatischen Heterocyclus bilden. G⁻ entspricht einem Anion.

In einem kationischen Tensid der Formel 9 ist ein gegebenenfalls substituierter, aliphatischer Kohlenwasserstoffrest R₁ beispielsweise durch Aryloxy-kurzkettiges-alkoxy- substituiertes kurzkettiges Alkyl, geradkettiges oder verzweigtes Alkyl mit 7-22, insbesondere 12-20, Kohlenstoffatomen, oder Alkenyl mit 8-20, insbesondere 12-20, Kohlenstoffatomen und 1-4 Doppelbindungen.

Bevorzugt werden geradkettige Alkyle mit einer geraden Anzahl von 12-22 Kohlenstoffatomen, beispielsweise n-Dodecyl, n-Tetradecyl, n-Hexadecyl, n-octadecyl, n-Eicosyl oder n-Docosyl eingesetzt.

Alkenyl mit 8-24, insbesondere 12-22, Kohlenstoffatomen und 0-5, insbesondere 1-3, Doppelbindungen ist beispielsweise 1-Octenyl, 1-Nonenyl, 1-Decenyl, 1-Undecenyl, 1-Dodecenyl, 9-cis-Dodecenyl (Lauroleyl), 1-Tridecenyl, 1-Tetradecenyl, 9-cis-Tetradecenyl (Myristoleyl), 1-Pentadecenyl, 1-Hexadecenyl, 9-cis-Hexadecenyl (Palmitoleinyl), 1-Heptadecenyl, 1-Octadecenyl, 6-cis-Octadecenyl (Petroselinyl), 6-trans-Octadecenyl (Petroselaidinyl), 9-cis-Octadecenyl (Oleyl), 9-trans-Octadecenyl (Elaidinyl), 9-cis-12-cis-Octadecadienyl (Linoleyl), 9-cis-11- trans-13-trans-Octadecatrienyl (alpha-Eläostearinyl), 9-trans-11-trans-13-trans-Octadecatrienyl (beta-Eläostearinyl), 9-cis-12-15-cis-Octadecatrienyl (Linolenyl), 9-, 11-, 13-, 15-Octadecatetraenyl (Parinaryl), 1-Nonadecenyl, 1-Eicosenyt, 9-cis-Eicosenyl (Gadoleinyl), 5-, 11-, 14-Eicosatrienyl oder 5-, 8-, 11-, 14-Eicosatetraenyl (Arachidonyl).

Bevorzugt ist Alkenyl mit 12-20 Kohlenstoffatomen und einer Doppelbindung, beispielsweise 9-cis-Dodecenyl (Lauroleyl), 9-cis-Tetradecenyl (Myristoleyl), 9-cis-Hexadecenyl (Palmitoleinyl), 6-cis-Octadecenyl (Petroselinyl), 6-trans-Octadecenyl (Petroselaidinyl), 9-cis-Octadecenyl (Oleyl), 9-trans-Octadecenyl (Elaidinyl) oder 9-cis-Eicosenyl (Gadoleinyl).

Methyl oder Ethyl sind zwei Beispiele für kurzkettiges Alkyl R₂, R₃ oder R₄ in Substanzen gemäß Formel 9.

Zwei Beispiele für Phenyl-kurzkettiges-alkyl in R₂ sind Benzyl oder 2-Phenylethyl.

Ein aliphatischer Heterocyclus, welcher von R₂ und R₃ zusammen mit dem Stickstoffatom gebildet wird, ist beispielsweise ein monocyclischer, fünf- oder sechsgliedriger Aza-, Oxaaza- oder Thiazacyclylrest, z.B. Piperidino, Morpholino oder Thiamorpholinio.

Substituenten dieses Heterocylus sind die Substituenten R₁ und R₄ am Stickstoff sowie gegebenenfalls an einem Kohlenstoffatom Nieder- alkyl, z.B. Methyl, Ethyl, n-Propyl oder n-Butyl.

Ein Heterocyclus, welcher von R₂ und R₃ zusammen mit dem Stickstoffatom gebildet wird und an einem Kohlenstoffatom durch kurzkettiges Alkyl substituiert ist, ist z.B. 2-, 3- oder 4-Methylpiperidinio, 2-, 3- oder 4-Ethylpiperidinio oder 2- oder 3-Methylmorpholinio.

Ein aromatischer Heterocyclus, welcher von R₂, R₃ und R₄ zusammen mit dem Stickstoffatom gebildet wird, ist beispielsweise ein monocyclischer, fünf-oder sechsgliedriger, Aza-, Diaza-, Oxaaza- oder Thiazacyclylrest, z.B. Pyridinio, Imidazolinio, Oxazolinio oder Thiazolinio oder beispielsweise ein benzokondensierter Monoazabicyclylrest, z.B. Chinolinio oder Isochinolinio.

Substituenten solcher Heterocyclen sind der Rest R₁ am Stickstoffatom sowie gegebenenfalls an einem Kohlenstoffatom kurzkettiges Alkyl, z.B. Methyl oder Ethyl, Hydroxy-kurzkettiges Alkyl, z.B. Hydroxymethyl oder 2-Hydroxyethyl, Oxo, Hydroxy oder Halogen, z.B. Chlor oder Brom.

Ein Heterocyclus, welcher von R₂, R₃ und R₄ zusammen gebildet wird und an einem Kohlenstoffatom durch die genannten Reste substituiert ist, ist beispielsweise ein 2- oder 4-kurzkettiges-Alkylpyridinio, z.B. 2- oder 4-Methyl oder 2- oder 4-Ethylpyridinio, Di-kurzkettiges-Alkylpyridinio, z.B. 2,6-Dimethyl-, 2-Methyl-3-ethyl-, 2-Methyl-4-ethyl-, 2-Methyl-5-ethyl-, oder 2-Methyl-6-ethylpyridinio, 2-, 3- oder 4-Halogen-pyridinio, z.B. 2-, 3- oder 4-Chlorpyridinio oder 2-, 3- oder 4-Brompyridinio, 2-kurzkettiges Alkylimidazolinio, - oxazolinio oder -thiazolinio, z.B. 2-Methyl- oder 2-Ethylimidazolinio, -oxazolinio oder -thiazolinio oder 2-kurzkettiges Alkyl-8-halogenchinolinio, z.B. 2-Methyl-8-chlorchinolinio.

Ein kationisches Tensid der Formel 9 ist vorzugsweise N-Benzyl-N,N-dimethyl-N-2-(2-(4-(1,1,3,3-tetramethylbutyl)-phenhydroxy)-ethhydroxy)-ethylammoniochlorid, N-Benzyl-N,N-dimethyl-N-2-(2-(3(methyl-4-(1,1,3,3-tetramethylbutyl)-phenhydroxy)-ethhydroxy)-ethylammoniochlorid (Methylbenzethoniumchlorid), n-Dodecyltrimethylammoniochlorid oder -bromid, Trimethyl-n-tetradecylammoniochlorid oder - bromid, n-Hexadecyltrimethylammoniochlorid oder -bromid (Cetyltrimethyl-ammoniumchlorid oder -bromid), Trimethyl-n-octadecylammoniochlorid oder -bromid, Ethyl-n-dodecyldimethylammoniochlorid oder -bromid, Ethyldimethyl-n-tetradecylammoniochlorid oder -bromid, Ethyl-n-hexadecyldimethylammoniochlorid oder -bromid, Ethyldimethyl-n-octadecylammoniochlorid oder -bromid, n-Alkylbenzyldimethylammoniochlorid oder -bromid (Benzalkoniumchlorid oder -bromid), z.B. Benzyl-n-dodecyldimethylammoniochlorid oder bromid, Benzyldimethyl-n-tetradecylammoniochlorid oder -bromid, Benzyl-n-hexadecyldimethyl-ammoniochlorid oder -bromid oder Benzyldimethyl-n-octadecylammonio-chlorid oder -bromid, N-(n-Decyl)-pyridiniochlorid oder -bromid, N-(n-Dodecyl)-pyridiniochlorid oder -bromid, N-(n-Tetradeyl)-pyridiniochlorid oder -bromid, N-(n-Hexadecyl)-pyridiniochlorid oder -bromid (Cetylpyridiniumchlorid) oder N-(n-Octadecyl)-pyridinio-chlorid oder -bromid oder eine Mischung von diesen randaktiven Substanzen.

Für biologische Zwecke werden besonders häufig die folgenden Tenside verwendet: N,N-bis(3-D-glucon-amidopropyl)cholamid (BigCHAP), Bis(2-ethylhexyl)natrium-sulfosuccinat, Cetyltrimethyl-ammonium-bromid, 3-((Cholamidopropyl)-dimethylammonio)-2-hydroxy-1-propansulfonat (CHAPSO), 3-((Cholamidopropyl)-dimethylammonio)-1-propansulfonat (CHAPS), Cholat- Natriumsalz, Decaoxyethylen-dodecyl-ether (Genapol C-100), Decaethylen-isotridecyl-ether (Genapol X-100), Decanoyl-N-methyl-glucamid (MEGA-10), Decyl-glucosid, Decyl-maltosid, 3-(Decyldimethylammonio)-propan-sulfonat (Zwittergent 3-10), Deoxy-bigCHAP, Deoxycholat, Natriumsalz, Digitonin, 3-(Dodecyldimethylammonio)-propan-sulfonat (Zwittergent 3-12), Dodecyl-dimethyl-amin-oxid (EMPIGEN), Dodecyl-maltosid, Dodecylsulfat, Glyco-cholat, Natriumsalz, Glyco-deoxycholat, Natriumsalz, Heptaethylen-glycol-octyl-phenylether (Triton X-114), Heptyl-glucosid, Heptyl-thioglucosid, 3-(Hexadecyldimethylammonio)-propan-sulfonat (Zwittergent 3-14), Hexyl-glucosid, Dodecyl-dimethyl-amin-oxid (Genaminox KC), N-Dodecyl-N,N-dimethylglycin (Empigen BB), N-Decyl-sulfobetain (Zwittergent 3-10), N-Dodecyl-sulfobetain (Zwittergent 3-12), N-Hexadecyl-sulfobetain (Zwittergent 3-16), N-Tetradecyl-sulfobetain (Zwittergent 3-14), N-Octyl-sulfobetain (Zwittergent 3-08), Nonaethylen-glycol-monododecyl-äther (THESIT), Nonaethylen-glycol-octyl-phenol-ether (Triton X-100), Nonaethylen-glycol-octyl-phenyl-ether (NP-40, Nonidet P-40), Nonaethylen-dodecyl-äther, Nonanoyl-N-methyl-glucamid (MEGA-9), Nonaoxyethylen-dodecylether (Lubrol PX, Thesit), Nonyl-glucosid, Octaethylen-glycol-isotridecyl-ether (Genapol X-080), Octaethylen-dodecylether, Octanoyl-N-methyl-glucamid (MEGA-8), 3-(Octyldimethylammonio)-propan-sulfonat (Zwittergent 3-08), Octyl-glucosid, Octyl-thioglucosid, Pentadecaethylen-isotridecyl-ether (Genapol X-150), Polyethylen-polypropylen-glycol (Pluronic F-127), Polyoxyethylen-sorbitan-monolaurat (Tween 20), Polyoxyethylen-sorbitan-monooleat (Tween 80), Taurodeoxycholat-Natriumsalz, Taurocholat-Natriumsalz, 3-(Tetradecyldimethylammonio)-propan-sulfonat (Zwittergent 3-14), usw.

Für pharmakologische Zwecke sind besonders gut geeignet: Cetyl-trimethyl-ammonium-salze (z.B. Hexadecyltrimethylammoniumbromid, Trimethylhexadecylamin-Bromsalz), Cetylsulfatsalze (z.B. Na-Salz, Lanette E), Cholatsalze (z.B. Na- und Ammonium-Form) Decaoxyethylen-dodecyl-ether (Genapol C-100), Deoxycholatsalze, Dodecyl-dimethyl-amin-oxid (Genaminox KC, EMPIGEN), N-Dodecyl-N,N-dimethylglycin (Empigen BB), 3-(Hexadecyldimethylammonio)-propan-sulfonat (Zwittergent 3-14), Fettsäuresalze und Fettalkohole, Glyco-deoxycholatsalze, Laurylsulfatsalze (Natrium Dodecylsulfat, Duponol C, SDS, Texapon K12), N-Hexadecyl-sulfobetain (Zwittergent 3-16), Nonaethylen-glycol-octyl-phenyl-ether (NP-40, Nonidet P-40), Nonaethylen-dodecyl-äther, Octaethylen-glycol-isotridecyl-ether (Genapol X-080), Octaethylen-dodecyl-ether, Polyethylenglykol-20-Sorbitan-Monolaurat (Tween 20), Polyethylenglykol-20-Sorbitan-Monostearat (Tween 60), Polyethylenglykol-20-Sorbitan-Monooleat (Tween 80), Polyhydroxyethylen-Cetylstearylether (Cetomacrogo, Cremophor O, Eumulgin, C 1000) Polyhydroxyethylen-4-Laurylether (Brij 30), Polyhydroxyethylen-23-Laurylether (Brij 35), Polyhydroxyethylen-8-Stearat (Myrj 45, Cremophor AP), Polyhydroxyethylen-40-Stearat (Myrj 52), Polyhydroxyethylen-100-Stearat (Myrj 59), polyethoxyliertes Rizinusöl 40 (Cremophor EL), polyäthoxyliertes hydriertes Rizinsöl (Cremophor RH 40, Cremophor RH 60) polyethoxylierte pflanzliche Öle (Lebrafils), Sorbitan-Monolaurat (Arlacel 20, Span 20), Taurodeoxycholatsalze, Taurocholatsalze, Polyethylenglykol-20-Sorbitan-Palmitat (Tween 40), Myrj 49 und Polyethylenglykolderivate des Ricinols usw.

### WIRKSTOFFE:

Die erfindungsgemäßen Transfersomen eignen sich zur Applikation unterschiedlichster Wirkstoffe, insbesondere z. B. zu therapeutischen Zwecken. So können erfindungsgemäße Präparate enthalten:
- mindestens einen adrenocorticostatischen Wirkstoff, insbesondere Metyrapon;
- mindestens einen Trägerstoff, Zusatzstoff oder Wirkstoff, der zu den beta-Adrenolytica (Beta blocking agents) gehört, insbesondere Acetobol, Alprenolol, Bisoprololfumarat, Bupranolol, Carazolol, Celiprolol, Mepindolsulfat, Metipranolol, Metoprolotartat, Nadolol, Oxyprenolol, Pindolol, Sotalol, Tertatolol, Timolohydrogenmaleat und Toliprolol, besonders bevorzugt Atenolol oder Propranolol;
- mindestens einen Trägerstoff, Zusatzstoff oder Wirkstoff, der zu den Androgenen oder Antiandrogenen gehört, insbesondere Drostanolonpropionat, Mesterolon, Testosteronundecanoat, Testolacton, Yohimbin, beziehungsweise Chloramidinonacetat, Cyproteronacetat, Ethinylestradiol oder Flutamid;
- mindestens einen Trägerstoff, Zusatzstoff oder Agens mit antiparasitärer Wirkung, insbesondere Phanquinon, Benzyobenzoat, Bephenium-hydroxy-naphthoat, Crotamiton, Diäthylcarbamazin, Levamisol, Lindan, Malathion, Mesulfen (2,7-Dimethylantren), Metronidazol oder Tetramisol;
- mindestens einen anabolischen Wirkstoff, insbesondere Clostebolacetat, Cyanocobolamin, Folsäure, Mestanolon, Metandienon, Metenolon, Nandrolon, Nandrolondecanoat, Nandrolon-hexyloxyphenylpropionat, Nandrolon-phenyl-propionat, Norethandrolon, Oxaboloncipionat, Piridoxin oder Stanozolol;
- mindestens einen Wirkstoff, der zu einer systemischen Anästhesie oder Analgesie beiträgt, insbesondere Chlorobutanol, Ketamin, Oxetacain, Propanidid und Thiamylal, Aminophenol-Derivate, Aminophenazol-Derivate, Antranilsäure- und Arylpropionsäurederivate, Azapropazon, Bumadizon, Chloroquin- und Codein-Derivate, Diclophenac, Fentanil, Ibuprofen, Indometacin, Ketoprofen, Methadon-Substanzen, Morazon, Morphin und seine Derivate, Nifenazon, Nifluminsäure, Pentazozin, Pethidin, Phenazopyridin, Phenylbutazon-Derivate (wie z.B. 3,5 Pyrazolidindion), Pherazon, Piroxicam, Propoxyphen, Propyphenazon, Pyrazol- und Phenazon-Derivate (Aminophenazon, Metamizol, Monophenylbutazon, Oxyphenbutazon, Phenylbutazon bzw. Phenazon-Salyzilat), Salicylsäure-Derivate, Sulfasalazin, Tilidin; Acetylsalicylsäure, Äthylmorphin, Alclofenac, Alphaprodin, Aminophenazon, Anileridin, Azapropazon, Benfotiamin, Benorilat, Benzydamin, Cetobemidon, Chlorphenesincarbamat, Chlorthenoxazin, Codein, Dextromoramid, Dextropropoxyphen, Ethoheptazin, Fentanyl, Fenyramidol, Fursultiamin, Flupirtinmaleat, Glafenin, Hydromorphon, Lactylphenetidin, Levorphanol, Mefenamsäure, Meptazonol, Methadon, Mofebutazon, Nalbufin, Na-Salz des Noramidopyrinium-methansulfonats, Nefopam, Normethadon, Oxycodon, Paracetamol, Pentazocin, Pethidin, Phenacetin, Phenazocin, Phenoperidin, Pholcodin, Piperylon, Piritramid, Procain, Propyphenazon, Salicylamid, Thebacon, Tiemonium-jodid, Tramadon;
- mindestens einen Stoff aus der Klasse der Analeptica, z.B. Aminophenazol, Bemegrid, Coffein, Doxapram, Ephedrin, Prolintan, bzw. Nialamid und Tranylcypromin; außerdem Vitamine, pflanzliche Extrakte aus Baldrian, Semen Colae, Campher, Menthol;
- mindestens einen Stoff aus der Klasse der Antiallergica, z. B. Agentien aus den Klassen der Globuline, Korticoide oder Antihistaminica (wie z.B. Beclometason-, Betametason-Cortison-, Dexametason-Derivate, usw.), ferner Bamipinacetat, Buclizin, Clemastin, Clemizol, Cromoglicinsäure, Cyproheptadin, Diflucorolonvalerat, Dimetotiazin, Diphenhydramin, Diphenylpyralin, Ephedrin, Fluocinolan, Histapyrrodin, Isothipendyl, Methadilazin, Oxomemazin, Paramethason, Predniliden, Theophillin, Tolpropamin Tritoqualin, usw. eingesetzt. Zu bevorzugten Wirkstoffe gehören ferner Agentien, die dadurch gekennzeichnet sind, daß sie mit der Produktion immunologisch aktiver Substanzen, z.B. Interleukinen, Interferonenen, Leukotrienen, Prostaglandinen, usw. interferieren. Dazu gehören auch bestimmte Lipide und Lipoide, z.B. Phosphatidylcholin, Diacylglycerole, oder Fettsäuren und ihre Ester, die Ketten mit mehreren, bevorzugt 3-6, besonders häufig 3 oder 4, Doppelbindungen haben, vorzugsweise vom n-3 Typus, und/oder hydroxygeniert, verzweigt oder zu einem (Teil)Ring geschlossen sind.
- mindestens einen Stoff, der eine antiarrhythmische Wirkung aufweist, wie z.B. Cardiaca und beta-Blocker, Ajmalin, Bupranolol, Chinidin, Digoxinderivate, Diltiazem, Disopyramiddihydrogensulphat, Erythromycin, Disopyramid, Gallopamil, Ipratropiumbromid, Lanatosid, Lidocain, Lorcainid, Orciprenalinsulfat, Procainamid, Propafenon, Sparteinsulfat, Verapamil, Toliprolol;
- ein Antiarterioscleroticum, wie z.B. Clofibrat.
- mindestens eine Substanz, die zu den Antiasthmatica und/oder Bronchospasmolytica gehört, z.B. Amiodaron, Carbuterol, Fenoterol, Orciprenalin, Sotalol, oder Theophillin-Derivate, sowie Corticoide (wie z.B. Beclomethason, Dexamethason, Hydrocortison, Prednisolon), häufig in Kombinationen mit Purinen;
- mindestens einen Stoff aus der Klasse der Antibiotica, z.B. Actinomycin, Alamethicin, Alexidin, 6-Aminopenicillan Säure, Amoxicillin, Amphotericin, Ampicillin, Anisomycin, Antiamoebin, Antimycin, Aphidicolin, Azidamfenicol, Azidocillin, Bacitracin, Beclomethason, Benzathin, Benzylpenicillin, Bleomycin, Bleomycin sulfat, Calcium lonophor A23187, Capreomycin, Carbenicillin, Cefacetril, Cefaclor, Cefamandole nafat, Cefazolin, Cefalexin, Cefaloglycin, Cefaloridin, Cefalotin, Cefapirin, Cefazolin, Cefoperazon, Ceftriaxon, Cefuroxim, Cephalexin, Cephaloglycin, Cephalothin, Cephapirin, Cerulenin, Chloramphenicol, Chlortetracyclin, Chloramphenicol diacetat, Ciclaciliin, Clindamycin, Chlormadinone Acetat, Chlorpheniramin, Chromomycin A3, Cinnarizin, Ciprofloxacin, Clotrimazol, Cloxacillin, Colistin methanesulfonat, Cycloserin, Deacetylanisomycin, Demeclocyclin, 4,4'-Diaminodiphenyl sulfon, Diaveridin, Dicloxacillin, Dihydrostreptomycin, Dipyridamol, Doxorubicin, Doxycyclin, Epicillin, Erythromycin, Erythromycinstolat, Erythromycinethylsuccinat, Erythromycin stearat, Ethambutol, Flucloxacillin, Fluocinolone Acetonid, 5-Fluorocytosin, Filipin, Formycins, Fumaramidomycin, Furaltadon, Fusid Säure, Geneticin, Gentamycin, Gentamycin sulfat, Gliotoxin, Gfamicidin, Griseofulvin, Helvol Säure, Hemolysin, Hetacillin, Kasugamycin, Kanamycin (A), Lasalocid, Lincomycin, Magnesidin, Melphalan, Metacyclin, Meticillin, Mevinolin, Mikamycin, Mithramycin, Mithramycin A, Mithramycin complex, Mitomycin, Minocyclin, Mycophenol Säure, Myxothiazol, Natamycin, Nafcillin, Neomycin, Neomycin sulfat, 5-Nitro-2-furaldehydsemicarbazon, Novobiocin, Nystatin, Oleandomycin, Oleandomycin phosphat, Oxacihin, Oxytetracyclin, Paromomycin, Penicillin, Pecilocin, Pheneticillin, Phenoxymethylpenicillin, Phenyl Aminosalicylat, Phleomycin, Pivampicillin, Polymyxin B, Propicillin, Puromycin, Puromycin Aminonucleosid, Puromycin Aminonucleosid 5'-monophosphat, Pyridinol carbamat, Rolitetracyclin, Rifampicin, Rifamycin B, Rifamycin SV, Spectinomycin, Spiramycin, Streptomycin, Streptomycin sulfat, Sulfabenzamid, Sulfadimethoxin, Sulfamethizol, Sulfamethoxazol, Tetracyclin, Thiamphenicol, Tobramycin, Troleandomycin, Tunicamycin, Tunicamycin A1-Homolog, Tunicamycin A2-Homolog, Valinomycin, Vancomycin, Vineomycin A1, Virginiamycin M1, Viomycin, Xylostasin;
- mindestens einen Stoff, der zu den Antidepressiva oder Antipsychotica gehört, z.B. diverse Monoaminoxidase-Hemmer, Tri- und Tetrazyclische Antideptressiva, usw. Häufig werden Alprazolam, Amitriptylin, Chlorpromazin, Clomipramin, Desipramin, Dibenzepin, Dimetacrin, Dosulepin, Doxepin, Fluvoxaminhydrogenmaleat, Imipramin, Isocarboxazid, Lofepramin, Maprotilin, Melitracen, Mianserin, Nialamid, Noxiptilin, Nomifensin, Nortriptylin, Opipramol, Oxypertin, Oxytriptan, Phenelzin, Protriptylin, Sulpirid, Tranylcypromin, Trosadon, Tryptophan, Vitoxazin, usw. verwendet.
- mindestens einen Stoff, der zu den Antidiabetica gehört, wie z.B. Acetohexamid, Buformin, Carbutamid, Chlorpropamid, Glibenclamid, Glibornurid, Glymidine, Metformin, Phenformin, Tolazamid, Tolbutamid;
- mindestens einen Stoff, der als Gegengift (Antidot) dient, beispielsweise gegen Metallvergiftungen, Insektizidvergiftungen, Drogen, gegen Blutgifte usw. Einige Beispile sind z.B. diverse Chelatoren, Amiphenazol Obidoxim-chlorid, D-Penicillamin, Tiopromin, usw;
- mindestens einen Stoff, der zu den Antiemetica gehört. Geeignete Wirkstoffe dafür sind z.B. Alizaprid, Benzquinamid, Betahistidin-Derivate, Cyclizin, Difenidol, Dimenhydrinat, Haloperidol, Meclozin, Metoclopramid, Metopimazin, Oxypendyl, Perphenazin, Pipamazin, Piprinhydrinat, Prochlorperazin, Promazin, Scopolamin, Sulpirid, Thiethylperazin, Thioproperazin, Triflupromazin, Trimethobenzamid, usw, die häufig in Kombination mit Vitaminen und/oder Antiallergica verwendet werden;
- mindestens einen Stoff, der zu den Antiepileptica gehört. Geeignete Wirkstoffe dafür sind z.B. Barbexaclon, Barbiturate, Beclamid, Carbamazepin, Chloralhydrat, Clonazepam, Diazepam, Ethosuximid, Ethylphenacemid, Lorazepam, Mephenytoin, Mesuximid, Oxazolidine, Phenaglycodol, Phensuximid, Phenytoin, Primidon, Succinimid-Derivate, Sultiam, Trimethadion, Yalproinsäure, usw. Häufig gehören die Zutaten in die Klasse der Hypnotica und Sedativa. Besonders häufig wird Carbamazepin verwendet.
- mindestens einen Stoff mit antifibrinolytischer Wirkung, z.B. Aminocapronsäure oder Tranexamsäure.
- mindestens einen Stoff, der zu den Anticonvulsiva gehört, z.B. Beclamid, Carbamazepin, Clomethiazol, Clonazepam, Methylphenobarbital, Phenobarbital oder Sultiam;
- mindestens einen Stoff, der in den Cholinhaushalt eingreift, z.B. eine anticholinergische Wirkung ausübt. Als Cholinergica können unter anderem verwendet werden: Aubenoniumchlorid, Carbachol, Cerulezid, Dexpanthenol und Stigmin-Derivate (z.B. Distigminbromid, Neostigminmethylsulfat, Pyridostigminbromid) Als Anticholinergica dienen häufig Atropin, Atropinmethonitrat, Benactyzin, Benzilonium-bromid, Bevonium-methylsulfat, Chlorbenzoxamin, Ciclonium-bromid, Clidinium-bromid, Dicycloverin, Diphemanil-methylsulfat, Fenpiverinium-bromid, Glycopyrroniumbromid, Isopropamid-jodid, Mepenzolat-bromid, Octatropin-methylbromid, Oxyphencyclimin, Oxyphenonium-bromid, Pentapiperid, Pipenzolat-bromid, Piperidolat, Pridinol, Propanidid, Tridihexethyl-jodid und Trospiumchlorid als Agenzien für diesen Zweck benutzt. Auch Cholinesterase-Inhibitoren, wie z.B. Ambenonium-chlorid, Demecarium-bromid, Echothiopate-jodid, usw. sind für diesen Zweck nützlich;
- mindestens einen Stoff zur Beeinflußung, zumeist Herabsetzung, der Wirkung oder Konzentration von Histamin (Antihistaminika). Bevorzugt werden hypoallergisch wirkende Träger oder randaktive Stoffe mit n-3 (omega-3), seltener mit oder n-6 (omega-6), mit zumeist mehreren, häufig 3-6 Doppelbindungen, gelegentlich auch mit Hydroxy, seltener Methyl-, oder Oxo-Seitengruppen, bzw. in Epoxykonfiguration, eingesetzt. Weitere geeignete Wirkstoffe sind unter anderem Aethylendiamin, Alimemazin, Antazolin, Bamipin, Bromazin, Brompheniramin, Buclizin, Carbinoxamin, Chlorcyclizin, Chloropyramin, Chlorphenanin, Chlorphenoxamin, Cimetidin, Cinnarizin, Clemastin, Clemizol, Colamin (z.B. Diphenhydramin), Cyclizin, Dexbrompheniramin, Dexchlorpheniramin, Difenidol, Dimetinden, Dimetotiazin, Diphenhydramin, Diphenylpyralin, Dixyrazin, Doxylamin, Histapyrrodin, Isothipendyl, Mebhydrolin, Meclozin, Medrylamin, Mepyramin, Methdilazin, Pheniramin, Piperacetazin, Piprinhydrinat, Pyrilamin (Mepyramin), Promethazin, Propylamin, Pyrrobutanin, Thenalidin, Tolpropamin, Tripelennamin, Triprolidin, usw;
- mindestens einen Stoff, der zu den Antihypertonica gehört, z.B. viele alpha-Rezeptoragonisten, Aldosteron-Antagonisten, Angiotensin-Converting-Enzyme-Hemmer, Antisymphaticotonica, beta-Blocker, Calzium-Antagonisten, Diuretica, Vasodilatoren, usw. Geeignete Wirkstoffe dafür sind z.B. Alpenolol, Atenolol, Bendroflumethiazid, Betanidin, Butizid, Chlortalidon, Clonidin, Cycletanin, Cyclopenthiazid, Debrisoquin, Diazoxid, Dihydralazin, Dihydroergotaminmethansulfonat, Doxazinmesilat, Guanethidin, Guanoclor, Guanoxan, Hexamethonium-chlorid, Hydralazin, Labetalol, Mecanylanin, Methyldopa, Pargylin, Phenoxybenzamin, Prazosin, Quinethazon, Spironolacton, Bescinnamin, Reserpin, Trichlormethiazid oder Vincamin;
- mindestens einen Stoff, der ein Inhibitor biologischer Aktivität ist, z.B. Actinomycin C1, alpha-Amanitin, Ampicillin, Aphidicolin, Aprotinin, Calmidazolium (R24571), Calpain-Inhibitor I, Calpain-Inhibitor II, Castanospermin, Chloramphenicol, Colcemid, Cordycepin, Cystatin, 2,3-Dehydro-2-desoxy-N-acetyl-neuraminsäure, 1-Desoxymannojirimycin-hydrochlorid, 1-Desoxynojirimycin,Diacylglycerolkinase-Inhibitor, P1, P5-Di(adenosin-5'-)pentaphosphat, Ebelacton A, Ebelacton B, Erythromycin, Ethidiumbromid, N-Hydroxyharnstoff, Hygromycin B, Kanamycinsulfat, alpha2-Macroglobulin, N-Methyl-1-desoxynojirimycin, Mitomycin C, Myxothiazol, Novobiocin, Phalloidin, Phenylmethylsulfonylfluorid, Puromycin-dihydrochlorid, Rifampicin, Staurosporin, Streptomycinsulfat, Streptozotocin, g-Strophanthin, Swainsonin, Tetracyclin-hydrochlorid, Trifluoperazin-dihydrochlorid, Tunicamycin, usw. Nützliche Proteinasen Inhibitoren sind z.B. (4-Amidinophenyl)-methansulfonylfluorid (APMSF), Antipain-dihydrochlorid, Antithrombin III, alphal-Antitrypsin, Aprotinin, Bestatin, Calpain-Inhibitor I, Calpain-Inhibitor II L-1-Chlor-3-(4-tosylamido)-7-amino-2-heptanon-hydrochlorid (TLCK), L-1-Chlor-3-(4-tosylamido)-4-phenyl-2-butanon (TPCK), Chymostatin, Cystatin, 3,4-Dichlorisocoumarin, E 64, Elastatinal, Hirudin, Kallikrein-Inhibitor (Aprotinin) L-Leucinthiol, Leupeptin, Pepstatin, Phenylmethylsulfonylfluorid (PMSF), Phosphoramidon, TLCK (Tosyl-lysin-chlormethylketon), TPCK(Tosyl-phenylalanin-chlormethylketon), Trypsin-Inhibitoren, usw;
- mindestens einen Stoff, der zu den Antihypotonica gehört. Häufig sind die entsprechenden Agenzien gleichzeitig auch Analeptica, Kardiaca oder Corticoide. Zu den gut geeigneten Wirkstoffen gehören unter anderem Angiotensinamid, Cardaminol, Dobutamin, Dopamin, Etifelmin, Etilefrin, Gepefrin, Heptaminol, Midodrin, Oxedrin, usw., ganz besonders Norfenefrin;
- mindestens einen Stoff, der zu den Antikoagulantien gehört. Zu den dafür geeigneten Wirkstoffen gehören aus den Klassen der Coumarin-Derivate, Heparin und Heparinoide, Hirudin und verwandte Stoffe, Dermatansulfat usw. Häufig werden verwendet Acenocumarin, Anisindion, Diphenadion, Ethylbiscoumacetat, Heparin, Hirudin, Phenprocoumon sowie Warfarin;
- mindestens einen Stoff, der zu den Amtimycotica gehört. Zu den dafür gut geeigneten Wirkstoffe gehören z.B. Amphotericin, Bifanozol, Buclosamid, Chinolin-sulfat Chlormidazol, Chlorphenesin, Chlorquinaldol, Clodantoin, Cloxiquin, Cyclopiroloxamin, Dequaliniumchlorid, Dimazol, Fenticlor, Flucytosin, Griseofulvin, Ketoconazol, Miconazol, Natamycin, Sulbentin, Tioconazol, Tolnaftat, usw. Besonders häufig werden Amphotericin, Clotrimazol oder Nystatin verwendet;
- mindestens einen Stoff, der zu der Klasse der Antimyasthenica gehört, wie z.B. Pyridostigmin-bromid;
- mindestens einen Stoff, der wirksam gegen morbus Parkinson ist, z.B. Amantadin, Benserazid, Benzatropin, Biperiden, Cycrimin, Levodopa, Metixen, Orphenadrin, Phenglutarimid, Pridinol, Procyclidin, Profenamin oder Trihexyphenidyl;
- mindestens einen Stoff, der ein Antiphlogisticum ist, z.B. Aescin, Acetylsalicylsäure, Alclofenac, Aminophenazon, Azapropazon, Benzydamin, Bumadizon, Chlorthenoxazin, Diclofenac, Flufenaminsäure, Glafenin, Ibuprofen, Indometacin, Kebuzon, Mefenamsäure, Metiazinsäure, Mesalazin, Mofebutazon, Naproxen, Nifluminsäure, Salze, z.B. Na-Salz, von Noramidopyrinium-methan-sulfonat, Orgotein, Oxyphenbutazon, Phenylbutazon, Propyphenazon, Pyridoxin, Tolmetin, usw.. Besonders häufig wird Ibuprofen verwendet. Häufig haben die für diesen Zweck verwendeten Wirkstoffe auch eine antihistaminische oder analgetische Wirkung oder gehören in die Klassen der Corticoide, Venenmittel, Opthalmica oder Otologica;
- mindestens ein Antipyreticum ist, z.B. Acetylsalicylsäure, Alclofenac, Aminophenazon, Benzydamin, Bumadizon, Chinin, Chlorthenoxazin, Lactylphenetidin, Meprob, Paracetamol, Phenacetin, Propyphenazon oder Salicylamid verwendet;
- mindestens einen Stoff mit antirheumatischer Wirkung, z.B. Acetylsalicylsäure, Benorilat, Chloroquin, Diclofenac, Fenoprofen, Flufenaminsäure, Ibuprofen, Kebuzon, Lactylphenetidin, Mefenamsäure, Mofebutazon, Naproxen, Natriumaurothiomalat, Nifenazon, Nifluminsäure, D-Penicillamin und Salicylamid. Bevorzugt werden hypoallergisch wirkenden randaktiven Stoffe, Träger und/oder Wirkstoffe, z.B. aus den Klassen der Analgetika, ferner Cortikoide und Glucokortikoide, Enzyme oder Vitamine, usw., verwendet; außerdem Antiphlogistika wie z.B. Chinin, Nikotinsäure-, Nonylsäure- sowie Salicylsäure-Derivate, Meprobamat, usw;
- mindestens ein Antisepticum wie Acriflaviniumchlorid, Cetalkonium-chlorid, Cetylpyridinium-chlorid, Chlorhexidin, Chlorquinaldol, Dequaliniumchlorid, Domiphen-bromid, Ethacridin, Hexetidin, Merbromin, Nitrofural, Oxyquinol, Phanquinon, Phenazopyridin oder Phenylmercuriborat, sowie Fetsäuren mit einer ungeraden Zahl der Kohlenstoffatome;
- mindestens ein Atemanalepticum oder Atemstimulans, z.B. Amiphenazol, Ascorbinsäure, Coffein, Cropropamid, Crotethamid, Etamivan, Ephedrin, Fominoben, Nicethamid; bzw. z.B. Aminophenazol oder Doxapram;
- mindestens ein Broncholyticum, wie Bamifyllin, Beclometason, Dexometason (wie z.B. in Dexometason-21-isonicotinat), Diprophyllin, Ephinedrin (z.B. Ephinedrinhydrogentartrat), Fenoterol, Hexoprenalin, Ipratropium-bromid, Isoetarin, Isoprenalin, Orciprenalin, Protokylol, Proxyphyllin, Reproterol, Salbutamol, Terbutalin, Tetroquinol, Theophyillin, usw., und biologische Extrakte, z.B. aus Anis, Eukalyptus, Thymian, usw;
- ein Cardiotonicum, besonders Aminophyllin, Benfurodilhemisuccinat, Etofyllin, Heptaminol, Protheobromin oder Proxyphyllin;
- mindestens einen Stoff aus der Klasse der Chemotherapeutica wie etwa Acediasulfon, Acriflaviniumchlorid, Ambazon, Dapson, Dibrompropamidin, Furazolidon, Hydroxymethyinitrofurantoin, Idoxuridin, Mafenid u. Sulfatolamid, Mepacrin, Metronidazol, Nalidixinsäure, Nifuratel, Nifuroxazid, Nifuarazin, Nifurtimox, Ninorazol, Nitrofurantoin, Oxolinsäure, Pentamidin, Phenazopyridin, Phthalylsulfathiazol, Pyrimethamin, Salazosulfapyridin, Sulfacarbamid, Sulfacetamid, Sulfachlorpyridazin, Sulfadiazin, Sulfadicramid, Sulfadimethoxin, Sulfaethidol, Sulfafurazol, Sulfaguanidin, Sulfaguanol, Sulfamethizol, Sulfamethoxazol und Cotrimoxazol, Sulfamethoxydiazin, Sulfamethoxypyridazin, Sulfamoxol, Sulfanilamid, Sulfaperin, Sulfaphenazol, Sulfathiazol, Sulfisomidin, Tinidazol, Trimethoprim, usw.;
- mindestens einen Stoff aus der Klasse der Coronardilatatoren. z.B. Bamifyllin, Benziodaron, Carbochromen, Dilazep, Dipyridamol, Etafenon, Fendilin, Hexobendin, Imolamin, Lidoflazin, Nifedipin, Oxyfedrin, Pentaerythrityltetranitrat, Perhexilin, Prenylamin, Propatylnitrat, Racefemin, Trolnitrat, Verapamil, Visnadin, usw.;
- mindestens ein Cytostaticum, z.B. aus den Klassen der Alkylantien, Antibiotica, Platinderivate, Hormone und ihrer Hemmer, Interferone, usw. Sehr häufig werden verwendet: Aclarubicin, Azathioprin, Bleomycin, Busulfan, Calciumfolinat, Carboplatin, Carmustin, Chlorambucil, Cis-Platin, Cyclophosphamid, Cytarabin, Daunorubicin, Epirubicin, Fluorouracil, Fosfestrol, Hydroxycarbamid, Ifosfamid, Lomustin, Melphalan, Mercaptopurin, Methotrexat, Mitomycin C, Mitopodozid, Mitramicyn, Nimustin, Pipobroman, Prednimustin, Procarbazin, Testolacton, Theosulfan, Thiotepa, Tioguanin, Triaziquon, Trofosfamid, Vincristin, Vindesin, Vinblastin, Zorubicin, usw.;
- ein Darmantisepticum, wie z.B. Broxyquinolin, Clioquinol, Diodohydroxyquinolin, Halquinol, usw.;
- mindestens ein Diureticum, z.B. Acetazolamid, Aminophyllin, Bendroflumethiazid, Bumetanid, Butizid, Chlorazanil, Chlormerodrin, Chlorothiazid, Chlortalidon, Clopamid, Clorexolon, Cyclopenthiazid, Cyclothiazid, Etacrynsäure, Furosemid, Hydrochlorothiazid, Hydroflumethiazid, Mefrusid, Methazolamid, Paraflutizid, Polythiazid, Quinethazon, Spironolacton, Triamteren, Trichlormethiazid, Xipamid, usw.;
- mindestens einen Ganglienblocker, z.B. Gallamintriethiodid, Hexamethonium-chlorid, Mecamylamin, usw.;
- mindestens einen Stoff zur Behandlung von Gicht, bevorzugt Analgetika, ferner, z.B. Allopurinol, Benzbromaron, Colchicin, Benziodaron, Probenecid, Sulfinpyrazon, Tenoxicam, usw. und ganz besonders häufig Allopurinol;
- mindestens ein Glucocorticoid, z.B. Beclomethason, Betamethason, Clocortolon, Cloprednol, Cortison, Dexamethason (z.B. als Dexamethasonphosphat), Fludrocortison, Fludroxycortid, Flumetason, Fluocinolonacetonid, Fluocinonid, Fluocortolon (z.B. als Fluocortoloncapronat oder Fluocortolontrimethyl-acetat), Fluorometholon, Fluprednidenacetat, Hydrocortison (auch Hydrocortison-21-acetat, Hydrocortison-21-phosphat, usw.), Paramethason, Prednisolon (z.B. als Methylprednisolon, Prednisolon-21-phosphat, Prednisolon-21-sulfobenzoat, usw.), Prednison, Prednyliden, Pregnenolon, Triamcinolon, Triamcinolonacetonid, usw.;
- mindestens ein Grippetherapeuticum, wie z.B Moroxydin.
- mindestens ein Hämostaticum wie Adrenalon, Ascorbinsäure, Butanol, Carbazochrom, Etamsylat, Protamin, Samatostatin, usw. Auch Hypophisen-Hormone und Vitamine können für diesen Zweck gut eingesetzt werden;
- mindestens ein Hypnoticum, z. B. aus der Klasse der Barbiturate, Benzodiazepine, Bromverbindungen, Ureide, usw. Häufig werden für diesen Zweck Acecarbromal, Alimemazintartrat Allobarbital, Amobarbital, Aprobarbital, Barbital, Bromisoval, Brotizolam, Carbromal, Chloralhydrat, Chloralodol, Chlorobutanol, Clomethiazol, Cyclobarbital, Diazepam, Diphenhydramin, Doxylamin, Estazolam, Ethchlorvynol, Ethinamat, Etomidat, Flurazepam, Glutethimid, Heptabarb, Hexobarbital, Lormetazepam, Malperol, Meclozin, Medozin, Methaqualon, Methyprylon, Midazolam, Nitrazepam, Oxazepam, Pentobarbital, Phenobarbital, Promethazin, Propallylonal, Pyrithyldion, Secbutabarbital, Secobarbital, Scopolamin, Temazepam, Triazolam, Vinylbital, usw; außerdem werden Extrakte aus Melisse, Baldrian, Passiflora verwendet;
- mindestens ein Immunglobulin, z.B. aus den Klassen IgA, IgE, IgD, IgG, IgM, oder ein Immunglobulinfragment, z.B. ein Fab- oder Fab2-Fragment, oder die entsprechende variable bzw. hypervariable Region, gegebenenfalls kombiniert mit anderen Stoffen und/oder chemisch, biochemisch oder gentechnisch manipuliert;
   Ein Immunglobulin kann vom Typ IgA, IgD und IgE, IgG (z.B. Ig G1, Ig G2, Ig G3, Ig G4) oder IgM sein. In dieser Anmeldung werden darunter auch chemische oder biochemische Abbauprodukte der Immunglobuline (Ig) verstanden, Ig G, gamma-Kette, Ig G, F(ab')2 Fragment, Ig G, F(ab) Fragment, Ig G, Fc Fragment, Ig kappa-Kette, leichte Ketten von Ig-s (z.B. kappa und lambda-Kette), aber auch noch kleinere Immunglobulinteile, wie z.B. die variable oder hypervariable Region, oder künstliche Abwandlungen von irgendeiner dieser Substanzen.
- mindestens einen Stoff mit Wirkung zur Immunstimulation, Immunsuppression, Erzeugung von Immunglobulinen oder sonstigen immunologisch wirksamen Substanzen (Endotoxinen, Cytokinen, Lymphokinen, Prostaglandinen, Leukotrienen, anderen Immunmodulantien oder biologischen Botstoffen), einschließlich Vakzinen. Ebenso können Antikörper gegen irgendeine dieser Substanzen verwendet werden. Bevorzugt werden Immuntransfersomen mit oder ohne Endotoxinen, Cytokinen, Prostaglandinen, Leukotrienen, mit anderen Immunmodulantien, immunologisch wirksammen Zell- oder Molekülfragmenten, sowie entsprechenden Antagonisten, Derivaten oder Vorläufern eingesezt. Besonders bevorzugt sind dabei Lipid A und andere Glycolipide, Muraminsäurenderivate, Trehalosederivate, Phythämagglutinine, Lectine, Polyinosin, Polycytidylsäure (Poli I:C), Dimepranol-4-acetamidobenzoat, Erythropoietin, 'Granulocyte-Macrophage Colony Stimulating Factor' (GM-CSF), Interleukine I und II, III und VI, Interferone alpha, β und/oder gamma, Leukotriene A, B, C, D, E und F, Propandiamin, Prostaglandine A, B, C, D, E, F, und I (Prostacyclin), Tumor Necrose Faktor-alpha (TNF-alpha), Thromboxan B, sowie Immunglobuline der Klassen IgA, IgE, IgD, IgG, IgM; aber auch Gewebsextrakte und Pflanzenextrakte, ihre chemische, biochemische oder biologische Nachahmungen bzw. ihre Teile, z.B. charakteristische Peptidketten. Zur Immunsupression werden häufig Ganciclovir, Azathiiprin, Cyclosporin, FK 506 usw. verwendet;
- mindestens ein Kontrazeptivum, wie z.B. Medroxyprogesteronacetat, Lynesterol, Lvonorgestrel, Norethisteron, usw;
- mindestens ein Kreislaufanalepticum wie Cafedrin, Etamivan, Etilefrin, Norfenefrin, Pholedrin, Theodrenalin, usw;
- mindestens ein Lebertherapeuticum wie Orazamid, Silymarin, oder Tiopromin;
- mindestens ein Stoff mit einer lichtschützenden Funktion, wie z.B. Mexenon;
- mindestens ein Antimalariamittel, wie z.B. Amodiaquin, Hydroxychloroquin oder Mepacrin;
- mindestens einen Stoff als Mittel gegen Migräne oder Schizophrenie, z.B. Analeptica, beta-Blocker, Clonidin, Dimetotiazin, Ergotamin, Lisurid(hydrogenmaleat), Methysergid, Pizotifen, Propranolol, Proxibarbal, usw. Noch besser geeignet sind jedoch Serotonin-Antagonisten oder Blocker eines Serotonin-Rezeptors, z.B. vom 5-HT1, 5-HT2 oder 5-HT3 ist. Gut geeignet für die Verwendung im Sinne dieser Erfindung sind ferner Rezeptor-Blocker AH21467 (Glaxo), AH25086 (Glaxo), GR43175 (Glaxo), GR38032 (Glaxo, = Ondansetron), 5-Hydroxytriptamin, Ketanserin, Methiothepin, alpha-Methyl-5HT, 2-Methyl-5HT, usw.;
- mindestens ein Mineralcorticoid, wie z.B. Aldosteron, Fludrocortison, Desoxycortonacetat, ihre Derivate, usw.;
- mindestens einen Morphin-Antagonisten (wie z.B. Amiphenazol, Lealvallorphan, Nalorphin) oder einen Stoff mit morphinähnlichen Eigenschaften (wie z.B. Casomorphin, Cyclo(Leu-Gly), Dermorphin, Met-Enkephalin, Methorphamid
   (Tyr-Gly-Gly-Phe-Met-Arg-Arg-Val), Morphiceptin, Morphine modulierendes Neuropeptid (Ala-Gly-Glu-Gly-Leu-Ser-Ser-Pro-Phe-Trp-Ser-Leu-Ala-Ala-Pro-Gln-Arg-Phe-NH2) usw.;
- mindestens ein Muskelrelaxans, häufig aus den Gruppen von kompetitiv oder depolarisierend wirkenden Curare-Stoffen, Myotonolytika oder Analgetica. Zu geeigneten Stoffen mit dieser Wirkung gehören z.B. Acetylsalicilsäure, Alcuronium-chlorid, Azapropazon, Atracuriumbesilat, Baclofen, Carisoprodol, Chininderivate, Chlormezanon, Chlorphenesincarbamat, Chlorzoxazon, Dantrolen, Decamethoniumbromid, Dimethyltubocurariniumchlorid, Fenyramidol, Gallamintriethiodid, Guaiphensin, Hexafluoreniumbromid, Hexacarbacholinbromid, Memantin, Mephenesin, Meprobamat, Metamisol, Metaxalon, Methocarbamol, Orphenadrin, Paracetamol, Phenazon, Phenprobamat, Suxamethoniumchlorid, Tetrazepam, Tizanidin, Tubocurarinchlorid, Tybamat, usw.;
- mindestens ein Narkoticum, z.B. Alfentanil, Codein, Droperidol, Etomidat, Fentanil, Flunitrazepam, Hydroxybuttersäure, Ketamin, Methohexital, Midazolam, Thebacon, Thiamylal, Thiopental, usw. und die entsprechenden Derivate;
- mindestens einen Stoff mit neuraltherapeutischer Wirkung wie z.B. Anästhetica und Vitamine, Atropin-Derivate, Benfotiamin, Cholin-Derivate, Coffein, Cyanocobolamin, alpha-Liponsäure, Mepivacain, Phenobarbital, Scopolamin, Thiaminchloridhydrochlorid, usw., und ganz besonders Procain;
- mindestens ein Neurolepticum, z.B. Butyrophenon-Derivate, Phenotiazin-Derivate, trizyklische Neuroleptika, ferner Acetophenazin, Benperidol, Butaperazin, Carfenazin, Chlorpromazin, Chlorprothixen, Clopenthixol, Clozapin, Dixyrazin, Droperidol, Fluanison, Flupentixol, Fluphenazin, Fluspirilen, Haloperidol, Homofenazin, Levomepromazin, Melperon, Moperon, Oxipertin, Pecazin, Penfluridol, Periciazin, Perphenazin, Pimozid, Pipamperon, Piperacetazin, Profenamin, Promazin, Prothipendyl, Sulforidazin, Thiopropazat, Thioproperazin,
   Thioridazin, Tiotixen, Trifluoperazin, Trifluperidol, Triflupromazin, usw. Besonders häufig werden Haloperidol und Sulperid verwendet;
- mindestens einen Neurotransmitter oder seinen Antagonisten. Vorzugsweise werden Acetylcholin, Adrenalin, Curare (und z.B. sein Antagonist Edrophonium-chlorid), Dopamin, Epehdrin, Noradrenalin, Serotonin, Strychnin, Vasotonin, Tubocurarin, Yohimbin, usw., verwendet;
- mindestens ein Opthalmicum, häufig aus den Gruppen der Anästhetica, Antibiotica, Corticoida, Augentonica, Chemotherapeutica, Glaukommittel, Virustatica, Antiallergica, eine gefäßerweiternde Substanz, oder ein Vitamin;
- mindestens ein Parasympathicomimeticum (z.B. Bethanechol-chlorid, Carbachol, Demecarium-bromid, Distigminbromid, Pyridostigmin-bromid, Scopolamin) oder ein Parasympathicolyticum (wie z.B. Benzatropin, Methscopolamin-bromid, Pilocarpin oder Tropicamid);
- mindestens ein Mittel zur Behandlung von Psoriasis und/oder Neurodermitis. Bevorzugt werden hypoallergisch wirkenden Träger oder randaktive Stoffe mit n-3 (omega 3), seltener mit oder n-6 (omega 6), mit zumeist mehreren, häufig 3-6 Doppelbindungen und/oder Hydroxy, seltener Methyl-, oder Oxo-Seitengruppen; diese können auch als Seitenketten an weiteren Wirkstoffmolekülen auftreten. Seitengruppen am 15-Kohlenstoffatom sind besonders wirksam. Als zusätzliche Wirkstoffe können unter anderem auch Antimycotica, Cytostatica, Immunsuppressiva oder Antibiotica verwendet werden;
- mindestens ein pupillenerweiterndes Medikament (Mydriaticum), wie z.B. Atropin, Atropinmethonitrat, Cyclopentolat, Pholedrin, Scopolamin oder Tropicamid;
- mindestens einen Stoff mit psychostimulierender Wirkung. Gut geeignet für solche Anwendung sind z.B. Amphetaminil, Fencamfamin, Fenetyllin, Meclofenoxat, Methamphetamin, Methylphenidat, Pemolin, Phendimetrazin, Phenmetrazin, Prolintan oder Viloxazin;
- mindestens ein Rhinologicum, wie z.B. Buphenin, Cafaminol, Carbinoxamid, Chlorphenamim, Chlortenoxazin, Clemastin, Dextromethorpan, Etilefrin, Naphazolin, Norephedrin, Oxymetazolin, Phenylaprhin, Piprinydrinat, Pseudoephedrin, Salicylamid, Tramazolin, Triprolidin, Xylometazolin, usw, und aus biologischen Quellen besonders Radix Gentiane Extrakt;
- mindestens ein Schlafmittel (wie z.B. schlafinduzierendes Peptid (Trp-Ala-Gly-Gly-Asp-Ala-Ser-Gly-Glu)), oder einen Schlafmittel-Antagonisten (wie z.B. Bemegrid);
- mindestens ein Sedativum oder ein Beruhigungsmittel (Tranquilizer), z.B. als Sedativa Acecarbromal, Alimemazin, Allobarbital, Aprobarbital, Benzoctamin, Benzodiazepin-Derivate, Bromisoval, Carbromal, Chlorpromazin, Clomethiazol, Diphenyl-Methan-Derivate, Estazolam, Fenetyllin, Homofenazin, Mebutamat, Mesoridazin, Methylpentynol, Methylphenobarbital, Molindon, Oxomemazin, Perazin, Phenobarbital, Promethazin, Prothipendyl, Scopolamin, Secbutabarbital, Trimetozin, usw. und als Tranquilizer Azacyclonol, Benactyzin, Benzoctamin, Benzquinamid, Bromazepam, Chlordiazepoxid, Chlorphenesincarbanat, Cloxazolam, Diazepam, Dikalium-chlorazepat, Doxepin, Estazolam, Hydroxyzin, Lorazepam, Medazepam, Meprobamat, Molindon, Oxazepam, Phenaglycodol, Phenprobamat, Prazepam, Prochlorperazin, Rescinnamin, Reserpin oder Tybamat. Auch Drogen, wie z.B. Distraneurin, Hydantoinderivate, Malonylharnsäure-Derivate (Barbiturate), Oxazolidin-Derivate, Scopolamin, Valepotriat, Succinimid-Derivate, oder Hypnotika (z.B. Diureide (wie Barbiturate)), Methaqualon, Meprobromat, Monoureide (wie Carbromal), Nitrazepam, oder Piperidin-dione, können für diesen Zweck verwendet werden. Als Antidepressiva werden bevorzugt unter anderem Thymoleptika, wie z.B. Librium oder Tofranil, benutzt;
- einen Stoff aus der Klasse der Spasmolytica, z.B. Adiphenin, Alverin, Ambicetamid, Aminopromazin, Atropin, Atropinmethonitrat, Azintamid, Bencyclan, Benzaron, Bevonium-methylsulfat, Bietamiverin, Butetamat, Butylscopolammoniumbromid, Camylofin, Carzenid, Chlordiazepoxid, Cionium-bromid, Cyclandelat, Cyclopentolat, Dicycloverin, Diisopromin, Dimoxylin, Diphemanil-methylsulfat, Ethaverin, Ethenzamid, Fencarbamid, Fenpipramid, Fenpivennum-bromid, Gefarnat, Glycopyrroniumbromid, Hexahydroadiphenin, Hexocycliummethylsulfat, Hymecromon, Isomethepten, Isopropamidjodid, Levomethadon, Mebeverin, Metamizon, Methscopolamin-bromid, Metixen, Octatropin-methylbromid, Oxazepam, Oxybutin, Oxyphenonium-bromid, Papaverin, Paracetamol, Pentapiperid, Penthienat-methobromid, Pethidin, Pipenzolat-bromid, Piperidolat, Pipoxolan, Propanthelin-bromid, Propylphenazon, Propyromazin-bromid, Racefemin, Scopolamin, Sulpirid, Tiemonium-jodid, Tridihexethyljodid, Tropenzilinbromid, Tropinbenzilat, Trospiumchlorid, Valethamatbromid, usw.; ferner Belladona Alkaloide, Papaverin und seine Derivate, usw.;
- mindestens ein Sympathicolyticum, z.B. Azapetin oder Phentolamin;
- mindestens ein Sympathicomimeticum, z.B. Bamethan, Buphenin, Cyclopentamin, Dopamin, L-(-)-Ephedrin, Epinephrin, Etilefrin, Heptaminol, Isoetarin, Metaraminol, Methamphetamin, Methoxamin, Norfenefrin, Phenylpropanolamin, Pholedrin, Propylhexedrin, Protokylol oder Synephrin;
- mindestens ein Tuberkulostaticum, z.B. Antibiotica, p-Aminosalicylsäure, Capreomycin, Cycloserin, Dapson, Ethambutol, Glyconiazid, Iproniazid, Isoniazid, Nicotinamid, Protionamid, Pyrarinamid, Pyrodoxin, Terizidon, usw., davon ganz besonders bevorzugt Ethambitol und Isoniazid;
- mindestens ein Urologicum, z.B. ein Blasenatoniemittel (wie Cholincitrat, Distigminbromid, Yohimbin), ein Harninfektionstherapeuticum (Antibioticum, Chemotherapeuticum, bzw. Nitrofurantoid-, Chinolon-, oder Sulfonamid-Derivat oder); ferner Adipinsäure, Methionin, Methenamin-Derivate, usw.;
- mindestens einen Stoff, der zu den Vasoconstrictoren zählt. Häufig werden für diesen Zweck Adrenalon, Epinephrin, Felypressin, Methoxamin, Naphazolin, Oxymetazolin, Tetryzolin, Tramazolin ode Xylometazolin benutzt;
- mindestens einen Stoff, der ein Vasodilatator ist, wie beispielsweise Azapetin, Banethan, Bencyclan, Benfurodilhemisuccinat, Buphenin, Butalamin, Cinnarizin, Diprophyllin, Hexyltheobromin, Ifenprodil, Isoxsuprin, Moxisylyt, Naftidrofuryl, Nicotinylalkohol, Papaverin, Phenoxybenzamin, Piribedil, Primaperon, Tolazolin, Trimetazidine, Vincamin oder Xantinol-nicotinat;
- mindestens ein Venenmittel, z.B. Aescin, Benzaron, Calcium-Dobesilat, Dihydroergotaminmesilat, Diosmin, Hyydroxyethylrutosid, Pignogenol, Rutosid-aesinat, Tribenosid, Troxerutin, usw.;
- mindestens ein Virustaticum, z. B. immunstimulierende Präparate, die durch die Verwendung von zusätzlichen Medikamenten, wie z.B. Moroxydin oder Tromantadin noch wirksamer sein können;
- ein Wundenbehandlungsmittel, z.B. Dexpanthenol; Wachstum stimulierende Faktoren, Enzyme oder Hormone, besonders wenn sie in Kombination mit Trägern, die essenziellen Stoffe enthalten, sind jedoch zumeist noch wirksamer. Auch Povidon-Jod, ungeradkettige Fettsäuren, Cetylpyridiniumchlorid, Chinolin-Derivate bekannter Antibiotica und Analgetica sind nützlich.
- mindestens einen Stoff, der toxisch wirkt oder selbst ein Toxin ist;
   Toxine aus pflanzlichen oder mikrobiellen Quellen, insbesondere 15-Acetoxyscirpenol, 3-Acetyldeoxynivalenol, 3alpha-Acetyldiacetoxyscirpenol, Acetyl T-2 toxin, Aflatoxicol I, Aflatoxicol II, Aflatoxin B1, Aflatoxin B2, Aflatoxin B2alpha, Aflatoxin G1, Aflatoxin G2, Aflatoxin G2alpha, Aflatoxin M1, Aflatoxin M2, Aflatoxin P1, Aflatoxin Q1, Alternariol monomethyl ether, Aurovertin B, Botulinum toxin D, Cholera toxin, Citreoviridin, Citrinin, Cyclopiazonsäure, Cytochalasin A, Cytochalasin B, Cytochalasin C, Cyrochalasin D, Cytochalasin, Cytochalasin H, Cytochalasin J, Deoxynivalenol, Diacetoxyscirpenol, 4,15-Diacetylverrucarol, Dihydrocytochalasin B, Enterotoxin STA, Fusarenon X, Iso T-2 Toxin, O-Methylsterigmatocystin, Moniliformin, Monoacetoxyscirpenol, Neosolaniol, Ochratoxin A, Patulin, Penicilinsäure, Pertussis toxin, Picrotoxin, Pr-toxin, Prymnesin, Radicinin, Roridin A, Rubratoxin B, Scirpentriol, Secalonsäure D, Staphylococcalenterotoxin B, Sterigmatocystin, Streptolysin O, Streptolysin S, Tentoxin, Tetrahydrodeoxyaflatoxin B1, Toxin A, Toxin II, HT-2 toxin, T-2-tetraol, T-2 toxin, Trichothecin, Trichothecolon, T-2 triol, Verrucarin A, Verrucarol, Vomitoxin, Zearalenol und Zearalenon.
- mindestens eine bei Mensch und Tier wachstumsbeeinflußende Substanz, z.B. Basic Fibroblast Growth Factor (bFGF), Endothelial Cell Growth Factor (ECGF), Epidermal Growth Factor (EGF), Fibroblast Growth Factor (FGF), Insulin, Insulin-like Growth Factor I (IGF I), Insulin-like Growth Factor II (IGFII), Nerven Wachstums-Faktorbeta (NGFbeta), Nerven Wachstums-Faktor 2,5S (NGF 2,5S), Nerven Wachstums-Faktor 7S (NGF 2,5S), Wachstums-Faktor aus Plättchen (Platelet-Derived Growth Factor (PDGF)), usw.;
- einen Träger und/oder Wirkstoff, der auf und in der Barriere, z.B. Haut, eine Schutzschicht gegen Gift, Licht-, UV-, gamma - bzw. sonstige Strahlung oder gegen biologischen Schadstoffe, wie z.B. Viren, Bakterien, Toxine, usw., bildet. Die Träger und/oder Wirkstoffe können dabei die schädliche Wirkung chemisch, biochemisch oder biologisch hemmen, oder aber die Penetration solcher Schadstoffe verringern oder verhindern;
- mindestens ein Fungizid, Herbizid, Pestizid, oder Insektizid;
- mindestens ein Pflanzenhormon, z.B. Abscisinsäure, Abscisinsäure-Methylester, 3-Acetyl-4-thiazolidine-carboxylsäure, 1-Allyl-1-(3,7-dimethyloctyl)-piperidinium bromid, 6-Benzylaminopurin, 6-Benzylaminopurin 9-(beta - glucosid), Butanediosäure mono(2,2-dimethyl hydrazid), Chlorocholin chlorid, 2-Chloroethyl-tris-(2'-methoxyethoxy)silan, 2-(o-Chlorophenoxy)-2-methylpropionsäure, 2-(p-Chlorophenoxy)-2-methylpropionsäure, 2-(o-Chlorophenoxyipropionsäure, 2-(m-Chlorophenoxy)propionsäure, Clofibrinsäure, Colchicin, o-coumarinsäure, p-coumarinsäure, Cycloheximid, alpha,beta-dichloroisobuttersäure, 2-(2,4-dichlorophenoxy)propanolsäure, 2,3-dihydro-5,6-diphenyl 1,4-oxathiin, Dihydrozeatin, 6-(gamma,gamma-Dimethylallylamino)purin ribosid, 3-(2-[3,5-Dimethyl-2-oxocyclohexyl-2-hydroxyethyl])-glutarimid, Trans-2-dodecenediosäure, Ethyl-8-chloro-1 H-indazol-3-yl-acetat, N6-Furfuryladenosin, 6-Furfurylaminopurinribosid, Gibberellinsäure Methylester, Gibberellin A3-Acetat, Gibberellin A1 Methylester, Gibberellin A4 Methylester, Gibberellin A5 Methylester, Gibberellin A7 Methylester, Gibberellin A9 Methylester, Gibberellin A3 Methylester 3,13-diacetat gibberinsäure, Allo-gibberinsäure, Gibberinsäure Methylester, Glyoxim, 22(S),23(S)-Homobrassinolid, 9-Hydroxyfluoren 9-Carboxylat, Indol-3-acetsäure, Indol-3-acetsäure ethylester, Indol-3-propanosäure, N6-(2-isopentenyl)adenin, N6-(2-isopentenyl)adenosin, 2-Isopropyl-4-dimethylamino-5-methylphenyl-1-piperidine-carboxylat Methylchlorid, Kinetinglucosid, Kinetinribosid, Melissylalkohol, 1-Methyladenin, Methyl 2-chloro-9-hydroxy-fluorene-9-carboxylat, Melhyl 3,6-Dichloro-O-anisat, 6-Methylmercaptopurin, 1-Naphthylacetamid, Nonanosäure Methylester, 6-Piperidino-1-purin, N-Triacontanol, (-)-Xanthoxin, Zeatin glucoside, etc.;
- mindestens ein Pheromon oder einen pheromonähnlichen Stoff, unter anderen (-)-Bornyl Acetat, trans-5-Decenol, cis-5-Decenyl Acetat, trans-5-Decenyl Acetat, 2,6-Dichlorophenol, 1,7-Dioxaspiro[5.5]undecan, trans-8,trans-10-Dodecadienol([E,E]-8,10-DDDOL), trans-7,cis-9-Dodecadienyl Acetat ([E,Z]-7,9-DDDA), trans-8,trans-10-Dodecadienyl Acetat ([E,E]-8,10-DDDA), cis-7-Dodecen-1-ol (Z-7-DDOL), trans-10-Dodecenol, cis-7-Dodecenyl Acetat (Z-7-DDA), cis-8-Dodecenyl Acetat, trans-8-Dodecenyl Acetat, trans-8-Dodecenyl Acetat, 11-Dodecenyl Acetat, cis-7,8-Epoxy-2-methyl-octadecan, cis-9-Heneicosen, cis-7,cis-11-Hexadecadienylacetat ([Z,Z]-7,11-HDDA), cis-7,trans-11- Hexadecadienyl Acetat ([Z,E)-7,11-HDDA), cis-9-Hexadecenal (Z-9-HDAL), cis-11-Hexadecenal (Z-11-HDAL), cis-11-Hexadecenol (Z-11-HDOL), cis-11-Hexadecenyl Acetat (Z-11-HDA), trans-2-Hexenyl Acetat, cis-7-Tetradecenal (Z-7-TDAL), cis-9-Tetradecenol (Myristoleyl alcohol; Z-9-TDOL), cis-7-Tetradecenol (Z-7-TDOL), cis-11-Tetradecenol, cis-7-Tetradecenyl Acetat (Z-7-TDA), cis-9-Tetradecenyl Acetat (Myristoleyl Acetat; Z-9-TDA), cis-11-Tetradecenyl Acetat (Z-11-TDA), trans-11-Tetradecenyl Acetat (E-11-TDA), cis-9-Tetradecenyl formate (Myristoleyl-Format; Z-9-TDF), isoamyl Acetat (acetic acid 3-methylbutyl ester), 2-Methyl-3-buten-2-ol, 3-Methyl-2-cyclohexen-1-ol, cis-14-Methyl-8-Hexadecenal, cis-2-Methyl-7-octadecen, 4-Methylpyrrole-2-carboxylsäuremethyl Ester (Methyl 4-methylpyrrole 2-carboxylate) cis-13-octaDecemal 13-Octadecyn-1-ol, 2-(Phenyl)ethyl propionate (Phenylethanolpropanoat), Propyl cyclohexylacetat, cis-9,trans-11-Tetradecadienol ([Z,E]-9,11-TDDOL), cis-9,trans-11-Tetradecadienyl Acetat ([Z,E]-9,11-TDDA), cis-9,trans-12-Tetradecadienyl Acetat ([Z,E]-9,12-TDDA), Trichloroessigsäure Ester, cis-9-Tricosen, Undecanal, etc.;
- mindestens einen Farbstoff;
- mindestens ein Kohlenhydrat;.
   Ein Kohlenhydrat hat normalerweise die Grundformel Cₓ(H₂O)_{y}, wie z.B. in Zucker, Stärke, Zellulose, kann aber auch auf vielfaltige Weise derivatisiert sein.
   Ein monomerer Kohlenhydratrest ist beispielsweise ein natürlicher Monosaccharidrest, der zumeist ein Addukt einer als Aldose oder Ketose vorliegenden Pentose oder Hexose ist und im Prinzip in L- oder D-Konfiguration vorliegen kann. Aus Platzgründen, und wegen deren besonderer biologischen Relevanz, sind die folgenden Aufzählungen lediglich auf die zweitgenannten beschränkt.
   Eine Aldose mit fünf Kohlenstoffatomen (Aldo-Pentose, oder einfach Pentose) ist z.B. D-Arabinose, D-Lyxose, D-Ribose oder D-Xylose.
   Eine Ketose mit fünf Kohlenstoffatomen (Keto-Pentose) ist z.B. D-Ribulose oder D-Xylulose.
   Eine Aldose mit sechs Kohlenstoffatomen (Aldo-Hexose, auch einfach Hexose) ist z.B. D-Allose, D-Altrose, D-Galactose, D-Glucose, D-Mannose oder D-Talose. Eine Ketose mit sechs Kohlenstoffatomen (oder einfach KetoHexose) ist z.B. D-Fructose, D-Psicose, D-Sorbose oder D-Tagatose.
   Eine Hexose befindet sich besonders häufig in cyklischer Form, liegt z.B. als Pyranose (Aldose) vor; alpha- oder beta-D-Glucopyranose sind zwei Beispiele dafür. Ein weiterer Hexose-Typ ist Furanose, beispielsweise in einer alpha- oder beta-D-Fructose. Der Pyranosylrest ist vorzugsweise durch eine Hydroxygruppe verestert, die sich in der 1- oder 6-Stellung befindet; der Furanosylrest ist vorzugsweise durch entsprechende Gruppen in 1- oder 5-Stellung verestert.
   Ein Kohlenhydratrest ist ferner ein natürlicher Disaccharidrest, z.B. ein aus zwei Hexosen gebildeter Disaccharidrest. Ein solcher Disaccharidrest entsteht beispielsweise durch Kondensation von zwei Aldosen, z.B. D-Galactose oder D-Glucose, oder einer Aldose, z.B. D-Glucose mit einer Ketose, z.B. Fructose. Aus Zwei Aldosen gebildete Disaccharide, z.B. Lactose oder Maltose, sind vorzugsweise über die Hydroxygruppe, die sich in 6-Stellung des betreffenden Pyranosylrests befindet, mit der Phosphatidylgruppe verestert. Aus einer Aldose und einer Ketose gebildete Disaccharide, z.B. Saccharose, sind vorzugsweise über die in 6-Stellung des Pyranosylrests oder über die in 1-Stellung des Furanosylrest befindliche Hydroxygruppe verestert.
   Ein Kohlenhydratrest ist außerdem ein derivatisierter Mono-, Di- oder Oligosaccharidrest, worin beispielsweise die Aldehydgruppe und/oder ein oder zwei endständige Hydroxygruppen zu Carboxygruppen oxydiert siud, z.B. ein D-Glucar-, D-Glucon- oder D-Glucoronsäurerest, welche vorzugsweise als zyklische Lactonreste vorliegen. Ebenso können in einem derivatisierten Mono- oder Disaccharidrest Aldehyd- oder Ketogruppen zu Hydroxygruppen reduziert sein, z.B. in Inosit, Sorbit oder D-Mannit. Ferner können die Hydroxygruppen durch Wasserstoff, z.B. in Desoxyzucker, wie 2-Desoxy-D-ribose, L-Fucose oder L-Rhamnose, oder durch Aminogruppen, z.B. in Aminozucker, wie D-Galactosamin oder D-Glucosamin, ausgetauscht sein.
   Ein Kohlenhydrat kann auch ein Spaltprodukt sein, daß sich durch Umsetzung eines der genannten Mono- oder Disaccharide mit einem starken Oxidationsmittel, z.B. Perjodsäure, gebildet hat. Zu den wichtigen biologisch aktiven oder biologisch bedeutenden Kohlenhydraten gehören z.B. 2-Acetamido-N-(epsilon-amino-caproyl)-2-deoxy-betagluccopyranosylamin, 2-Acetamido-1-amino-1,2-dideoxy-beta-glucopyranose, 2-Acetamido-1-beta-(aspartamido)-1,2-dideoxyglucose, 2-Acetamido-4,6-O-benzyliden-2-deoxy-beta-glucopyranose, 2-Acetamido-2-deoryallose, 3-Acetamido-3-deoxyallose, 2-Acetamido-2-deoxy-3-O-(beta-galactopyranosyl)-galactopyranose, 2-Acetamido-2-deoxy-4-O-([4-O-beta-galactopyranosyl-beta-galactopyranosyl]-beta-galactopyranosyl)-glucopyranose, 2-Acetamido-2-deoxy-3-O-(beta-galactopyranosyl)-alpha - glucopyranose, 6-O-(2-acetamido-2-deoxy-4-O-[beta-galactopyranosyl]-beta-glucopyranosyl)-galactopyranose, 4-O-Acetamido-2-deoxy-6-O-(beta-galacto-4-O-(6-O-[2-acetamido-2-deoxy-beta-glucopyranosyl]-beta - galactopyranosyl) glucopyranose, 2-Acetamido-2-deoxygalactose, 2-Acetamido-2-deoxyglucose, 3-Acetamido-3-deoxyglucose pyranose, 6-O-(2-acetamido-2-deoxy-beta-glucopyranosyl)-galactopyranose, 2-Acetamido-2-deoxy-1-thio-beta-glucopyranose 3,4,6-triacetat, Acetopyruvat Säure, N-Acetylchondrosamin, N-Acetylgalactosamin, N-Acetylglucosamin, N-Acetyl-alpha-glucosamin 1-phosphat, N-Acetylglucosamin 6-phosphat, N-Acetylglucosamin 3-sulfat, N-Acetylglucosamin 6-sulfat, N-Acetylheparin, N-Acetyllactosamin, N-Acetyl-beta-mannosamin, N-Acetylneuramin Säure, N-Acetylneuramin-lactose, 1-O-Acetyl-2,3,5-tri-O-benzoyl-beta-ribofuranose, trans-Aconit Säure, Adenine-9-beta-arabinofuranosid, Adenosin 5'-diphospho-glucose, Adenosin 5'-diphosphomannose, Adonit, Adonitol, Adonose, Agar, Algin, Algin Säure, Beta-allose, Alpha glycerophosphat, Alpha ketoglutar Säure, Altrose, (-)-Altrose, p-Aminobenzyl-1-thio-2-acetamido-2-deoxy-beta-glucopyranosid, N-epsilon-Aminocaproyl-beta-fucopyranosylamin, N-epsilon- minocaproyl-alpha-galactopyranosylamin, 2-Amino-2-deoxygalactopyranose, 6-Amino-6-deoxyglucopyranose, 1-Amino-1-deoxy-beta-glucose, 6-Aminohexyl-N-acetyl-beta-thioglucosaminid, 6-Aminohexyl-1-thio-beta-galactopyranosid, 5-Aminoimidazole-4-carboxamidoxime-1-beta-ribofuranosyl 3':5'-cyclo-Monophosphat, delta-Aminolevulin Säure, p-Aminophenyl-2-acetamido-2-deoxy-beta-glucopyranosid, p-Aminophenyl-2-acetamido-2-deoxy-1-thio-beta-glucopyranosid, p-Aminophenyl-alpha-fucopyranosid, p-Aminophenyl-alpha-galactopyranosid, p-Aminophenyl-beta-galactopyranosid, p-Aminophenyl-alpha-glucopyranosid, p-Aminophenyl-beta -glucopyranosid, C-Aminophenyl-beta-glucuronid, p-Aminophenyl-1-thio-beta-glucuronid, p-Aminophenyl-beta-lactopyranosid, p-Aminophenyl-alpha-mannopyranosid, p-Aminophenyl-beta-thiofucopyranosid, p-Aminophenyl-1-thio-beta-galactopyranosid, p-Aminophenyl-1-thlobeta-glucopyranosid, p-Aminophenyl-1-thlo-beta-xylopyranosid, p-Aminophenyl-beta-xylopyranosid, 5-Amino-1-(beta-ribofuranosyl)imidazole 4-carboxamid, Amygdalin, N-amyl beta-glucopyranosid, Amylopectin, Amylose, Apigenin 7-O-hesperidosid, Arabinitol, Arabinocytidin, 9-beta - Arabinofuranosyladenin, 1-beta-Arabinofuranosylcytosin, Arabinose, Arabinose 5-phosphaT, Arabinosylcytosin, Arabit, Arabitol, Arbutin, Atp-ribose, Atractylosid, Aurothioglucose, n-Butyl 4-O-beta -galactopyranosyl-beta-glucopyranosid, Calcium gluconat, Calcium heptagluconat, Carboxyatractylosid, Carboxymethylamylose, Carboxymethylcellulose, Carboxyethylthioethyl-2-acetamido-2-deoxy- 4-O-beta-galacto pyransol-beta-glucopyranosid, Carboxyethylthioethyl 4-O-(4-O-[6-O-alpha-glucopyranosyl-alpha-glucopyranosyl]-alpha-glucopyranosyl)-beta-glucopyranosid, 4-O-(4-O-[6-O-beta-D-Galactopyranosylbeta-D-galactopyranosyl]-D-glucopyranose, Carrageenan, D(+)Cellobiose, D(+)Cellopentaose, D(+)Cellotetraose, D(+)Cellotriose, Cellulose, Cellulose caprat, Cellulose carbonat, Chitin, Chitobiose, Chitosan, Chitotriose, alpha-Chloralose, beta-Chloralose, 6-Chloro-6-deory-alpha-glucopyranose, Chondroitin sulfat, Chondrosamin, Chondrosin, Chrysophan Säure, Colomin Säure, Convallatoxin, alpha-Cyclodextrin, beta-Cyclodextrin, Cytidin 5'-diphosphoglucose, Cytosin 1-beta-arabinofuranosid, Daunosamin, n-Decyl-beta-glucopyranosid, 5-Deoxyarabinose, 2-Deoxy-2-fluoroglucose, 3-Deoxy-3-fluoroglucose, 4-Deoxy-4-fluoroglucose, 6-Deoxygalacto pyranose, 2-Deoxygalactose, 1-Deoxyglucohex-1-eno-pyranose tetrabenzoat, 2-Deoxyglucose, 6-Deoxyglucose, 2-Deoxyglucose 6-phosphat, 1-Deoxymannojerimycin, 6-Deoxymannose, 1-Deoxy-1-morpholinofructose, 1-Deoxy-1-nitroalutol, 1-Deoxy-1-nitroaltitol, 1-Deoxy-1-nitrogalactitol, 1-Deoxy-1-nitromannitol, 1-Deoxy-1-nitrosorbitol, 1-Deoxy-1-nitrotalitol, Deoxynojirimycin, 3-Deoxy-erythro-pentose, 2-Deoxy-6-phosphoglucon Säure, 2-Deoxyribose, 3-Deoxyribose, 2-Deoxy-alpha-risose 1-phosphat, 2-Deoryribose 5-phosphat, 5-Deoxyxylofuranose, Dextran, Dextransulfat, Dextrin, Dextrose, Diacetonefructose, Diacetonemannitol, 3,4-Di-O-acetyl-6-deoxyglucal, Di-O-acetylrhamnal, 2,3-Diamino-2,3-dideoxy-alpha-glucose, 6,9-Diamino-2-ethoxyacridin lactat, 1,3:4,6-Di-O-benzylidenemannitol, 6,6'-Dideoxy-6,6'-difluorotrehalose, Digalactosyl Diglycerid, Digalacturon Säure, (+)Digitoxose, 6,7-Dihydrocoumarin-9-glucosid, Dihydroxyaceton, Dihydroxyaceton phosphat, Dihydroxyfumar in Säure, Dihydroxymale Säure, Dihydroxytartar Säure, Dihydrozeatinribosid, 2,3-Diphosphoglycerol Säure, Dithioerythritol, Dithiothreitol, n-Dodecyl beta-glucopyranosid, n-Dodecyl beta-maltosid, Dulcitol, Elemigummi, Endotoxin, Epifucose, Errthritol, erythro-Pentulose, Erythrose, Erythrose 4-phosphat, Erythrulose, Esculin, 17-beta-Estradiol-3-glucuronid 17-sulfat, Estriol glucuronid, Estron beta-glucuronid, Ethodin, Ethyl 4-O-beta-D-Galactopyranosyl)-beta-D-glucopyranosid, Ethyl2-acetamido-4-O-(2-acetamido-2-deoxy-beta-glucopyranosyl)-6-O-(alpha -fucopyranosyl)-2-deoxy-beta-glucopyranosid, Ethyl2-acetamido-2-deoxy-4-O-(4-O-alpha-galactopyranosyl-beta-galactopyranosyl)-beta-glucopyranosid, Ethyl cellulose ethylen glycol chitln, Ethyl 4-O-(4-O-alpha-galacto-pyranosyl-beta-galactopyranosyl)-beta-glucopyranosid, Ethyl 4-O-beta-galactopyranosyl-beta-glucopyranosid, Ethyl pyruvat, Ethyl beta -thioglucosid, Etiocholan-3alpha-ol-17-on glucuronid, Ficoll, 6-Fluoro-6-deoxyglucose, Frangulosid, Fraxin, Fructosazin, beta -(-)Fructose, Fructose-1,6-diphosphat, Fructose-2,6-diphosphat, Fructose-1-phosphat, Fructose-6-phosphat, Fucoidan, Fucose, alpha -(-)-Fucose-1-phosphat, Fucosylamin, 2'-Fucosyllactose, 3-Fucosyllactose, Fumarat Säure, Galactal, Galactitol, Galactopyranosylamin, 3-O-beta -Galactopyranosyl-arabinose, 4-O-beta-Galactopyranosyl-fructofuranose, 4-O-(4-Obeta-Galactopyranosyl beta-galactopyranosyl)-glucopyranose, 4-O-alpha-Galactopyranosylgalactopyranose, 6-Obeta-Galactopyranosylgalactose, 4-O-(beta-Galactopyranosyl)-alpha-mannopyranose, alpha-Galactopyranosyl 1-phosphat, Galactopyranosyl-beta-thiogalactopyranosid, (+)Galactosamin, alpha-Galactosamin 1-phosphat, alpha-Galactose 1-phosphat, Galactose 6-phosphat, Galactose 6-sulfat, 6-(alpha-Galactosido)glucose, Galacturon Säure, beta-Gentiobiose, Glucan, Glucitol, Glucohepton Säure, Glucoheptwe, Glucoheptulose, Gluconat 6-phosphat, Glucon Säure, 1-O-alpha-Glucopyranosyl-beta-fructofuranosid, 6-O-alpha-Glucopyranosylfructose, 1-O-alpha-Glucopyranosyl-alpha-glucopyranosid, 4-O-beta-Glucopyranosylglucopyranose, 4-O-(4-O-[6-O-alpha-Glucopyranosylalpha-glucopyranosyl]-alpha-glucopyranosyl) glucopyranose, (+)Glucosamin, alpha-Glucosamin 6-2,3-disulfat, alpha-Glucosamin 1-phosphat, Glucosamin 6-phosphat, Glucosamin 2-sulfat, alpha-Glucosamin 3-sulfat, Glucosamin 6-sulfat, Glucosamin Säure, Glucose, alpha-Glucose 1,6-diphosphat, Glucose 1-phosphat, Glucose 6-phosphat, Glucose 6-sulfat, Glucuronamid, Glucuron Säure, alpha-Glucuron Säure 1-phosphat, Glyceraldehyd, Glyceraldehyd 3-phosphat, Glycerat 2,3-diphosphat, Glycerat 3-phosphat, Glyceral Säure, alpha-Glycerophosphat, beta-Glycerophosphat, Glycogen, Glycolaldehyd, Glycol chitosan, N-glycolylneuramin Säure, Glycyrrhiz Säure, Glyoxyl Säure, Guanosin, 5'-diphosphoglucose, Gulose, Gummis (accroides, Agar, Arab, Carrageenan, Damar, Elemi, Ghatti, Guaiac, Guar, Karaya, Locust bonne, Mast, Pontianak, Storax, Tragacanth, Xanthan), Heparin und heparinähnliche Substanzen (Mesoglycan, Sulodexid, usw.), Heptakis (2,3,6-tri-O-methyl)-beta-cyclodextrin, Heptanoyl-N-methylglucamid, n-Heptyl beta-glucopyranosid, Hesperidin, N-Hexyl-beta-glucopyranosid, Hyaluron Säure, 16alpha-Hydroxyestronglucuronid, 16-beta-Hydroxyestron glucuronid, Hydroxyethyl Stärke, Hydroxypropylmethylcellulose, 8-Hydroxyquinolin-beta-glucopyranosid, 8-Hydroryquinolin glucuronid, Idose, (-)-Idose, Indole-3-lactat Säure, Indoxyl-beta-glucosid, epi-Inositol, myo-Inositol, myo-Inositol bisphosphat, myo-Inositol-1,2-cyl phosphat, scyllo-Inositol, Inositolhexaphosphat, Inositolhexasulfat, myo-Insoitol 2-monophosphat, myo-Inositol trisphosphat, (q)-epi-Inosose-2, scyllo-Inosose, Inulin, Isomaltose, Isomaltotriose, Isosorbid dinitrat, 11-Ketoandrosteron beta-glucuronid, 2-Ketoglucon Säure, 5-Ketoglucon Säure, alpha-Ketopropion Säure, Lactal, Lactat Säure, Lactitol, Lactobion Säure, Lacto-N-tetraose, Lactose, alpha-lactose 1-phosphat, Lactulose, Laminaribiose, Laminnarin, Levoglucosan, beta-levulose, Lichenan, Linamarin, Lipopolysaccharides, Lithiumlactat, Lividomycin a, Lyxose, Lyxosylamin, Maltitol, Maltoheptaose, Maltohexaose, Maltooligosaccharid, Maltopentaose, Maltose, alpha-(+)Maltose 1-phosphat, Maltotetraose, Maltotriose, Malvidin-3,5-diglucosid, Mandelonitril beta-glucosid, Mandelonitril glucuron-Säure, Mannan, Mannit, Mannitol, Mannitol 1-phosphat, alpha-mannoheptitol, Mannoheptulose, 3-O-alpha-Mannopyranosyl-mannopyranose, alpha(+)Mannopyranosyl-1-phosphat, Mannosamin, Mannosan, Mannose, a(+)Mannose 1-phosphat, Mannose 6-phosphat, (+)Melezitose, a(+)Melibiose, Mentholglucuron Säure, 2-(3'-Methoxyphenyl)-N-acetylneuramin Säure, Methyl 3-O-(2-acetamido-2-deoxy-beta-galactopyranosyl)-alpha-galactopyranosid, Methyl 4-O-(3-O-[2-acetamido-2-deoxy-4-O-beta-galactopyranosyl beta-glucopyranosyl]-beta-galactopyranosyl)-betaglucopyranosid, Methyl 2-acetamido-2-deoxy-beta-glucopyranosid, Methyl3-O-(2-acetamido-2-deoxy-beta-glucopyranosyl)-beta-galactopyranosid, Methyl6-O-(2-acetamido)-2-deoxy-beta-glucopyranosyl)-alpha-mannopyranosid, Methyl acosaminid, Methyl alpha-altropyranosid, Methyl3-amino-3-deoxy-alpha-mannopyranosid, Methyl betaarabinopyranosid, Methyl 4,6-O-benzyliden-2,3-di-O-toluenesulfonyl-alpha-galactopyranosid, Methyl 4,6-0-benzylidene-2,3-di-O-p-toluenesulfonyl-alpha-glucopyranosid, Methyl cellulose, Methyl alpha-daunosaminid, Methyl6-deory-alpha-galactopyranosid, Methyl 6-deoxy-beta-galactopyranosid, Methyl 6-deoxy-alpha-glucopyranosid, Methyl 6-deoxy-beta-glucopyranosid, Methyl 3,6-di-O-(alpha-mannopyranosyl)-alpha-mannopyranosid, 1-O-Methyl-alpha-galactopyranosid, 1-O-Methyl-beta-galactopyranosid, Methyl 3-O-alpha-galactopyranosyl-alphagalactopyranosid, Methyl-3-O-beta-galactopyranosyl-beta-galactopyranosid, 4-O-(2-O-Methyl-beta-galactopyranosyl) glucopyranose, Methyl 4-O-beta-galactopyranosyl-beta-glucopyranosid, Methyl-4-O-(beta-galactopyranosylalpha-mannopyranosid, 5-5-Methylgalacto pyranose, Methylgalactosid, N-Methylglucamin, 3-O-Methyl-alpha-glucopyranose, 1-O-Methyl-alpha-glucopyranosid, 1-O-Methyl-beta-glucopyranosid, alpha-Methyl glucosid, beta-Methyl glucosid, Methyl glycol chitosan, Methyl-alpha- mannopyranosid, Methyl-2-O-alpha-mannopyranosyl-alphamannopyranosid, Methyl 3-O-alpha -mannopyranosyl-alpha-mannopyranosid, Methyl-4-O-alpha-mannopyranosylalpha-mannopyranosid, Methyl 6-O-alpha-mannopyranosyl-alpha-mannopyranosid, Methyl alpha-rhamnopyranosid, Methyl alpha-ribofuranosid, Methyl beta-ribofuranosid, Methylbeta-thiogalactosid, Methyl 2,3,5-tri-O-benzoylalpha-arabinofuranosid, 4-methylumbelliferyl2-acetamido-4,6-O-benzylidene-2-deoxy-beta-glucopyranosid, 4-Methylumbelliferyl N-acetyl-beta-galactosaminid, 4-methylumbelliferyl N-acetyl-alpha-glucosaminid, 4-methylumbelliferyl-N-acetyl-beta-glucosaminid, 4-methylumbelliferyl-alpha-arabinofuranosid, 4-methylumbelliferyl-alphaarabinopyranosid, 4-methylumbelliferyl-beta-cellobiosid, 4-methylumbelliferyl-beta -N,N'-diacetylchitobiosid, 4-methylumbelliferyl alpha-fucosid, 4-methylumbelliferyl beta-fucosid, 4-methylumbelliferyl alpha - galactopyranosid, 4-methylumbelliferyl beta-galactopyranosid, 4-methylumbelliferyl alpha-galactosid, 4-methylumbelliferyl beta -glucopyranosid, 4-methylumbelliferyl alpha-glucosid, 4-methylumbelliferyl beta-glucosid, 4-methylumbelliferyl beta -glucuronid, 4-methylumbelliferyl beta-mannopyranosid, 4-methylumbelliferylbeta-N,N',n"-triacetylchitotriose, 4-methylumbelliferyl2,3,5-tri-O-benzyl-alpha-arabinofuranosid, 4-methylumbelliferyl beta-xylosid, Methyl beta-xylopyranosid, 2-O-Methylxylose, alpha-Methylxylosid, beta-Methylxylosid, Metrizamid, 2'-Monophosphoadenosin 5'diphosphoribose, 2'-Monophosphoinosin 5'-diphosphoribose, Mucin, Muramin-Säure, Naringin, Natrium Lactat, Natrium Polypectat, Natrium Pyruvat, Neoagarobiose, Neoagarohexaitol, Neoagarohexaose, Neoagarotetraose, beta-Neocarrabiose, Neocarrabiose 4/1-sulfat, Neocarrahexaose(2/4,4/1,4/3,4/5)-tetrasulfat, Neocarratetraose (4/1,4/3)-disulfat, Neocarratetraose(4/1)-sulfat, Neohesperidin, Dihydrochalcon, Neohesperidose, Neuramin Säure, Neuramin Säure beta-methylglycosid, Neuramin-lactose, Nigeran, Nigerantetrasaccharid, Nigerose, n-Nonyl glucosid, n-Nonylbeta-glucopyranosid, Octadecylthioethyl 4-O-alpha-galactopyranosyl-beta -galactopyranosid, Octadecylthioethyl 4-O-(4-O-[6-O-alpha-glucopyranosyl-alpha-glucopyranosyl]-alpha-glucopyranosyl)-betaglucopyranosid, Octanoyl n-methylglucamid, n-Octyl alpha-glucopyranosid, N-Octyl beta-glucopyranosid, Oxidierte Stärke Pachyman, Palatinose, Panose, Pentaerythritol, Pentaerythritol diformal, 1,2,3,4,5-Pentahydroxy, Capronsäure, Pentosanpolysulfat, Perseitol, Phenolphthalein glucuronsäure, Phenolphthalein mono-beta-glucosiduron Phenyl 2-acetamido-2-deoxy-alpha-galactopyranosid, Phenyl2-acetamido-2-deoxy-alpha-glucopyranosid, alpha-Phenyl-N-acetyl-glucosaminid, beta-Phenyl N-acetyl-glucosaminid, Phenylethyl beta-galactosid, Phenyl beta-galactopyranosid, Phenyl beta-galactosid, Phenyl alpha-glucopyranosid, Phenyl beta-glucopyranosid, Phenyl alpha-glucosid, Phenyl beta-glucosid, Phenyl beta-glucuronid, beta-phenyllactat Säure, Phenyl alpha-mannopyranosid, beta-phenylpyruvat Säure, Phenyl beta-thiogalactopyranosid, Phenyl beta-thiogalactosid, Phospho(enol)pyruvat, (+)2-Phosphoglycer Säure, (-)3-Phosphoglycer Säure, Phosphohydroxypyruv Säure, 5-phosphorylribose 1-pyrophosphat, Phyt Säure, Poly-N-acetylglucosamin, Polygalacturon Säure, Polygalacturon Säure methyl ester, Polypectate, sodium, Polysaccharid, 5beta-Pregnane-3alpha,2oalpha-diol glucuronid, n-Propyl4-O-beta-galactopyranosyl-beta-glucopyranosid, Prunasin, Psicose, Pullulan, Quinolyl-8beta-glucuron Säure, (+)Raffinose, alpha-Rhamnose, Rhapontin, Ribitol, Ribonolacton, Ribose, d-2-Ribose, alpha-Ribose 1 -phosphat, Ribose 2-phosphat, Ribose 3-phosphat, Ribose 5-phosphat, Ribulose, Ribulose-1,5-diphosphat, Ribulose 6-phosphat, Sacchar Säure, Saccharolactat Säure, Saccharose, Salicin, Sarcolactat Säure, Schardinger-alpha-dextrin, Schardinger-beta-dextrin, Sedoheptulosan, Sedoheptulose 1,7-diphosphat, Sial Säure, Sialyllactose, Sinigrin, Sorbitol, Sorbitol 6-phosphat, (+)-Sorbose, (-)Sorbose, Stachyose, Stärke, Storax, Styrax, Sucrose, Sucrose monocaprat, Tagatose, alpha-Talose, (-)-Talose, Tartar Säure, Testosterone-beta-glucuronid, 2,3,4,6-Tetra-O-methyl-glucopyranose, Thiodiglucosid, 1-Thio-beta-galactopyranose, beta-Thioglucose, 5-Thioglucose, 5-Thioglucose 6-phosphat, Threitol, Threose, (+)Threose, (-)Threose, Thymidin 5'-diphosphoglucose, Thymin 1-beta-arabinofuranosid, Tragacanth, (+)Trehalose, Trifluorothymin, Deoxyribosid, 3,3',5-trihydrory-4'-methoxy-stilbene-3-O-beta-glucosid, Trimethylsilyl(+)arabinose, Trimethylsilyldulcitol, Trimethylsilyl-beta (-) fructose, Trimethylsilyl(+) galactose, Trimethylsilyl-alpha -(+)-glucose, Trimethylsilyl(+) mannitol, Trimethylsilyl(+]rhamnose, Trimethylsilyl(-) sorbitol, Trimethylsilyl(+)xylose, rac-1-O-Tritylglycerol, (+)Turanose, N-Undecyl beta-glucopyranosid, Uracil beta-arabinofuranosid, Uridin 5'-diphospho-N-acetylglucosamin, Uridin 5'-diphosphogalactose, Uridin 5'-diphosphoglucose, Uridin5'-diphospho-glucuron Säure, Uridin 5'-diphosphomannose, Uridin 5'-diphosphoxylose, Vancomycin, Xanthan gum, Xylan, Xylit, Xylitol, Xylobiose, alpha-xylopyranosyl 1-phosphat, Xylose, alpha-xylose 1-phosphat, Xylose 5-phosphat, Xylotriose, Xylulose, Xylulose 5-phosphat, Yacca, Zeatin ribosid, Zinclactat, Zymosan A, usw. Die Bezeichnungen Desoxyribonuclein- (DNA) und Ribonucleinsäure (RNA) haben die übliche Bedeutung; vorzugsweise werden DNA und RNA, oder ihre Antagonisten, zumeist mit einer ausgeprägten biologischen Wirkung eingesetzt.
- mindestens ein Nukleotid, Peptid, Protein und dergleichen;
   Nukleotide, die in und mittels Transfersomen transportiert werden können, sind unter anderen Adenin, Adenosin, Adenosin-3',5'-zyklischer Monophosphat, N6,O2'-dibutyryl, Adenosin-3',5'-zyklischer Monophosphat, N6,O2'-dioctanoyl, Adenosin, N6-cyclohexyl, Salze von Adenosin-5'-diphosphat, Adenosin-5'-monophosphorsäure, Adenosin-5'-O-(3-thiotriphosphat), Salze von Adenosin-5'-triphosphat, 9-beta-D-Arabinoturanosyladenin, 1-beta-D-Arabinoturanosylcytosin, 9-beta-D-Arabinoturanosylguanin, 9-beta-D-Arabinoturanosylguanin 5'-Triphosphat, 1-beta-D-Arabinoturanosylthymine, 5-Azacytidin, 8-Azaguanin, 3'-Azido-3'-deoxythymidin, 6-Beniylaminopurine, Cytidin Phosphoramidit, beta-Cyanoethyl Diisopropyl, 249802Cytidin-5'-triphosphat, 2'-Deoxyadenosin, 2'-Deoxyadenosin 5'-Triphosphat, 2'-Deoxycytidin, 2'-Deoxycytidin 5'-Triphosphat, 2'-Deoxyguanosin, 2'-Deoxyguanosin 5'-Triphosphat, 2',3'-Dideoxyadenosin, 2',3'-Dideoxyadenosin 5'-Triphosphat, 2',3'-Dideoxycytidin, 2',3'-Dideoxycytidin 5'-Triphosphat, 2',3'-Dideoxyguanosin, 2',3'-Dideoxyguanosin 5'-Triphosphat, 2',3'-Dideoxyinosine, 2',3'-Dideoxythymidin, 2',3'-Dideoxythymidin 5'-Triphosphat, 2',3'-Dideoxyuridin, N6-Dimethylallyladenin, 5-Fluoro-2'deoxyuridin, 5-Fluorouracil, 5-Fluorouridin, 5-Fluorouridin 5'-Monophosphat, Formycin A 5'-Triphosphat, Formycin B, Guanosin-3'-5'-zyldischer Monophosphat, Guanosin-5'-diphosphat-3'-diphosphat, Guanosin-5'-O-(2-thiotriphosphat), Guanosin-5'-O-(3'-thiotriphosphat), Guanosin 5'-Triphosphat, 5'-Guanylyl-imidodiphosphat, Inosine, 5-lodo-2'-deoxyuridin, Nicotinamide-Adenin Dinucleotide, Nicotinamide-Adenin Dinucleotide, Nicotinamide-Adenin Dinucleotide Phosphat, Oligodeoxythymidyl Säure, (p(dT)10), Oligodeoiythymidyl Säure (p(dT)12-18), Polyadenylsäure (poly A), Polyadenylsäure-Oligodeoxythymidynsäure, Polycytidylsäure, Poly(deoxyadenyl-deoxiythymidyl-Säure, Polydeoxyadenyl-Säure-Oligodeoxythymidynsäure, Polydeoxythymidylinsäure, Polyinosinsäure-Polycytidylsäure, Polyuridynsäure, Ribonucleinsäure, Tetrahydrouridin, Thymidin, Thymidin-3',5'-diphosphat, Thymidin Phosphoramidit, beta-Cyanoethyl Diisopropyl, 606102 Thymidin 5'-Triphosphat, Thymin, Thymine Ribosid, Uracil, Uridin, Uridin-5'-diphosphoglucose, Uridin 5'-Triphosphat, Xanthin, Zeatin, Transeatin Ribosid, usw. Weitere nützliche Polymere sind: Poly(dA) ss, Poly(A) ss, Poly(C) ss, Poly(G) ss, Poly(U) ss, Poly(dA)-(dT) ds, komplementäre Homopolymere, Poly (d(A-T)) ds, Copolymer, Poly(dG)·(dC) ds, komplementäre Homopolymere, Poly (d(G-C)) ds, Copolymer, Poly (d(l-C)) ds, Copolymer, Poly(I)-Poly(C) ds, usw. Ein Oligopeptid oder ein Polypeptid besteht vorzugsweise aus 3-250, häufig aus 4-100, sehr häufig 4-50, Aminosäuren, die mittels Peptidbrücken miteinander verknüpft sind. Aminosäuren sind zumeist vom Typ alpha- und linksdrehend; Ausnahmen, wie z.b. in Dermorphin, sind jedoch möglich.
   Peptide, die biologisch und/oder therapeutisch bedeutend sind und sich gut für den Einsatz in Kombination mit Transfersomen eignen, sind zum Beispiel: N-Acetyl-Ala-Ala-Ala-, N-Acetyl-Ala-Ala-Ala Methylester, N-Acetyl-Ala-Ala-Ala-Ala, N-Acetyl-Asp-Glu, N-Acetyl-Gly-Leu, Nalpha-Acetyl-Gly-Lys Methylester Acetat, Acetyl-hirudin-Fragment, Acetyl-5-hydroxy-Trp-5-hydroxy-Trp Amid, Des-Acetyl-alpha-melanocyte stimulierender Hormon, N-Acetyl-Met-Asp-Arg-Val-Leu-Ser-Arg-Tyr, N-Acetyl-Met-Leu-Phe, Acetyl-muramyl-Ala-isoGln, N-Acetyl-Phe-Tyr, N-Acetyl-Phe-norLeu-Arg-Phe Amid, N-Acetyl-renin substrate tetradecaPeptid, N-Acetyl-transformierender Wachstumfaktor, Adipokinetischer Hormon II, Adjuvant Peptid, Adrenal peptide E, Adrenocorticotroper Hormon (ACTH 1-39, Corticotropin A) und seine Fragmente wie z.B. 1-4 (Ser-Tyr-Ser-Met), 1-10 (Ser-Tyr-Ser-Met-Glu-His-Phe-Arg-Trp-Gly), 1-17, 1-24 and 1-39, 1-24 and 1-39, 11-24, 18-39, Ala-Ala, beta-Ala-Ala, Ala-Ala-Ala, Ala-Ala-Ala Methylester, Ala-Ala-Ala-Ala, Ala-Ala-Ala-Ala-Ala, Ala-Ala-Ala-Ala-Ala-Ala, Ala-Ala-Phe, 7-Amido-4-methylcoumarin, Ala-Ala-Phe p-nitroanilid, Ala-Ala-Val-Ala p-nitroanilid, Ala-Arg-Pro-Gly-Tyr-Leu-Ala-Phe-Pro-Arg-Met Amid, beta-Ala-Arg-Ser-Ala-Pro-Thr-Pro-Met-Ser-Pro-Tyr, Ala-Asn, Ala-Asp, Ala-Glu, Ala-gamma-Gln-Lys-Ala-Ala, Ala-Gly, beta-Ala-Gly, Ala-Gly-Glu-Gly-Leu-Ser-Ser-Pro-Phe-Tyr-Ser-Leu-Ala-AlaPro-Gln-Arg-Phe Amid, Ala-Gly-Gly, Ala-Gly-Ser-Glu, Ala-His, beta-Ala-His, Ala-isoGln-Lys-Ala-Ala, Ala-Ile, Ala-Leu, beta-Ala-Leu, Ala-Leu-Ala, Ala-Leu-Ala-Leu, Ala-Leu-Gly, Ala-Lys, beta-Ala-Lys, Ala-Met, N-beta-Ala-1-methyl-His, Ala-norVal, Ala-Phe, beta-Ala-Phe, Ala-Phe-Lys 7-amido-4-methylcoumarin, Ala-Pro, Ala-Pro-Gly, Ala-sarcosin, Ala-Ser, Ala-Ser-Thr-Thr-Thr-AsN-Tyr-Thr, Ala-Ser-Thr-Thr-Thr-Asn-Tyr-Thr Amid, Ala-Thr, Ala-Trp, beta-Ala-Trp, Ala-Tyr, Ala-Val, beta-Ala-Val, beta-Ala-Trp-Met-Asp-Phe Amid, Alytesin, Amanitin, Amastatin, Angiotensin I (Asp-Arg-Val-Tyr-Ile-His-Pro-Phe-His-Leu), II II (Asp-Arg-Val-Tyr-Ile-His-Pro-Phe), III und verwandte Peptide, Angiotensin II Antagonist, Angiotensin II Rezeptor bindendes Protein, Angiotensin konvertierendes Enzym und seine Inhibitoren (z.B. Entipain, Bestatin, Chymostatin, E-64, Elastatinal, usw.) Anserin, Antid, Aprotinin, Arginine vasopressin-Ala-Gly, Arg-Ala, Arg-Arg-Leu-Ile-Glu-Asp-Ala-Glu-Tyr-Ala-Ala-Arg-Gly, Arg-Asp, Arg-Glu, Arg-Gly, Arg-Gly-Asp, Arg-Gly-Asp-Ser, Arg-Gly-Asp-Ser-Pro-Ala-Ser-Ser-Lys-Pro, Arg-Gly-Glu-Ser, Arg-Gly-Phe-Phe-Tyr-Thr-Pro-Lys-Ala, Arg-His-Phe, Arg-Ile, Arg-Leu, Arg-Lys, Arg-Lys-Asp-Val-Tyr, Arg-Phe, Arg-Phe-Asp-Ser, Arg-Pro-Pro-Gly-Phe-Ser-Pro-Phe-Arg, Arg-Ser-Arg, Arg-Ser-Arg-His-Phe, Arg-Val, Asn-Pro-Asn-Ala-Asn-Pro-Asn-Ala, Asn-Pro-Asn-Ala-Asn-Pro-Asn-Ala-Asn-Pro-Asn-Ala, alpha-Asp-Ala, Asp-Ala-Glu-Asn-Leu-Ile-Asp-Ser-Phe-Gln-Glu-Ile-Val, Asp-Asp, alpha-Asp-Glu, alpha-AsP-Gly, beta-Asp-Gly, beta-Asp-His, Asp-Leu Amid, beta-Asp-Leu, alpha-Asp-Lys, alpha-Asp-Phe Amid, alpha-Asp-Phe, alpha-Asp-Phe Methylester, beta-Asp-Phe Methylester, alpha-Asp-Ser-Asp-Pro-Arg, Asp-Val, beta-Asp-Val, 'Atrial natriuretic peptid', besonders seine Fragmente 1-32 und 5-28, Atriopeptin I, II, und III, Auriculin A und B, Beauvericin, Beniotript, Bestatin, N-benzylierte Peptide, Big gastrin I, Bombesin, (D-Phe12,Leu14) (Tyr4), Lys3-Bombesin, Tyr4-Bombesin, Docosapeptid und Dodecapeptid aus adrenalen Medulla, Bradykinin (Arg-Pro-Pro-Gly-Phe-Ser-Pro-Phe-Arg) und verwandte Peptide, Bradykinin-Verstärker, gehirnnatriuretisches Peptid, Buccalin, Bursin, S-t-butyl-Cys, Caerulein, Calcitonin, 'Calcitonin gene related peptide' I und II, calmodulinbindende Domäne, N-Carboxymethyl-Phe-Leu, N-((R,S)-2-Carboxy-3-pheenyl-propionyl)-Leu, kardioaktive Peptide A und B, Caronosin, beta-Casomorphin, CD4, Cerebellin, N-Chloroacetyl-Gly-Gly, chemotaktische Peptide, wie z.B. formylierte Substanzen, Cholecystokinin-Fragmente, z.B. Cholecystokinin Oktapeptid, Coherin, usw.
   Ebenfalls erwähnenswert sind Collagen Peptide, Conicostatin, Conicotropin auslösender Faktor, Conotoxin G1, M1 und GVIA, Corticotropin ähnliches Peptid aus dem intermediärem Lobus, Corticotropin auslösender Faktor und verwandte Peptide, C-Peptid, Tyr-C-Peptid, Peptide, die mit cyclischem Calcitonin verwandt sind, Cyclo(His-Phe-), Cyclo(His-Pro-), Cyclo(Leu-Gly-), Cyclo(Pro-Gly-), Cys-Asp-Pro-Gly-Tyr-Ile-Ser-Arg Amid, Cys-Gln-Asp-Ser-Glu-Thr-Arg-Thr-Phe-Tyr, DAGO, Delta-sleep inducing Peptid, Dermorphin, (Ser(Ac)7)-dermorphin, Diabetesassoziierte Peptide und ihre Amide, Nalpha,Nepsilon-diacetyl-Lys-Ala-Ala, N-2,4-Dinitrophenyl-Pro-Gln-Gly-Ile-Ala-Gly-Gln-Arg, Diprotin A, Dynorphine, wie z.B. Dynorphin A(Tyr-Gly-Gly-Phe-Leu-Arg-Arg-Ile-Arg-Pro-Lys-Leu-Lys-Trp-Asp-Asn-Gln), Fragmente 1-6 (Leucine Enkephalin-Arg), 1-8, 1-13 or E-64, Dynorphin B, Ebelactone (e.g. A und B) Ecarin, Elastatinal, Eledoisin und verwandte Peptide, alpha-, beta- und gamma-Endorphin, Endothelins, Endorphine (z.B. alpha (beta-Lipotropin 61-76), (Tyr-Gy-Gly-Pze-Met-Thr-Ser-Glu-Lys-Ser-Gln-Thr-Pro-Leu-Val-Thr) beta (beta-Lipotropin 61-91) und andere beta-Lipotrophin-Fragmente, Enkephalin und Leu-Enkephalin (Tyr-Gly-Gly-Phe-Leu) und verwandte Peptide, Enkephalinase-Inhibitoren (z.B. Epiamastatin, Epibestatin, Foroxymithin, Leupeptin, Pepstatin, Nle-Sta-Ala-Sta), "Eosinophilotactic tetrapeptid", Epiamastatin, Epibestatin, Cys(Acm)20,31-epidermaler Wachstumsfaktor und seine Fragmente oder Rezeptoren, Epidermalmitose inhibierendes Pentapeptid, Trans-epoxysuccinyl-Leu amido-(4-guanidino)butan, Erythropoietin und Fragment, S-Ethylglutathion, Fibrinogenverwandtes Peptid, Fibrinopeptide A und B, Tyr-Fibrinopeptid A, (Glu1)-Fibrinopeptid S, Fibrinopeptid B-Tyr, Fibroblasten Wachstumsfaktor Fragment 1-11, Folliculares Gonadotropin freisetzendes Peptid, N-formylierte Peptide, Foroxymithin, N-(3(2-furyl)acryloyl) Peptid-Derivat, Galanin, GAP 1-13, Gastrisches inhibierendes Polypeptid, Gastrinverwandte Peptide und ihre Abwandlungen, "Gastrin releasing peptide", Gastrointestinalpeptide (z.B. Ala-Trp-Met-Asp-Phe-Amid, Bombesin, Caerulein, Cholecystokinin, Gelanin, Gastrin, Glucagon, Motilin, Neuropeptid K, Pancreatischer Polypeptid, Pancreozymin, Phi-27, Sekretin, Valosin, usw.), Gln-Ala-Thr-Val-Gly-Asp-Val-Asn-Thr-Asp-Arg-Pro-Gly-Leu-Leu-Asp-Leu-Lys, (des-His1, Glu9)-Glucagon Amid, Glucagon (1-37), Glucagon ähnliches Peptid I, alpha-Glu-Ala, Glu-Ala-Glu, Glu-Ala-Glu-Asn, alpha-Glu-Glu, gamma-Glu-Glu, gamma-Glu-Gln, gamma-Glu-Gly, PGlu-Gly-Arg-Phe Amid, alpha-Glu-Gly-Phe, gamma-Glu-His, gamma-Glu-Leu, alphaGlu-alpha-Lys, gamma-Gluepsilon-Lys, N-gamma-Glu-Phe, PGlu-Ser-Leu-Arg-Trp Amid, alpha-Glu-Trp, gamma-Glu-Trp, gamma-Glu-Tyr, alpha-Glu-Val, gamma-Glu-Val, PGlu-Val-Asn-Phe-Ser-Pro-Gly-Trp-Gly-Thr Amid, A-Glu-Val-Phe, Glutathione und verwandte Peptid, Glutathionsulfonsäure, Gly-Ala, Gly-beta-Ala, Gly-Ala-Ala, Gly-Ala-Ala-Ala-Ala, Gly-Ala-Tyr, Glyalpha-aminobutyric acid, Gly-gamma-aminobutyric acid, Gly-Arg-Ala-Asp-Ser-Pro-Lys, Gly-Arg-Ala-Asp-Ser-Pro-OH, Gly-Arg-Gly-Asp-Ser, Gly-Arg-Gly-Asp-Asn-Pro-OH, Gly-Arg-Gly-Asp-Ser-OH, Gly-Arg-Gly-Asp-Ser-Pro-Lys, Gly-Arg-Gly-Asp-Ser-Pro-OH, Gly-Arg-Gly-Asp-Thr-Pro, Gly-Arg-Gly-Asp-Thr-Pro-OH, Gly-Arg p-nitroanilid, Gly-Arg-Gly-Asp, Gly-Arg-Gly-Asp-Ser, Gly-Asn, Gly-Asp, Gly-Asp-Asp-Asp-Asp-Lys, Gly-Glu, Gly-Gly und ihre Derivate, wie z.B. Methyl-, Ethyl- oder Benzyl-Ester, bzw. Amid, Gly-Gly-Ala, Gly-Gly-Arg, Gly-Gly-Gly, Gly-Gly-Gly-Gly, Gly-Gly-Gly-Gly-Gly, Gly-Gly-Gly-Gly-Gly-Gly, Gly-Gly-Ile, Gly-Gly-Leu, Gly-Gly-Phe, Gly-Gly-Phe-Leu, Gly-Gly-Phe-Leu Amid, Gly-Gly-Phe-Met, Gly-Gly-Phe-Met Amid, Gly-Gly-sarcosin, Gly-Gly-Tyr-Arg, Gly-Gly-Val, Gly-His, Gly-His-Arg-Pro, Gly-His-Gly, Gly-His-Lys, Gly-His-Lys-OH, Gly-Ile, Gly-Leu Amid, Gly-Leu, Gly-Leu-Ala, Gly-Leu-Phe, Gly-Leu-Tyr, Gly-Lys, Gly-Met, Gly-norLeu, Gly-norVal, Gly-Phe Amid, Gly-Phe, Gly-Phe-Ala, Gly-Phe-Arg, Gly-Phe-Leu, Gly-Phe-Phe, Gly-Pro, Gly-Pro-Ala, Gly-Pro-Arg, Gly-Pro-Arg-Pro, Gly-Pro-Arg-Pro-OH, Gly-Pro-Gly-Gly, Gly-Pro-hydroxy-Pro, Gly-sarcosin, Gly-Ser, Gly-Ser-Phe, Gly-Thr, Gly-Trp, Gly-Tyr Amid, Gly-Tyr, Gly-Tyr-Ala, Gly-Val, Gly-Phe-Ser, Granuliberin R, Wachstumshormon freisetzender Faktor und seine Fragmente, Hexa-Ala, Hexa-Gly, Hippuryl-Arg (Hip-Arg), Hippuryl-Gly-Gly (Hip-Gly-Gly), Hippuryl-His-Leu (Hip-His-Leu), Hippuryl-Lys, Hippuryl-Phe, Hirudin und seine Fragmente, His-Ala, His-Gly, His-Leu, His-Leu-Gly-Leu-Ala-Arg, His-Lys, His-Phe, His-Ser, His-Tyr, HIV Hüllenprotein (GP120), Hydra-Peptide, P-hydroxyhippuryl-His-Leu, Hypercalcemie-Malignitäts Faktor (1-40), Insulinketten B und C, P-iodo-Phe, Ile-Asn, Ile-Pro-Ile, Insulinähnlicher Wachstumsfaktor I (besonders Fragment 1-70), Insulinähnlicher Wachstumsfaktor II (besonders Fragment 33-40), Interleukin-1B Fragment 163-171, Isotocin, Kassinin (As-Val-Pro-Lys-Ser-Asp-AGlön-he-Val-Gly-Leu-Met-NH2), Katacalcin (Calcitonin Vorläufer-Peptid), Tyr-Katacalcin, Kemptid, Kentsin, Kyotorphin, Laminin Nonapeptid, Laminin Pentapeptid, Laminin Pentapeptidamid, Leucine Enkephalin und verwandte Peptide, Leucopyrokinin, Leu-Ala, Leu-beta-Ala, Leu-Arg, Leu-Asn, Leucokinin I(Asp-Pro-Ala-Phe-Asn-Ser-Trp-Gly-NH2) und II, Leucin-Enkephalinamid (Leu-Enkephalinamid) und verwandte Peptide, Leu-Gly, Leu-Gly-Gly, Leu-Gly-Phe, Leu-Leu Amid, Leu-Leu, Leu-Leu-Leu Amid, Leu-Leu-Leu, Leu-Leu-Phe Amid, Leu-Leu-Tyr, Leu-Lys-Lys-Phe-Asn-Ala-Arg-Arg-Lys-Leu-Lys-Gly-Ala-Ile-Leu-Thr-Thr-Met-Leu-Ala, Leu-Met, Leu-Met-Tyr-Pro-Thr-Tyr-Leu-Lys, Leu-Phe, Leu-Pro, Leu-Pro-Pro-Ser-Arg, Leu-Ser, Leu-Ser-Phe, Leu-Trp, Leu-Tyr, Leu-Val, Leukotrien, Leu-Leu Methylester, Leupeptin, Leu-Ser-p-nitro-Phe-Nle-Ala-Leu Methylester, beta-Lipotropin-Fragment, Litorin, Luteinizing Hormon freisetzendes Hormon und verwandte Peptide, Lymphocyte Activating Pentapeptid, Lys-Ala, Lys-Ala-7-amido-4-methylcoumarin, Lys-Asp, Lys-Cys-Thr-Cys-Cys-Ala, Lys-Glu-Glu-Ala-Glu, Lys-Gly, Lys-Leu, Lys-Lys, Lys-Met, Lys-Phe, Lys-Pro-Pro-Thr-Pro-Pro-Pro-Glu-Pro-Glu-Thr, Lys-Serum thymischer Faktor, Lys-Trp-Lys, Lys-Tyr-Trp-Trp-Phe Amid, Lys-Val, Macrophagen inhibierendes Peptid (Tuftsinfragment 1-3, Thr-Lys-Pro), Magainin I und II, Mastzellen degranulierendes Peptid, Mastoparan, 'alphal-mating factor', Melanin-Concentrating Hormon, MCD Peptid, alpha-, beta-, gamma-, and delta-Melanocytstimulierendes Hormon und verwandte Peptide, Melittin, Mesotocin, Met-beta-Ala, Met-Asn-Tyr-Leu-Ala-Phe-Pro-Arg-Met Amid, Methionin-Enkephalin und verwandte Peptide, Met-Ala, Met-Ala-Ser, Met-Asn, Methionin-Enkephalin (Met-Enkephalin, Tyr-Gly-Gly-Phe-Met) und verwandte Peptide, Methionin-Enkephalinamide (Met-Enkephalinamide, Tyr-Gly-Gly-Phe-Met-NH2) und verwandte Peptide, Met-Gln-Trp-Asn-Ser-Thr-Thr-Phe-His-Gln-Thr-Leu-Gln-Asp-Pro-Arg-Val-Arg-Gly-Leu-Tyr-Phe-Pro-Ala-Gly-Gly, Met-Glu, Met-Gly, Met-Leu, Met-Leu-Phe, Met-Lys, Met-Met, Metorphamid, Met-Phe, Met-Pro, Met-Ser, Met-Tyr-Phe Amid, Met-Val, N-Methoxycarbonyl-Nle-Gly-Arg, P-Nitroanilin, Methoxysuccinyl-Ala-Ala-Pro-Val, Methoxysuccinyl-Ala-Ala-Pro-Val 7-amido-4-methylcoumarin, Metsomatotropin, Mollusken-cardioexzitatorisches Peptid, Morphiceptin, (Val3)-Morphiceptin, Motilin, MSH-Freisetzung inhibierender Faktor, 'Myelin basic protein' und seine Fragment, Naphthylamid-Derivate diverser Peptide, betanaphthyl-Ala-Cys-Tyr-Trp-Lys-Val-Gys-Thr Amid, alpha- und beta-Neoendorphin, alpha-Neurokinin, Neurokinin A (Substance K, Neuromedin L) and B, Neoendorphin (alpha: Tyr-Gly-Gly-Phe-Leu-Arg-Lys-Tyr-Pro, beta, usw.), Neuromedin B, C, K, U8, U-25, usw., Neurokinin A und B, Neuropeptide K und Y, Neurophysin I und II, Neurotensin und verwandte Peptide, Nitroanilidderivate von Peptiden, Nle-Sta-Ala-Sta, NorLeu-Arg-Phe Amid, Opioidpeptide (z.B. Adrenopeptid E, Ala-Gly-Glu-Gly-LEu-Ser-Ser-Pro-Pze-Trp-Ser-Leu-Ala-Ala-Pro-Gln-Arg-Phe-Amide, Casein-Fragmente, Casomorphin, N-CBZ-Pro-D-Leu, Dermorphin, Kyotorphin, Morphiceptin (Tyr-Pro-Phe-Pro-NH2), Meorphamide (Tar-Gly-Gly-Phe-Met-Arg-Arg-Val, Adrenorphin), Osteocalcin (bes. Fragment 7-19), Oxytocin und verwandte Peptide, Pancreastatin und Fragmente davon, wie z. B. 33-49, Pancreatisches Polypeptid, Pancreozymin, Parathyroidea-Hormon (Schilddrüsenhormon) und seine Fragment, besonders 1-34 and 1-84, Penta-Ala, Penta-Gly, Penta-Phe, Pepstatin A, Peptid YY, Peptid T, Phalloidin, Phe-Ala-Ala-p-nitro-Phe-Phe-Val-Leu 4-pyridyl Methylester, Phe-Leu-Phe-Gln-Pro-Gln-Arg-Phe Amid, Phe-Ala, Phe-Gly, Phe-Gly-Gly, Phe-Gly-Gly-Phe, Phe-Gly-Phe-Gly, Phe-Leu Amid, Phe-Leu, Phe-Leu-Arg-Phe Amid, Phe-Leu-Glu-Glu-Ile, Phe-Leu-Glu-Glu-Leu, Phe-Leu-Glu-Glu-Val, Phe-Met, Phe-Met-Arg-Phe Amid, Phe-Phe, Phe-Phe-Phe, Phe-Phe-Phe-Phe, Phe-Phe-Phe-Phe-Phe, Phe-Pro, Phe-Ser-Trp-Gly-Ala-Glu-Gly-Gln-Arg, Phe-Tyr, Phe-Val, PHI-27, PHM-27, Phosphoramidon, Physalaemin (pGlu-Ala-Asp-Pro-Asn-Lys-Phe-Tyr-Gly-Leu-Met-NH2), Preproenkephalin Fragment 128-140, Pressinoinsäure und verwandte Peptide, Pro-Asn, Proctolin (Arg-Tyr-Leu-pro-Thr), Proenkephalin, Pro-His-Pro-Phe-His-Phe-Phe-Val-Tyr-Lys, Pro-Ala, Pro-Arg 4-methoxy-beta-naphthylAmid, Pro-Asp, Proglumid, Pro-Gly, Pro-Gly-Gly, Prohydroxy-Pro, Pro-Ile, Pro-Leu, Pro-Leu-Gly Amid, Pro-Met, Pro-Phe Amid, Pro-Phe, Pro-Phe-Arg 7-amido-4-methylcoumarin, Pro-Phe-Gly-Lys, Pro-Trp, Pro-Tyr, Pro-Val, von cyclischer AMP-abhängige Proteinkinase und ihre Inhibitoren, PyroGlu-Ala-Glu, PyroGlu-Ala, PyroGlu-Ala-Glu, PyroGlu-Asn-Gly, PyroGlu-Gly-Arg p-Nitroanilid, PyroGlu-His-Gly Amid, PyroGlu-His-Gly, PyroGlu-His-Pro Amid, PyroGlu-His-Pro, PyroGlu-Lys-Trp-Ala-Pro, Ranatensin, Reninsubstrat Tetradecapeptid, N-(alpha-rhamnoyranosyloxyhydroxyphosphinyl) Leu-Trp, Sarcosyl-Pro-Arg p-nitroanilid, Sauvagin, schlafauslösendes Peptid (Trp-Ala-Gly-Gly-Asp-Ala-Ser-Gly-Glu), Secretin und verwandte Peptide, Ser-Ile-Gly-Ser-Leu-Ala-Lys, Ser-Ser-Ser, Serum thymic Faktor, Ser-Ala, Ser-beta-Ala, Ser-Asn, Ser-Asp, Ser-Asp-Gly-Arg-Gly, Ser-Glu, Ser-Gln, Ser-Gly, Ser-His, Ser-Leu, Ser-Met, Ser-Phe, Ser-Ser-Ser, Ser-Tyr, schlafauslösendes Peptid, Somatostatin und verwandte Peptide (z.B. Clyclo(p-Trp-Lys-Trh-Phe-Pro-Phe), Steroidogenese aktivierendes Polypeptid, Substanz-P (Arg-Pro-Lys-Pro-Gln-Gln-Phe-Phe-Gly-Leu-Met-NH2) und verwandte Peptide, N-Succinyl-Derivate diverser Peptide, Syndyphalin-20 (Tyr-D-Met(O)-Gly-Phe-ol), Tentoxin, Tetra-Ala, Tetra-Gly, Thiostrepton, DL-Thiorphan (Enkephalinase Inhibitor), Thr-beta-Ala, Thr-Asp, Thr-Leu, Thr-Lys-Pro-Arg, Thr-Ser, Thr-Ser-Lys, Thr-Tyr-Ser, Thr-Val-Leu, Thymopoietin-Fragment, Thymosin alphal und seine Fragmente Thymus zirkulierender Faktor, Thyrocalicitonin, Thyrotropin freisetzender Hormon, Tocinoinsäure, Tosylierte Peptide, Transformierende Wachstumsfaktoren, Tri-Ala, Tri-Ala Methylester, Trp-Ala, Trp-Ala-Trp-Phe Amid, Trp-Glu, Trp-Gly, Trp-Gly-Gly, Trp-His-Trp-Leu-Gln-Leu, Trp-His-Trp-Leu-Gln-Leu-Lys-Pro-Gly-Gln-Pro-Met-Tyr, Trp-His-Trp-Leu-Ser-Phe-Ser-Lys-Gly-Glu-Pro-Met-Tyr, Trp-Leu, Trp-Met-Asp-Phe Amid, Trp-norLeu-Arg-Phe Amid, Trp-Phe, Trp-Trp, Trp-Tyr, Tuftsin (Thr-Lys-Pro-Arg) und seine Fragmente, Tyr-Ala, Tyr-Ala-Gly, Tyr-Ala-Gly-Ala-Val-Val-Asn-Asp-Leu, Tyr-Ala-Gly-N-methyl-Phe 2-hydroxyethylAmid, Tyr-Ala-Phe-Met Amid, Tyr-Arg, Tyr-atriopeptin II, Tyr-Glu, Tyr-Gly, Tyr-Gly-Ala-Val-Val-Asn-Asp-Leu, Tyr-Gly-Gly, Tyr-Gly-Gly-Phe-Leu-Arg-Lys-Arg, Tyr-Gly-Gly-Phe-Met-Arg-Arg-Val Amid, Tyr-Gly-Trp-Phe-Phe Amid, Tyr-Leu, Tyr-Phe, Tyr-Phe-Met-Arg-Phe Amid, Tyr-Phe-Phe Amid, Tyr-Pro-Leu-Gly Amid, Tyr-Pro-Phe-Pro Amid, Tyr-Pro-Val-Pro Amid, Tyr-Thr-Gly-Leu-Phe-Thr, Tyr-Tyr-Phe Amid, Tyr-Trp-Ala-Trp-Phe Amid, Tyr-Trp-Ala-Trp-Phe methylAmid, Tyr-Tyr-Leu, Tyr-Tyr-Phe, Tyr-Tyr-Tyr, Tyr-Tyr-Tyr Methylester, Tyr-Tyr-Tyr-Tyr-Tyr-Tyr, Tyr-Val Amid, Tyr-Val, Tyr-Val-Gly, Urodilatin, Urotensin II, Valosin, Val-Ala, Val-Ala p-Nitroanilid,d, Val-Ala-Ala-Phe, Val-Asp, Val-Glu, Val-Gln, Val-Glu-Glu-Ala-Glu, Val-Glu-Ser-Ser-Lys, Val-Gly, Val-Gly-Asp-Gln, Val-Gly-Gly, Val-Gly-Ser-Glu, Val-Gly-Val-Ala-Pro-Gly, Val-His-Leu-Thr-Pro, Val-His-Leu-Thr-Pro-Val-Glu-Lys, Val-Leu, Val-Lys, Val-Met, Val-Phe, Val-Pro, Val-Pro-Asp-Pro-Arg, Val-Pro-Leu, Val-Ser, Val-Thr, Val-Trp, Val-Tyr, Val-Tyr-Val, Val-Val, vasoaktive intestinale Peptide und verwandte Peptide, vasopressinverwandte Peptide, Vasotocin und verwandte Peptide, Xenopsin, usw.
   Größere Polypeptide werden normalerweise unabhängig von ihrer Konformation als Proteine bezeichnet. Als ein Protein wird in dieser Beschreibung vorzugsweise ein Enzym oder Koenzym, ein Adhäsions- oder Erkennungsmolekül, wie z.B. ein CAMP oder OMP bzw. Lectin, ein Histokompatibilitätskomplex, wie z.B. MHC-I bzw. MHC-II, oder ein Immunglobulin (Antikörper) - oder aber (bio)chemische oder molekulargenetische Abwandlungen davon bezeichnet. Für die Anwendung im Sinne dieser Erfindung kommen von (bio)chemisch modifizierten Proteinen besonders (aber nicht ausschließlich) solche mit einem apolaren Rest, wie z.B. einer Alkyl, Acyl, Alkenoyl, usw. Kette, in Frage.
   Ein Enzym ist ein katalytisch aktives Protein. Enzyme werden in der Regel nach ihren Funktionen gruppiert. Die erfindungsgemäß wichtigsten sind (E.C. Nummern in Klammern):
   Oxidoreductasen, wie z.B.: Alcohol dehydrogenase (1.1.1.1), Alcohol dehydrogenase (NADP abhängige) (1.1.1.2), Glycerol dehydrogenase (1.1.1.6), Glycerophosphat dehydrogenase (1,1.1.8), Xylulose reductase (1.1.1.10), Polyol dehydrogenase (1.1.1.14), Sorbitol dehydrogenase (1.1.1.14), myo-inositol dehydrogenase (1.1.1.18), Uridin 5'-diphosphoglucose dehydrogenase (1.1.1.22), Glyoxalat reductase (1.1.1.26), Lactat dehydrogenase (1.1.1.27), Lactat dehydrogenase (1.1.1.28), Glycerat dehydrogenase (1.1.1.29), beta-Hydroxybutyrat dehydrogenase (1.1.1.30), beta-hydroxyacyl coa dehydrogenase (1.1.1.35), Malat dehydrogenase (1.1.1.37), Malat enzyme (1.1.1.40), Isocitrische dehydrogenase (1.1.1.42), 6-Phosphogluconat dehydrogenase (1.1.1.44), Glucose dehydrogenase (1.1.1.47), beta-Galactose dehydrogenase (1.1.1.48), Glucose-6-phosphat dehydrogenase (1.1.1.49), 3alpha-hydroxysteroid dehydrogenase (1.1.1.50), 3beta-Hydroxysteroid dehydrogenase (1.1.1.51), 3alpha,2betahydroxysteroid dehydrogenase (1.1.1.53), 3-phosphoglycerat dehydrogenase (1.1.1.95), Fucose dehydrogenase (1.1.1.122), Lactat dehydrogenase (cytochrom) (1.1.2.3), Glucose oxidase (1.1.3.4), Cholesterol oxidase (1.1.3.6), Galactose oxidase (1.1.3.9), Alcohol oxidase (1.1.3.13), Glycolat oxidase (1.1.3.15), Choline oxidase (1.1.3.17), Glycerol-3-phosphat oxidase (1.1.3.21), Xanthine oxidase (1.1.3.22), Alcohol dehydrogenase (1.1.99.8), Fructose dehydrogenase (1.1.99.11), Formaldehyde dehydrogenase (1.2.1.1), Format dehydrogenase (1.2.1.2), Aldehyde dehydrogenase (1.2.1.5), Glyceraldehyde-3-phosphat dehydrogenase (1.2.1.12), Gabase (1.2.1.16), Pyruvat oxidase (1.2.3.3), Oxalat oxidase (1.2.3.4), Dihydroorotat dehydrogenase (1.3.3.1), Lipoxidase (1.3.11.12), Alanine dehydrogenase (1.4.1.1), Glutamische dehydrogenase (1.4.1.3), Glutamatdehydrogenase (NADP) (1.4.1.4), L-aminosäuren oxidase (1.4.3.2), D-aminosäuren oxidase (1.4.3.3), Monoaminoxidase (1.4.3.4), Diaminoxidase (1.4.3.6), Dihydrofolat reductase (1.5.1.3), 5,10-Methylenetetrahydrofolat dehydrogenase (1.5.1.5), Saccharopin dehydrogenase NAD+ (1.5.1.7), Octopin dehydrogenase (1.5.1.11), Sarcosin oxidase (1.5.3.1), Sarcosin dehydrogenase (1.5.99.1), Glutathion reductase (1.6.4.2), Ferridoxin-NADP+ reductase (1.6.7.1), NADPH-FMN oxidoreductase (1.6.99.1), Cytochrom c reductase (1.6.99.3), NADH-FMN oxidoreductase (1.6.99.3), Dihydropteridin reductase (1.6.99.7), Uricase (1.7.3.3), Diaphorase (1.8.1.4), Lipoamid dehydrogenase (1.8.1.4), Cytochrom oxidase (1.9.3.1), Nitrat reductase (1.9.6.1), Phenolase (1.10.3.1), Ceruloplasmin (1.10.3.2), Ascorbat oxidase (1.10.3.3), NADH peroxidase (1.11.1.1), Catalase (1.11.1.6), Lactoperoxidase (1.11.1.7), Myeloperoxidase (1.11.1.7), Peroxidase (1.11.1.7), Glutathione peroxidase (1.11.1.9), Chloroperoxidase (1.11.1.10), Lipoxidase (1.13.1.12), Protocatechuat 3,4-dioxygenase (1.13.11.3), Luciferase (Leuchtkäffer) (1.13.12.7), Salicylat hydroxylase (1.14.13.7), p-Hydroxybenzoat hydroxylase (1.14.13.2), Luciferase (bacterielle) (1.14.14.3), Phenylalanine hydroxylase (1.14.16.1), Dopamine-beta-hydroxylase (1.14.17.1), Tyrosinase (1.14.18.1), Superoxid Dismutase (1.15.1.1), Ferredoxin-NADP reductase (1.18.1.2), usw. Transferasen, wie z.B.: Catechol o-methyltransferase (2.1.1.6), Phenylethanolamine n-methyl-transferase (2.1.1.28), Aspartat transcarbamylase (2.1.3.2), Ornithine carbamyltransferase (2.1.3.3), Transketolase (2.2.1.1), Transaldolase (2.2.1.2), Choline acetyltransferase (2.3.1.6), Carnitine acetyltransferase (2.3.1.7), Phosphotransacetylase (2.3.1.8), Chloramphenicol acetyltranferase (2.3.1.28), Kanamycin 6'-acetyltransferase (2.3.1.55), Gentamicin acetyltransferase (2.3.1.60), Transglutaminase (2.3.2.13), gamma-glutamyl transpeptidase (2.3.2.2), Phosphorylase A (2.4.1.1), Phosphorylase B (2.4.1.1), Dextransucrase (2.4.1.5), Sucrose phosphornase (2.4.1.7), Glycogen synthase (2.4.1.11), Uridin 6'-diphosphoglucuronyltransferase (2.4.1.17), Galactosyl transferase (2.4.1.22), Nucleoside phosphorylase (2.4.2.1), Orotidine-5'-monophosphat pyrophosphorylase (2.4.2.10), Glutathion s-transferase (2.5.1.18), Glutamin-oxalat transaminase (2.6.1.1), Glutamic-pyruvat transaminase (2.6.1.2), Gabase (2.6.1.19), Hexokinase (2.7.1.1), Galactokinase (2.7.1.6), Fructose-9-phosphat kinase (2.7.1.11), Gluconat kinase (2.7.1.12), Phosphoribulokinase (2.7.1.19), NAD kinase (Nicotinamid adenine dinucleotide kinase) (2.7.1.23), Glycerokinase (2.7.1.30), Choline kinase (2.7.1.32), Protein kinase (3':5'-cyclischer-AMP abhängige) (2.7.1.37), Phosphorylase kinase (2.7.1.38), Pyruvat kinase (2.7.1.40), Fructose-9-phosphat kinase (Pyrophosphat abhängige) (2.7.1.50), Acetat kinase (2.7.2.1), Carbamat kinase (2.7.2.2), 3-phosphoglycerische phosphokinase (2.7.2.3), Creatine phosphokinase (2.7.3.2), usw.
   Transpeptidase, wie z.B.: Esterase (3.1.1.1), Lipase (3.1.1.3), Phospholipase a (3.1.1.4), Acetylesterase (3.1.1.6), Cholinesterase, acetyl (3.1.1.7), Cholinesterase, butyryl (3.1.1.8), Pectinesterase (3.1.1.11), Cholesterol Esterase (3.1.1.13), Glyoxalase ii (3.1.2.6), Phosphatase, alkaline (3.1.3.1), Phosphatase acid (3.1.3.2), 5'-Nucleotidase (3.1.3.5), 3'-Nucleotidase (3.1.3.6), Glucose-6-phosphatase (3.1.3.9), Fructose-1,6-diphosphatase (3.1.3.11), Phytase (3.1.3.26), Phosphodiesterase i (3.1.4.1), Glycerophosphorylcholin (3.1.4.2), Phospholipase c (3.1.4.3), Phospholipase d (3.1.4.4), Deoxyribonuclease I (3.1.4.5), Deoxyribonuclease II (3.1.4.6), Ribonuclease N1 (3.1.4.8), Sphingomyelinase (3.1.4.12), Phosphodiesterase 3':5'-cyclische (3.1.4.17), Phosphodiesterase II (3.1.4.18), Endonuclease (3.1.4.21), Ribonuclease A (3.1.4.22), Ribonuclease B (3.1.4.22), 3'-Phosphodiesterase 2':3'-cyclic nucleotide (3.1.4.37), Sulfatase (3.1.6.1), Chondro-4-sulfatase (3.1.6.9), Chondro-6-sulfatase (3.1.6.10), Ribonuclease T2 (3.1.27.1), Ribonuclease T1 (3.1.27.3), Ribonuclease U2 (3.1.27.4), Nuclease (3.1.30.1), Nuclease, (aus Micrococcen) (3.1.31.1), alpha-Amylase (3.2.1.1), beta-Amylase (3.2.1.2), Amyloglucosidase (3.2.1.3), Cellulase (3.2.1.4), Laminarinase (3.2.1.6), Dextranase (3.2.1.11), Chitinase (3.2.1.14), Pectinase (3.2.1.15), Lysozyme (3.2.1.17), Neuraminidase (3.2.1.18), alpha-Glucosidase, Maltase (3.2.1.20), beta-Glucosidase (3.2.1.21), alpha-Galactosidase (3.2.1.22), beta-Galactosidase (3.2.1.23), alpha-Mannosidase (3.2.1.24), beta-Mannosidase (3.2.1.25), Invertase (3.2.1.26), Trehalase (3.2.1.28), beta-n-Acetylglucosaminidase (3.2.1.30), beta-Glucuronidase (3.2.1.31), Hyaluronidase (3.2.1.35), beta-Xylosidase (3.2.1.37), Hesperidinase (3.2.1.40), Pullulanase (3.2.1.41), alpha-Fucosidase (3.2.1.51), Mycodextranase (3.2.1.61), Agarase (3.2.1.81), Endoglycosidase F (3.2.1.96), Endo-alpha-n-acetylgalactosaminidase (3.2.1.97), NADase (nicotinamide adenine glycopeptidase) F (3.2.2.5), Dinucleotidase (3.2.2.18), Thiogluc (3.2.3.1), S-adenosylhomocysteinhydrolase (3.3.1.1), Leucin-aminopeptidase, (aus Cytosol) (3.4.11.1), Leucin-aminopeptidase, microsomale (3.4.11.2), Pyroglutamat-aminopeptidase (3.4.11.8), Carboxypeptidase A (3.4.12.2), Carboxypeptidase B (3.4.12.3), Prolidase (3.4.13.9), Cathepsin C (3.4.14.1), Carboxypeptidase W (3.4.16.1), Carboxypeptidase A (3.4.17.1), Carboxypeptidase B (3.4.17.2), alpha-Chymotrypsin (3.4.21.1), beta-Chymotrypsin (3.4.21.1), gamma-Chymotrypsin (3.4.21.1), delta-Chymotrypsin (3.4.21.1), Trypsin (3.4.21.4), Thrombin (3.4.21.5), Plasmin (3.4.21.7), Kallikrein (3.4.21.8), Enterokinase (3.4.21.9), Elastase, pancreatische (3.4.21.11), Protease (Subtilisin) (3.4.21.14), Urokinase (3.4.21.31), Elastase,leukocyte (3.4.21.37), Cathepsin B (3.4.22.1), Papain (3.4.22.2), Ficin (3.4.22.3), Bromelain (3.4.22.4), Chymopapain (3.4.22.6), Clostripain (3.4.22.8), Proteinase A (3.4.22.9), Pepsin (3.4.23.1), Renin (3.4.23.4), Cathepsin D (3.4.23.5), Protease (Aspergillopeptidase) (3.4.23.6), Collagenase (3.4.24.3), Collagenase (3.4.24.8), Pinguinain (3.4.99.18), Renin (3.4.99.19), Urokinase (3.4.99.26), Asparaginase (3.5.1.1), Glutaminase (3.5.1.2), Urease (3.5.1.5), Acylase I (3.5.1.14), Cholylglycine hydrolase (3.5.1.24), Urease(atp-hydrolyzing) (3.5.1.45), Penicillinase (3.5.2.6), Cephalosporinase (3.5.2.8), Creatininase (3.5.2.10), Arginase (3.5.3.1), Creatinase (3.5.3.3), Guanase (3.5.4.3), Adenosin-deaminase (3.5.4.4), 5'-Adenylatsäure-deaminase (3.5.4.6), Creatinine deiminase (3.5.4.21), Anorganische Pyrophosphatase (3.6.1.1), Adenosine 5'-triphosphatase (3.6.1.3), Apyrase (3.6.1.5), Pyrophosphatase, Nucleotid (3.6.1.9), usw.
   Lyasen, wie z.B.: Pyruvat-decarboxylase (4.1.1.1), Oxalat decarboxylase (4.1.1.2), Oxalacetat decarboxylase (4.1.1.3), Glutamische decarboxylase (4.1.1.15), Ornithine decarboxylase (4.1.1.17), Lysine decarboxyla (4.1.1.18), Arginin decarboxylase (4.1.1.19), Histidin decarboxylase (4.1.1.22), Orotidin 5'-monophosphat decarboxylase (4.1.1.23), Tyrosin decarboxylase (4.1.1.25), Phospho(enol) pyruvat carboxylase (4.1.1.31), Ribulose-1,5-diphosphat carboxylase (4.1.1.39), Phenylalanin decarboxylase (4.1.1.53), Hydroxymandelonitrilelyase (4.1.2.11), Aldolase (4.1.2.13), N-Acetylneuraminsäure aldolase (4.1.3.3), usw. Citrat lyase (4.1.3.6), Citrat synthase (4.1.3.7), Tryptophanase (4.1.99.1), Isozyme der carbonischen Anhydrase (4.2.1.1), Fumarase (4.2.1.2), Aconitase (4.2.1.3), Enolase (4.2.1.11), Crotonase (4.2.1.17), delta-Aminolevulinat dehydratase (4.2.1.24), Chondroitinase ABC (4.2.2.4), Chondroitinase AC (4.2.2.5), Pectolyase (4.2.2.10), Aspartase (4.3.1.1), Histidase (4.3.1.3), Phenylalanin Ammoniak-lyase (4.3.1.5), Argininosuccinate lyase (4.3.2.1), Adenylosuccinate lyase (4.3.2.2), Glyoxalase II (4.4.1.5), Isomerasen, wie z.B.: Ribulose-5'-phosphate 3-epimerase (5.1.3.1), Uridine 5'-diphosphogalactose 4-epimerase (5.1.3.2), Mutarotase (5.1.3.3), Triosephosphate isomerase (5.3.1.1), Phosphoriboisomerase (5.3.1.6), Phosphomannose isomerase (5.3.1.8), Phosphoglucose isomerase (5.3.1.9), Tautomerase (5.3.2.1), Phosphoglucomutase (5.4.2.2), Ligasen, wie z.B.: Aminoacyl-tRNA synthetase (6.1.1 ), S-acetyl coenzyme A synthetase (6.2.1.1), Succinic thiokinase (6.2.1.4), Glutamine synthetase (6.3.1.2), Pyruvat carboxylase (6.4.1.1),
   Als Proteasen werden bezeichnet unter anderen Aminopeptidase M, Aminosäure-Arylamidase, Bromelain, Carboxypeptidase A, Carboxypeptidase B, Carboxypeptidase P, Carboxypeptidase Y, Cathepsin C, Chymotrypsin, Collagenasen, Collagenase /Dispase, Dispase, Elastase, Endoproteinase Arg-C, Endoproteinase Asp-N sequencing grade, Encloproteinase Glu-C (Proteinase V8), Endoproteinase Glu-C sequencing grade, Endoproteinase Lys-C, Endoproteinase Lys-C sequencing grade, Endoproteinasen, Faktor Xa, Ficin, Kallikrein, Leucin-Aminopeptidase, Papain, Pepsin, Plasmin, Pronase, Proteinase K, Proteinase V8 (Endoproteinase Glu-C), Pyroglutamat-Aminopeptidase, Pyroglutamat-Aminopeptidase, Restrictionsprotease Faktor Xa, Subtilisin, Thermolysin, Thrombin, Trypsin, usw.
   Ein Koenzym im Sinne dieser Erfindung ist eine jede Enzymaktivität unterstützende Substanz. Zu den biologisch wichtigen Koenzymen gehören z.B. Acetyl-Coenzym A, Acetylpyridin-adenin-dinucleotid, Coenzym A, Flavin-adenin-dinucleotid, Flavin-mononucleotid, NAD, NADH, NADP, NADPH, Nicotinamid-mononucleotid, S-Palmitoyl-Coenzym A, Pyridoxal-5'-phosphorsäure, usw.
   Eine weitere Klasse der Proteine, die für diese Anwendung wichtig ist, sind Lektine. Als Quellen für Lektine kommen sowohl Pflanzen als auch tierisches Gewebe in Frage; besonders häufig werden jedoch verwendet: Abrus pregatorius, Agarigus bisporus, Agrostemma githago, Anguilla anguilla, Arachis hypogaea, Artogarpus integrifolia, Bandeiraea simplicifolia BS-I und BS-II, (Griffonia simplicifolia), Banhlula purpurea, Caragana arborescens, Cicer arietinum, Canavalia ensiformis (Jack Bean), Caragana arborescens (Siberian pea tree), Codium fragile (Grüne Meeressalgen), Concanavalin A (Con A), Cytisus scoparius, Datura stramonium, Dolichos biflorus, Erythrina corallodendron, Euonymus europaeus, Gelonium multiflorum, Glycine max (Soja), Griffonia simplicifolia, Helix aspersa (Gartenschnecke ), Helix pomatia (Weinbergschnecke), Laburnum alpinum, Lathyrus odoratus, Lens culinaris (Linse), Limulus polyphemus (Pfeilschwanzkrebs), Lycopersicon esculentum (Tomate), Lotus tetragonolobus, Luffa aegyptiaca, Maclura pomifera (Osaga Orange), Momordica charantia (Bitter pear melon), Naja mocambique (Mozambiqanische cobra), Naja Naja kaouthia, Mycoplasma gallisepticum, Perseau americana (Avocado), Phaseolus coccineus (Bohnen), Phaseolus limensis, Phaseolus lunatus, Phaseolus vulgaris, Phytolacga americana, Pseudomonas aeruginosa PA-I, Pisum sativum (Pea), Ptilota plumosa (Rote Meeresalgen), Psophocarpus tetragonolobus (Winged bean), Ricinus communis (Castor bean), Robinia pseudoacacia (False acacia, black locust), Sambucus nigra (Efeu), Saponaria officinalis, Solanum tuberosum (Kartoffel), Sophora japonica (Japanischer Pagodenbaum), Tetragonolobus purpureas (Winged or asparagus pea), (Lotus tetragonolobus), Tritigum vulgaris (Weizen(keime)), Ulex europaeus, Vicia faba, Vicia sativa, Vicia villosa, Vigna radiata, Viscum album (Mistel), Wisteria floribunda, usw.
   Weitere interessante Proteine sind z.B. Aktivator des Gewebe-Plasminogens, Insulin, Kallikrein, Keratin, Kininogen, Lactoterrin, Laminarin, Laminin, alpha2-Macroglobulin, alpha1-Microglobulin, F2-Microglobulin, Lipoproteine hoher Dichte basischer Myelin-Protein, Myoglobin, Neurofilament I, II, and III, Neurotensin, Oxytocin, Pancreatischer Oncotetaler Antigen, Parvalbumin, Plasminogen, Plättchen Faktor 4, 'Pokeweed Antiviral Protein', Porphobilinogen, Prealbumin, Prostate Specitic Antigen, Protamine Sulfate, Protein C, Protein C Activator, Protein S, Prothrombin, Retinol bindender Protein, S-100 Protein, Schwangershaftsprotein-1, Serum Amyloid A, Serum Amyloid P Komponente, Tenascin, Testosteron-Estradiol bindendes Globulin, Thioredoxin, Thrombin, Thrombocytin, beta-Thromboglobulin, Thromboplastin, mikrosomales Antigen aus Thyroidea Thyroidea stimulierender Hormon, Thyroxin bindendes Globulin, Transcortin, Transferrin, Ubiquitin, Vimentin, Vinculin, Vitronectin, usw.
   Typische Beispiele von tierischen und menschlichen Hormonen als erfindungsgemäße Wirkstoffe sind z.B. Adrenalin, Adrenocortischerotroper Hormon, Angiotensin, Antidiuretischer Hormon, Cholecystokinin, Chorionic gonadotropin, Corticotropin A, Danazol, Diethylstilbestrol, Diethylstilbestrol glucuronid, 13,14-dihydro-15-keto-prostaglandine, 1-(3',4'-dihydroxyphenyl)-2-aminoethanol, 5,6-dihydroxytryptamin, Epinephrin, Follikelstimulierender Hormon, Gastrin, Gonadotropin, β-Hypophamin, Insulin, Juveniler Hormon, 6-Ketoprostaglandine,15-Ketoprostaglandine, LTH, Luteinizing Hormon auslösender Hormon, Luteotroper Hormon, alpha-Melanocyten stimulierender Hormon, gamma-Melanocyten stimulierender Hormon, 5-Melanocyten stimulierender Hormon, Noradrenalin, Norepinephrin, Oxytocin, Parathyroid Hormon, Parathyroide Stoffe, Prolactin, Prostaglandine, Secretin, Somatostatin, Somatotropin (STH), Thymosin alpha 1, Thyrocalcitonin, Thyroglobulin, Thyroidea stimulierender Hormon, Thyrotroper Hormon, Thyrotropin auslösender Hormon, 3,3',5-Triiodothyroacetosäure, 3,3',5'-Triiodothyronin, TSH, Vasopressin, etc.
   Oestrogene sind zumeist Steroidhormone mit 18 Kohlenstoffatomen und einem ungesättigten (aromatischen) Ring. Zu den wichtigsten Oestrogenen gehören Chlorotrianisen, Diencestrol, Diethylstilboestrol, Diethylstilboestroldipropionat, Diethylstilboestroldisulfat, Dimestrol, Estradiol, Estradiolbenzoat, Estradiolundecylat, Estriolsuccinat, Estron, Ethinglestradiol, Nexoestrol, Nestranol, Oestradiolvalerat, Oestriol und Chinestrol.
   Gestagene sind zumeist synthetische Hormone mit zumeist progesteron-ähnlichen Eigenschaften; die wichtigsten Stoffe aus dieser Substanzklasse sind Allylestrenol, Chlormadinonacetat, Dimethisteron, Ethisteron, Hydroxyprogesteron-caproat, Lynestrenol, Medrogeston, Medroxyprogesteron-acetat, Megestrolacetat, Methyloestrenolon, Norethisteron, Norethisteron-acetat und Norgestrel.

Als Wirkstoffe können auch biologische Extrakte dienen. Als Quellen biologischer, pharmakologisch wirksamer Extrakte, die mittels Transfersomen als 'Wirkstoffe' durch die Haut transportiert werden können, verdienen besondere Erwähnung Acetobacter pasteurianum, Acokanthera ouabaio cathel, Aesculus hippocastanum, Ammi visnaga Lam., Ampi Huasca, Apocynum Cannabium, Arthrobotrys superba var. oligospora (ATCC 11572), Atropa belladonna, Bacillus Lentus, Bacillus polymyxa, Bacilius sphaericus, Castilloa elastica cerv., Chondrodendron tomentosum (Ampi Huasca), Convallaria majalis, Coronilla-Enzyme, Corynebacterium hoagii (ATCC 7005), Corynebacterium simplex, Curvularia lunata (Wakker) Boadijn, Cylindrocarpon radicola (ATCC 11011), Cynara scolymus, Datura Metel, Didymella, Digilanidase, Digitalis Lanata, Digitalis purpurea, Duboisia, Flavobacterium dehydrogenans, Fusarium exquiseti saccardo, Hyoscyamus niger, Jaborandi-Blätter (P. microphyilus Stapf), Mariendistel, Micromonosporapurpurea u. echinospora, Paecilomyces varioti Bainier var. antibioticus, Penicillium chrysogenum Thom, Penicillium notatum Westling, Penicillium patulum, Rauwolfia serpentina Benth., Rhizopus arrhizus Fischer (ATCC-11145), Saccharomyces cerevisiae, Schizomycetes ATCC-7063, Scilla maritima L., Scillarenase, Septomyxa affinis (ATCC 6737), Silybum marianum Gaertn. (Mariendistel), Streptomyces ambofaciens, Strophantusgratus, Strophantus Kombe, Thevetia peruviana, Vinca minor L. und Vinca rosea.

Falls nicht anders spezifiziert, können alle angegebenen Substanzen, Tenside, Lipide, Wirkstoffe oder Zusatzstoffe mit einem oder mehreren chiralen Kohlenstoffatom entweder als racemische Mischungen oder als optisch reine Enantiomere verwendet werden

### WIRKPRINZIP

Im Falle von Permeations-Barrieren kann der Wirkstofftransport durch solche Träger bewältigt werden, die die folgenden Grundkriterien erfüllen:
- Die Träger sollen einen Gradienten spüren oder aufbauen, der sie in oder über die Barriere treibt, z.B. von der Körperoberfläche in und unter die Haut, von der Blattoberfläche in das Blattinnere, von einer Seite der Barriere zur anderen;
- Der Permeationswiderstand, den die Träger in der Barriere spüren, soll möglichst klein sein im Vergleich zu der treibenden Kraft;
- Die Träger sollen fähig sein, in und/oder durch die Barriere zu permeiren, ohne dabei die eingeschlossenen Wirkstoffe unkontrolliert zu verlieren.

Ferner sollen die Träger vorzugweise eine Kontrolle über die Wirkstoffverteilung, die Wirkstoffeffekte sowie den zeitlichen Wirkungsablauf erlauben. Sie sollen fähig sein, im Bedarfsfall das Material auch in die Tiefe der Barriere und über diese hinweg zu bringen und/oder einen solchen Transport zu katalysieren. Und nicht zuletzt sollen die Träger den Wirkungsbereich und die Wirkungstiefe sowie - in günstigen Fällen - die Art der Zellen, Gewebsteile, Organe, oder Systemabschnitte, die erreicht oder behandelt werden, beeinflussen.

In erster Hinsicht kommen für die biologischen Anwendungen die chemischen Gradienten in Frage. Besonders geeignet sind die physiko-chemischen Gradienten, wie z.B. der (De)Hydratationsdruck (Feuchtigkeitsgradient) oder ein Konzentrationsunterschied zwischen dem Applikations- und Wirkungsort; aber auch elektrische oder magnetische Felder sowie thermische Gradienten sind in dieser Hinsicht interessant. Für technologische Anwendungen sind ferner der applizierte hydrostatische Druck oder ein bestehender Druckunterschied wichtig.

Um die zweite Bedingung zu erfüllen, müssen die Träger auf der mikroskopischen Skala ausreichend 'dünnflüssig' sein; nur dann können sie durch die Konstriktionen innerhalb der Permeabilitätsbarriere gelingen.

Der Permeationswiderstand nimmt verständlicherweise mit der Trägergröße ab. Aber auch die treibende Kraft ist häufig von der Trägergröße abhängig; bei größenunabhängigem Druck nimmt diese Kraft mit der Größe typischerweise ab. Darum ist die Übertragungeffizienz keine einfache Funktion der Größe, sondern weist häufig ein von der Wahl der Träger- und Wirkstoffe abhängiges Maximum auf.

Im Falle von molekularen Aggregaten wird der Permeationswiderstand zumeist durch die mechanische Elastizität und die Verformbarkeit des Trägers bestimmt; aber auch die Viskosität der Gesamtpräparation ist wichtig: die erste muß hoch genug, die andere ausreichend niedrig sein.

Als ein Kriterium für die Optimierung von supramolekularen Trägern im Sinne dieser Erfindung kann daher die Größe, aber noch mehr die Verformbarkeit dienen; beispielhaft für die letzte kann die Trägerfähigkeit betrachtet werden, sich zu krümmen oder Ausläufer zu bilden - als Funktion von allen relevanten Systemvariablen. (In der Praxis reicht es bereits aus, nur diejenigen Variablen zu untersuchen, die für eine kontrollierte Anwendung in Frage kommen. Die in dieser Anmeldung angeführten Beispiele umfassen daher lediglich die Variation der Konzentration von randaktiven Komponenten und die absolute Trägerkonzentration, die eine erzwungene Verkleinerung von Lipidvesikeln oder Vesikelpermeation beeinflussen.) Das gilt z.B. für eine transkutane oder transkutikale Stoffübertragung, aber auch für den Stofftransport durch die Lungenalveoli, in das Haar, in Gele und dergleichen.

Bezüglich des dritten Kriteriums spielt die Wahl der Träger, Wirkstoffe und Zusatzstoffe, sowie die applizierte Trägermenge oder Konzentration eine Rolle. Niedrige Dosierung führt meistens zu einer oberflächlichen Behandlung: Stoffe, die schlecht wasserlöslich sind, bleiben dabei zumeist in der apolaren Region der Permeabilitätsbarriere (z.B. in den Membranen der Epidermis) hängen; gut lösliche Wirkstoffe, die leicht aus den Trägern diffundieren, können eine andere Verteilung haben als die Träger; für solche Stoffe ist also auch die Durchläßigkeit der Transfersomen-Membrane wichtig. Randaktive Substanzen, die dazu neigen, aus den Trägern in die Barriere überzutreten, führen zu einer örtlich variablen Trägerzusammensetzung, usw. Diese Zusammenhänge sollen vor jeder Applikation überdacht und berücksichtigt werden. Bei der Suche nach Bedingungen, unter denen die einfachen Trägervesikeln zu Transfersomen werden, kann die folgende Faustregel verwendet werden.
- Als erstes werden die Bedingungen gesucht, unter denen die Trägervesikeln durch die Wirkung von randaktiven Substanzen solubilisiert werden. An diesem kritischen Punkt sind die 'Vesikel' maximal deformierbar, da sie im stetten Zusammenbau und Abbau begriffen sind. Gleichzeitig sind sie aber auch unstabil und unfähig, wasserlösliche Substanzen zu enthalten und zu übertragen.
- Als nächstes wird die Trägerzusammensetzung bzw. Konzentration durch die Verringerung der Randaktivität im System so angepasst, daß die Vesikel sowohl eine ausreichende Stabilität als auch eine ausreichende Deformierbarkeit, und daher zweckmäßige Permeationsfähigkeit, ausweisen. Unter Stabilität wird in dieesr Anmeldung neben dem mechanischem "Zusammenhalt" auch verstanden, daß sich der Substanz-, insbesondere der Wirkstoffgehalt der Trägerzusammensetzung beim Transport, insbesondere beim Permeationsvorgang, nicht oder nicht wesentlich ändert. Die Position des gesuchten Optimums ist dabei von einer Vielzahl der Randbedingungen abhängig. Die Art der Wirkstoffmoleküle spielt auch eine wichtige Rolle: je kleiner und je hydrophiler sind die zu übertragenden Agenzien, desto weiter muß das Trägersystem von dem Solubilisierungspunkt entfernt werden; auch die angepeilte Lagerungsfähigkeit der Träger ist wichtig: mit der Nähe zum Solubilisierungspunkt kann die Tendenz der Transfersomen, größere Partikel zu bilden, zunehmen, und die Lagerungsstabilität der Träger abnehmen.
- Abschließend werden die Systemparameter unter Berücksichtigung der angestrebten Applikationsmodi und Ziele nachoptimiert. Für eine rasche Wirkung ist hohe Permeationsfähigkeit erforderlich; für langsame Wirkstoffreisetzung eine allmähliche Barrieren-Penetration und entsprechend eingestellte Membranpermeabilität vorteilhaft; für die Tiefenwirkung ist eine hohe Dosis, für möglichst breite Verteilung eine nicht zu hohe Trägerkonzentration angeraten.

In dieser Anmeldung werden relevante Eigenschaften von Transfersomen als Träger für die Lipidvesikel besprochen. Die meisten Beispiele beziehen sich beispielhaft auf die Träger aus Phospholipiden, wobei jedoch die allgemeine Gültigkeit der Schlußfolgerungen nicht auf diese Trägerklasse oder Moleküle beschränkt ist. Die Lipidvesikel-Beispiele illustrieren lediglich die Eigenschaften, die zur Penetration durch die Permeabilitätsbarrieren, wie z.B. Haut, benötigt werden. Dieselben Eigenschaften ermöglichen Trägertransport auch durch die tierische oder menschliche Epidermis, Schleimhäute, pflanzliche Kuticula, über anorganische Membranen, usw.

Der wahrscheinliche Grund für die spontane Permeation von Transfersomen durch die 'Poren' in der Hornhautzellenschicht ist vermutlich, daß diese auf einer Seite in einem wässrigen Kompartment, der Subcutis, münden; die Transfersomen werden dabei durch den osmotischen Druck getrieben. Alternativ kann aber zusätzlich ein externer, z.B. hydrostatischer oder elektroosmotischer Druck appliziert werden.

Je nach Vesikelmenge können nach einer perkutanen Applikation die Lipidvesikel bis in die Subkutis gelangen. Die Wirkstoffe werden dabei, je nach der Größe, Zusammensetung und Formulierung der Träger oder Agentien, entweder lokal freigesetzt, proximal angereichert, oder aber über die Blutgefäße bzw. Lymphgefäße weitergeleitet und über den Körper verteilt.

Manchmals ist es angebracht, den pH-Wert der Formulierung gleich nach der Herstellung oder unmittelbar vor der Anwendung anzupassen. Eine solche Anpassung soll die Zerstörung der Systemkomponenten und/oder der Wirkstoffträger unter den anfänglichen pH-Bedingungen verhindern und die physiologische Verträglichkeit der Formulierung gewährleisten. Zur Neutralisierung werden zumeist physiologisch verträgliche Säuren oder Basen bzw. Pufferlösungen mit einem pH-Wert von 3-12, vorzugsweise 5 bis 9, besonders häufig 6-8, je nach dem Zweck und Ort der Applikation, verwendet. Physiologisch verträgliche Säuren sind beispielsweise verdünnte wässrige Mineralsäuren, wie z.B. verdünnte Salzsäure, Schwefelsäure oder Phosphorsäure, oder organische Säuren, z.B. Alkancarbonsäuren, wie Essigsäure. Physiologisch verträgliche Laugen sind z.B. verdünnte Natronlauge, entsprechend ionisierte Phosphorsäure, usw.

Die Herstellungstemperatur wird normalerweise den eingesetzten Substanzen angepaßt und liegt für die wässrige Präparationen üblicherweise zwischen 0 und 95 °C. Vorzugsweise arbeitet man in einem Temperaturbereich von 18-70 °C; besonders bevorzugt für die Lipide mit fluiden Ketten ist der Temperaturbereich zwischen 15 und 55 °C, für die Lipide mit geordneten Ketten zwischen 45 und 60 °C. Andere Temperaturbereiche sind für die nichtwässrigen Systeme oder für Präparationen, die Kryo- oder Hitzekonservantien enthalten, möglich.

Falls die Empfindlichkeit der Systemkomponenten das verlangt, können die Formulierungen kühl (z.B. bei 4°C) gelagert werden. Sie können auch unter Inertgas-, z.B. Stickstoffatmosphäre, hergestellt und aufbewahrt werden. Die Lagerungsdauer kann durch die Verwendung von Substanzen ohne Mehrfachbindungen sowie durch das Eintrocknen und Verwendung von Trockensubstanz, die erst an Ort und Stelle aufgelöst und aufgearbeitet wird, weiter erhöht werden.

In den meisten Fällen findet die Applikation der Träger bei Raumtemperatur statt. Einsätze bei tieferen Temperaturen oder bei höheren Temperaturen mit synthetischen Substanzen noch höhere Temperaturen sind indes durchaus möglich.

Die Präparate können im voraus oder an Ort und Stelle der Anwendung vorbereitet werden, wie das z.B. in P 40 26 833.0-43 oder anhand mehrerer Beispiele im Handbuch 'Liposomes' (Gregoriadis, G., Hrsg., CRC Press, Boca Raton, Fl., Vols 1-3, 1987) im Buch 'Liposomes as drug carriers' (Gregoriadis, G., Hrsg., John Wiley & Sons, New York, 1988), oder im Laboratoriumshanduch 'Liposomes. A Practical Approach' (New, R., Oxford-Press, 1989) beschrieben ist. Falls erforderlich, kann eine Wirkstoffsuspension unmittelbar vor dem Gebrauch verdünnt oder aufkonzentriert (z. B. per Ultrazentrifugation oder Ultrafiltration) bzw. mit weiteren Zusatzstoffen vermengt werden. Dabei muß jedoch die Möglichkeit einer Verschiebung des Optimums für die Trägerpermeation ausgeschlossen oder einkalkuliert werden.

Die Transfersomen gemäß dieser Anmeldung sind als Träger von lipophilen Stoffen, z.B. fettlöslichen biologischen Wirkstoffen, Therapeutika und Giften, usw. geeignet; von einem noch größeren praktischen Wert ist jedoch ihre Anwendung im Zusammenhang mit wasserlöslichen Substanzen, besonders wenn deren Molmasse größer als 1000 ist.

Die Transfersomen können ferner zur Stabilisierung von hydrolyseempfindlichen Stoffen beitragen und eine verbesserte Verteilung von Agentien in der Probe und am Ort der Applikation ermöglichen, sowie einen günstigeren zeitlichen Verlauf der Wirkstoffwirkung gewährleisten. Die Grundsubstanz, aus der die Träger bestehen, kann selbst eine vorteilhafte Wirkung haben. Die wichtigste Trägereigenschaft ist jedoch, den Materialtransport in und durch die Permeabilitätsbarriere zu ermöglichen, und somit Applikationen zu erlauben, die vor dieser Erfindung nicht durchführbar waren.

Die beschriebenen Formulierungen sind erfindungsgemäß optimiert für die topische Applikation an - oder in der Nähe von - Permeabilitätsbarrieren. Besonders interessant dürfte das Auftragen auf die Haut oder auf die pflanzliche Kuticula sein. (Sie sind aber auch für eine orale (p.o.) oder parenterale (i.v. i.m. oder i.p.) Applikation gut geeignet, besonders wenn die randaktiven Substanzen so gewählt sind, daß die Verluste am Applikationsort klein sind.) Randaktive Substanzen, die am Applikationsort weniger randaktiv, bevorzugt abgebaut, besonders stark aufgenommen oder verdünnt werden, sind in letzter Hinsicht besonders wertvoll.

Im dermatologischen Bereich werden bevorzugt bis zu 50, häufig bis zu 10, besonders häufig weniger als 2.5 oder sogar weniger als 1 mg Trägersubstanz pro cm² Hautfläche aufgetragen; die optimale Menge hängt von der Trägerzusammensetzung, angepeilten Wirktiefe und.Wirkdauer, sowie von dem Applikationsort. Im agrotechnischen Bereich liegen Applikationsmengen typischerweise niedriger, häufig unter 0.1g pro m².

Je nach der angestrebten Anwendung können die Formulierungen erfindungsgemäß auch geeignete Lösungsmittel bis zu einer Konzentration, die durch die jeweilige physikalische (keine Solubilisierung oder nennenswerte Optimumverschiebung), chemische (keine Beeinträchtigung der Stabilität), oder biologische bzw. physiologische (wenig unerwünschte Nebeneffekte) Verträglichkeit bestimmt wird.

Vorzugsweise kommen dabei unsubstituierte oder substituierte, z.B. halogenierte, aliphatische, cycloaliphatische, aromatische oder aromatisch-aliphatische Kohlenwasserstoffe, z.B. Benzol, Toluol, Methylenchlorid oder Chloroform, Alkohole, z.B. Methanol oder Ethanol, Propandiol, Erithritol, Niederalkancarbonsäureester, z.B. Essigsäurealkylester, z.B. Diethylether, Dioxan oder Tetrahydrofuran, oder Mischungen dieser Lösungsmittel, in Frage.

Übersichten der Lipide und Phospholipide, die zusätzlich zu den vorstehend genanntnen für eine Verwendung im Sinne dieser Anmeldung geeignet sind, sind in 'Form and Function of Phospholipids' (Ansell & Hawthorne & Dawson, Verfasser), 'An Introduction to the Chemistry and Biochemistry of Fatty acids and Their Glycerides' von Gunstone und in anderen Übersichtswerken enthalten. Die erwähnten Lipide und Tenside sowie andere, in Frage kommende randaktive Stoffe, und ihre Herstellung, sind bekannt. Ein Überblick der käuflich erhältlichen Tenside, sowie die Warenzeichen, unter denen diese Tenside von den Herstellerfirmen vertrieben werden, ist im Jahrbuch 'Mc Cutcheon's, Emulsifiers & Detergents', Manufacturing Confectioner Publishing Co, angegeben. Ein aktuelles Verzeichnis der pharmazeutisch akzeptablen Wirkstoffe ist z. B. dem 'Deutschen Arzneibuch' (und der jeweiligen Jahresausgabe der 'Rote Liste'), ferner aus British Pharmaceutical Codex, European Pharmacopoeia, Farmacopoeia Ufficiale della Republica Italiana, Japanese Pharmacopoeia, Nederlandse Pharmacopoeia, Pharmacopoeia Helvetica, Pharmacopee Francaise, The United States Pharmacopoeia, The United States NF, usw., entnehmbar. Ein ausführliches Verzeichnis der erfindungsgemäß geeigneten Enzyme ist in dem Band 'Enzymes', 3rd Edition (M. Dixon un E.C. Webb, Academic, San Diego, 1979) enthalten, aktuelle Neuentwicklungen sind der Reihe 'Methods in Enzymology' zu entnehmen. Zuckererkennende Proteine, die im Zusammenhang mit dieser Erfindung interessant sind, sind in dem Buch 'The Lectins: Properties, Functions, and Applications in Biology and Medicine' (I.E. Liener, N. Sharon, I.T. Goldstein, Eds. Academic, Orlando, 1986) sowie in aktuellen Fachpublikationen beschrieben; Agrotechnisch interessante Substanzen sind in 'The Pesticide Manual' (C. R. Worthing, S.B. Walker, Eds. British Crop Protection Council, Worcestershire, England, 1986, z.B. 8th edition) und in 'Wirkstoffe in Pflanzenschutz und Schädlingsbekämpfung', herausgegeben durch den Industrie-Verband Agrar (Frankfurt) angeführt; käuflich erhältliche Antikörper sind in dem Katalog 'Linscott's Directory', die wichtigsten Neuropeptide in 'Brain Peptides' (D.T. Krieger, M.J. Brownstein, J.B. Martin, Eds. John Wiley, New York, 1983), entsprechenden Ergänzungsbänden (z.B. 1987) und anderen Fachpublikationen aufgelistet.

Herstellungstechniken für Liposome,die sich überwiegend auch für die Herstellung von Tranfersomen eignen, sind in 'Liposome Technology' (Gregoriadis, Ed., CRC Press) oder in älteren Nachschlagewerken, z.B. in 'Liposomes in Immunobiology' (Tom & Six, Eds., Elsevier), in 'Liposomes in Biological Systems' (Gregoriadis & Allison, Eds., Willey), in'Targeting of Drugs' (Gregoriadis & Senior & Trouet, Plenum), usw., sowie in der einschlägigen Patentliteratur beschrieben.

Die folgenden Beispiele veranschaulichen die Erfindung, ohne sie zu beschränken. Temperaturen sind in Grad Celsius, Trägergrößen in Nanometer, Drucke in Pascal und sonstige Größen in üblichen SI Einheiten angegeben.

Verhältnis- und Prozentangaben sind molar, sofern nicht anders angegeben.

### Beispiele 1-13:

### Zusammensetzung:

| | |
|---|---|
| 250-372 mg | Phosphatidylcholin aus Sojabohnen (+95 % = PC) |
| 187-34.9 mg | Ölsäure (+99 %) |
| 0.312-0.465 ml | Ethanol, absolut |
| 10 mM | Hepes |

### Herstellung:

In unterschiedliche Volumina von alkoholischen PC-Lösungen, die 75 Mikromol Lipid enthalten, werden zunehmende Mengen von Ölsäure pipettiert, so daß eine Konzentrationsreihe von Lipid/Tensid-Verhältnissen entsteht, die beginnend mit einem Verhältnis 0.5 jeweils um einen Wert von 0.2 ansteigt. Anschließend werden zu jeder Lipidprobe 4.5 ml einer sterilen Pufferlösung zugespritzt und die Gemische bei 4 °C einen Tag lang inkubiert. Wenn der pH Wert durch die Zugabe von 1 M NaOH eingestellt werden muß, wird mit weiterer Behandlung noch 24 Stunden gewartet. Zur endgültigen Liposomenbildung werden die Proben durchmischt, durch einen Polycarbonatfilter (0.45 Mikrometer) gedrückt und in verschlossenen Glasröhrchen bei 4 °C aufbewahrt.

### Charakterisierung:

Der Permeationswiderstand wird dem relativen Druck, mit dem sich die Proben einer weiteren Filtration durch ein 0.2 Mikrometer-Filter widersetzen, gleichgesetzt. In dieser Anmeldung ist dieser Widerstand in relativen Einheiten von 1 bis 10 angegeben.

Die Vesikelgröße wird mittels dynamischer Lichtstreuung bei 33 °C mit einem Zeta-Sizer Gerät der Fa. Malvern bestimmt. Zur Analyse der Korrelationskurven wird eine Abwandlung des Programmes "Contin" verwendet.

In dieser Versuchsreihe liegt die Vesikelgröße ziemlich unabhängig von der Menge der randaktiven Substanz zwischen 300 und 350 nm.

### Permeation:

Der Permeationswiderstand nimmt mit fallender relativen Konzentration von Fettsäure in den Transfersomen zunächst zu. Dieser Trend ist jedoch nicht monoton. Bei einem Lipid/Tensid-Verhältnis von ca. 2 werden die Liposomen wieder permeationsfähiger, bis sie oberhalb von L/T=3 die Konstriktionen fast nicht mehr passieren können. Die Vesikel mit einem Lipid/Tensid Molverhältnis von 1/2 sind jedoch perfekt permeationsfähig. (Eine 8 % Lipidsuspension ist in diesem Fall fast so leicht filtrierbar wie Wasser.) Bei dieser Konzentration, die ungefähr 30 % der Solubilisierungsdosis der Fettsäure im alkalischen entspricht, werden die Liposomen also zu optimalen Transfersomen.

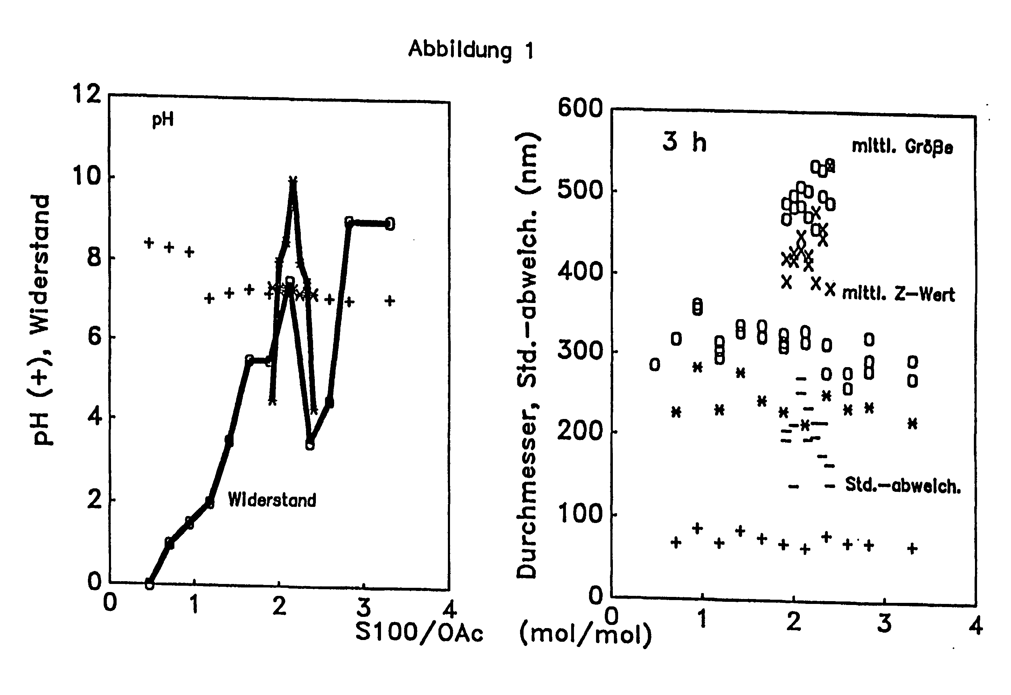

Die genauen Daten (0) sind in Abbildung 1 gezeigt. Die angegebenen Durchmesser wurden nach dem Permeationsversuch gemessen.

### Beispiele 14-20:

### Zusammensetzung:

| | |
|---|---|
| 349-358 mg | Phosphatidylcholin aus Sojabohnen (+95 % = PC) |
| 63.6-52.2 mg | Ölsäure (+99 %) |
| 10 mM | Hepes |

### Herstellung:

Zu entsprechenden Mengen vom Lipid und Fettsäure, die eine relative Konzentrationsreihe von L/T = 1.92 bis 2.4 in Schritten von 0.08 ergeben, werden 4.5 ml Puffer pipettiert; der pH-Wert wird auf 7.2-7.3 eingestellt. Nach 6 tägiger Inkubation bei 4 Grad werden die Liposomen beschallt, bis ihr mittlerer Durchmesser cca. 0.8 Mikrometer beträgt.

### Permeation und Charakterisierung:

Der Permeationswiderstand wird wie in den Beispielen 1-13 ermittelt. Seine Werte in Abhängigkeit von der Menge der randaktiven Substanzen ähneln Resultaten aus den Versuchen 1-13. Die Vesikel sind jedoch etwas größer (um 500 nm), was die vergleichsweise niedrige Flußgeschwindigkeit beim Passieren des Filters in diesem Versuch erklären läßt.

Die entsprechenden Meßdaten (+) sind in der Abbildung 1 dargestellt.

### Beispiele 21-31:

### Zusammensetzung:

| | |
|---|---|
| 322.6-372 mg | Phosphatidycholin aus Sojabohnen (+95 % = PC) |
| 96.8-34.9 mg | Ölsäure (+99 %) |
| 0.403-0.465 ml | Ethanol, absolut |
| 10 mM | Hepes |
| 130 mM | NaCl, p.a. |

### Herstellung:

Es wird im wesentlichen wie bei den Beispielen 14-20 verfahren. Der Unterschied besteht darin, daß die Elektrolytlösung isotonisch mit Blut ist.

### Permeation und Charakterisierung:

Der Permeationswiderstand entspricht im Rahmen der Meßfehler den Ergebnissen aus den Beispielen 1-13. Auch die Vesikelgrößen sind ähnlich. Gleich nach der Herstellung liegen sie im Bereich von 320-340 nm. 8 Tage später sind die Vesikel jedoch auf ca. 440 nm gewachsen.

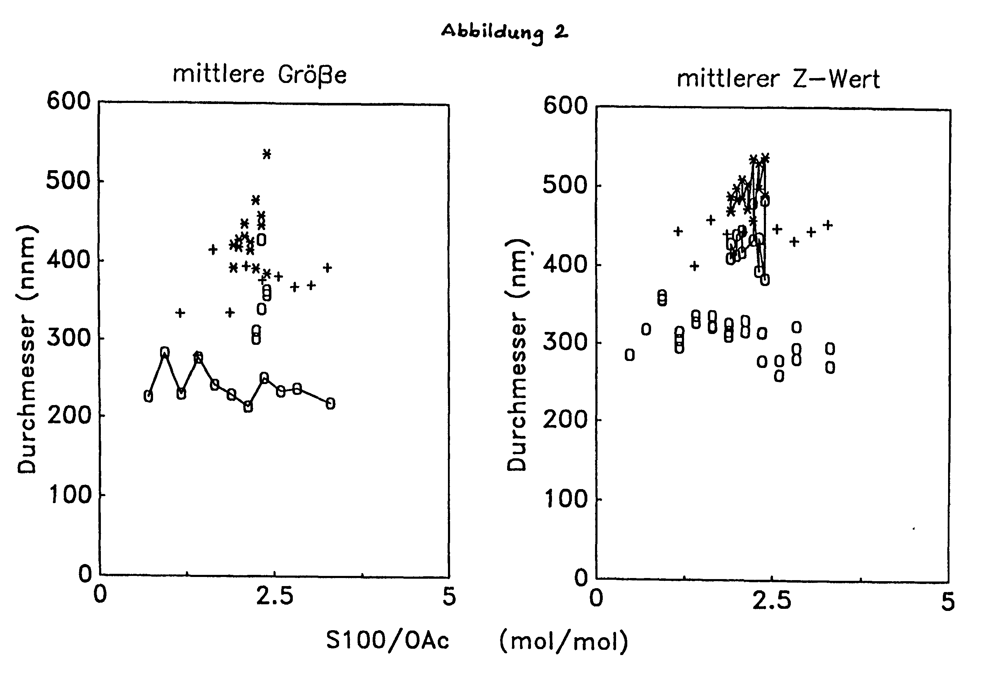

Die entsprechenden Meßdaten sind in der Abbildung 2 dargestellt.

### Beispiele 32-39:

### Zusammensetzung:

| | |
|---|---|
| 184.5-199.8 mg | Phosphatidylcholin aus Sojabohnen (+95 % = PC) |
| 20.5-22.2 mg | Phosphatidylglycerol aus Ei-PC (reinst, Na-Salz, =PG) |
| 44.9-26.1 µl | Ölsäure (+99 %) |
| 0.165-0.178 ml | Ethanol, absolut |
| 4.5 ml | Hepes, 10 mM |

### Herstellung:

Trockenes PG und alkoholische PC-Lösung werden durchgemischt, bis eine klare Lösung mit 90 % PC und 10 % PG vorliegt. Zu dieser Lösung wird Ölsäure hinzupipettiert; die resultierenden Lipid/Tensid-Verhältnise liegen zwischen 1.6 und 2.8; zusätzlich wird auch eine isomolare Probe gemacht. Diese Gemische werden mit jeweils 4.5 ml einer sterilen Pufferlösung vermengt (Lipidkonzentration 4 %) und nach dem Einstellen des pH-Wertes mit NaOH 3 Tage stehengelassen.

### Permeation und Trägercharakteristika:

Der Permeationswiderstand wird wie in den Beispielen 1-13 ermittelt. Die gemessenen Werte sind in der Regel kleiner als diejenigen, die für die ungeladenen Träger mit einem vergleichbarem L/T-Verhältnis charakteristisch sind; die niedrigere Lipidkonzentration spielt diesbezüglich eine untergeordnete Rolle, wie Experimente mit 4 % Suspension von PC und Olsäure gezeigt haben.

Auch im Falle von 4 % PC/PG-Gemischen ist ein Widerstandminimum zu finden; dieser liegt jedoch bei L/T-Werten, die um 20 % höher sind, als im Falle einer 8 % Lipidsuspension. Die Vesikeldurchmesser unterscheiden sich dagegen kaum von denjenigen, die in Beispielen 1-13 gemessen wurden.

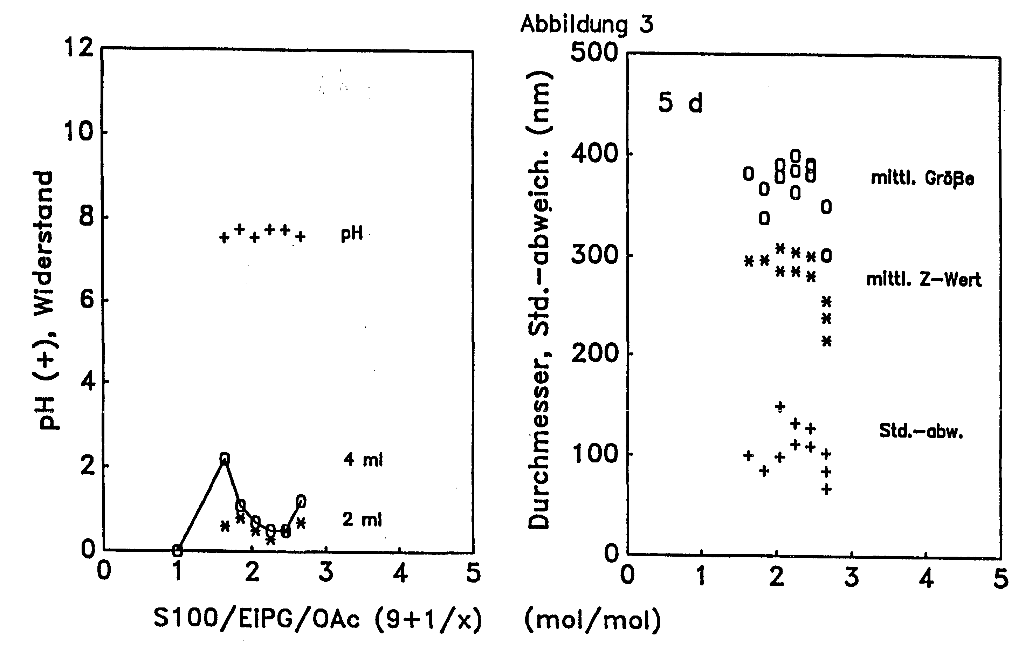

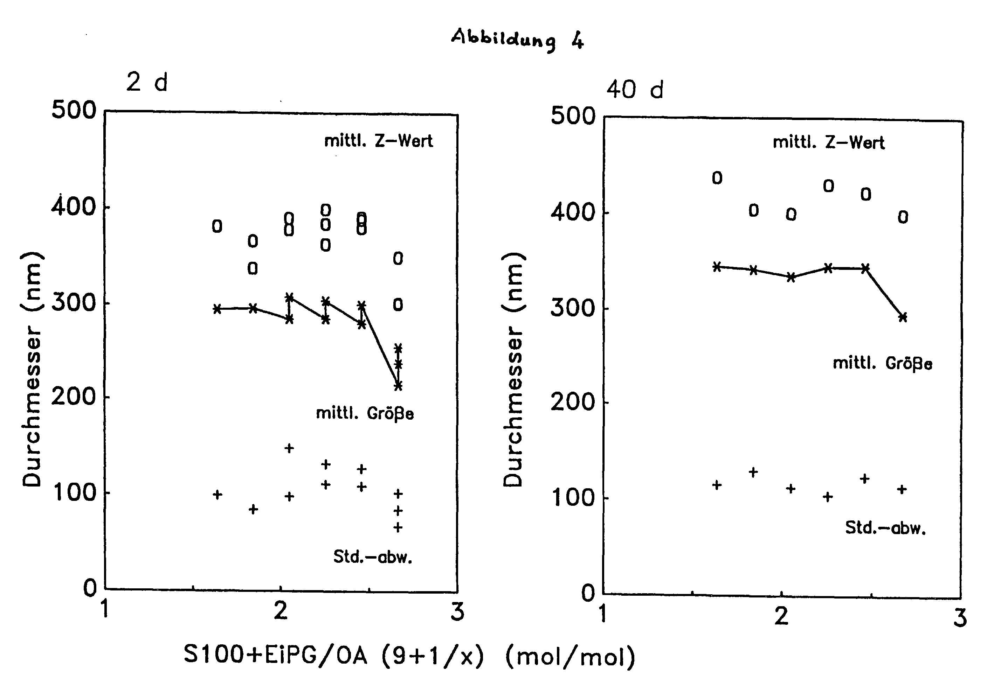

Die genauen Permeationsdaten sind in Abbildung 3 gezeigt. Die angegebenen Durchmesser wurden nach dem Permeationsversuch gemessen. Am Tag 40 nach der Herstellung sind sie jedoch kaum größer als am Anfang; Abbildung 4 illustriert das.

### Beispiele 40-49:

### Zusammensetzung:

| | |
|---|---|
| 301.3-335.4 mg | Phosphatidylcholin aus Sojabohnen (+95 % = PC) |
| 123.3-80.8 µl | Tween 80 (reinst) |
| 0.38-0.42 ml | Ethanol, absolut |
| 4.5 ml | Phosphatpuffer, isotonisch, steril |

### Herstellung:

In die entsprechenden Volumina einer alkoholischen PC-Lösung werden zunehmende Mengen von Tween 80 pipettiert. Dadurch entseht eine Konzentrationsreihe mit 12.5 bis 25 mol% Tensid (L/T = 4-8 ). Zusätzlich werden auch noch Probem mit L/T=2 und 3 hergestellt. Nach der Zugabe von Puffer entstehen Liposomen, die gleich danach mit Hilfe eines 0.8 Mikrometer-Filters etwas verkleinert werden.

### Permeation und Trägercharakteristika:

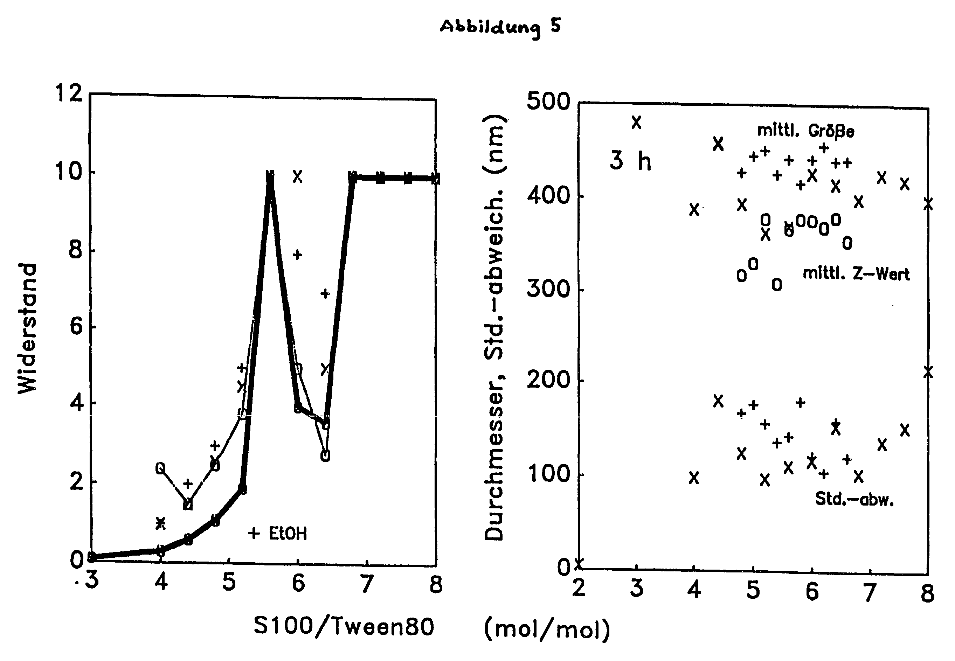

Der Permeationswiderstand wird auf die bereits beschriebene Weise gemessen. Die entsprechenden Werte (0) sind in dem linken Teil der Abbildung 5 gezeigt. Wie im Falle von ölsäurehaltigen Transfersomen ist relativ weit entfernt von dem Solubilisierungspunkt ein Bereich anomal hoher Permeationsfähigkeit (bei L/T = 6 ) zu sehen. Maximale Permeationsfähigkeit wird jedoch erst unterhalb von L/T=4 erreicht; das Transfersomenoptimum liegt also in einem Bereich, der sich um den Faktor 1.5-2 von dem Solubilisierungbereich unterscheidet.

Die genauen Permeationsdaten sind in Abbildung 5 gezeigt (breite Linien, linkes Bild). Die Meßdaten im rechten Bild dokumentierten die nach dem Permeationsversuch gemessenen Vesikeldurchmesser.

### Beispiele 50-61:

### Zusammensetzung:

| | |
|---|---|
| 314.2-335.4 mg | Phosphatidylcholin aus Sojabohnen (+95 % = PC) |
| 107.2-80.8 µl | Tween 80 (reinst) |
| 4.5 ml | Phosphatpuffer, isotonisch, steril |

### Herstellung:

Zu entsprechenden Mengen von PC werden zunächst Tween 80 und dann Phosphatpuffer, pipettiert. Das Gemisch wird auf einem Schüttler 4 Tage bei der Raumtemperatur gemischt. Danach wird wie in den Beispielen 40-49 verfahren.

### Permeation und Trägercharakteristika:

Die entsprechenden Permeabilitätsdaten sind in Abbildung 5 (dünne Striche) wiedergegeben. Sie bestätigen im wesentlichen die Ergebnisse der Beispiele 40-49.

### Beispiele 62-75:

### Zusammensetzung:

| | |
|---|---|
| 193-361 mg | Phosphatidylcholin aus Soja-Bohnen (Grade I, S100) |
| 207.2-38.8 mg | Na-Cholat, puriss. |
| 4.5 ml | Phosphatpuffer (isoton mit physiologischer Lösung) |
| | Ethanol, absolut |

### Herstellung:

Zu jeweils 0.5 mL einer heißen S100-Lösung in Ethanol (2/1, M/V) werden solche Mengen von Gallensäuresalz hinzugegeben, daß eine Reihe mit steigendem Lipid/Tensid-Verhältnis zwischen 1/2 und 5/1 entsteht. Die Gesamtlipidkonzentration am Ende ist jeweils 8 %.

### Vesikel-Permeation durch die Kontriktionen und Vesikel-Solubilisierung:

Der Permeationswiderstand der Proben wird wie in Beispielen 1-13 gemessen. Die Vesikelgröße wird mittels dynamischer Lichtstreuung bestimmt. (Radii von Teilchen, die kleiner sind als 5 nm, sind aufgrund der kleinen Leistung des verwendeten Lasers nicht erfaßbar.)

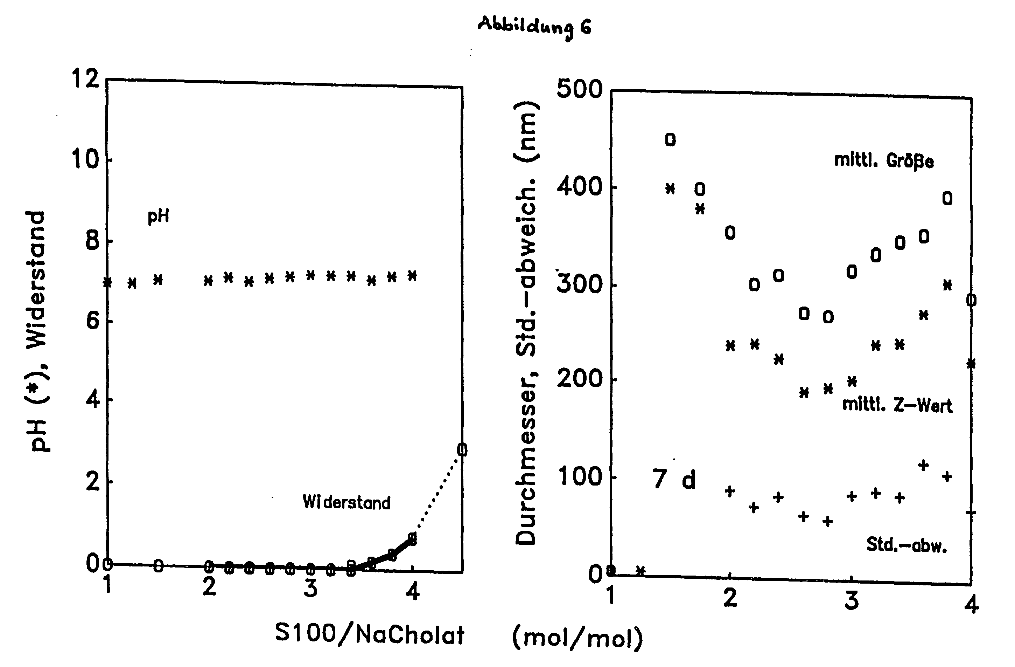

Die Meßergebnisse sind in Abb. 6 dargestellt. Sie zeigen, daß der Permeationswiderstand von Transfersomen mit einem L/T-Verhältnis unterhalb von 3.5/1 sehr klein ist, danach aber merklich zunimmt (linkes Bild); der Anstieg des mittleren Vesikeldurchmessers oberhalb von L/T = 2.75 (rechtes Bild) ist wahrscheinlich eine Folge verminderter Durchflußgeschwindigkeit (und daher verminderter hydrodynamischen Zerrkraft), die durch den gestiegenen Permeationswiderstand in diesem Konzentrationsbereich verursacht wird.

Unmittelbar oberhalb der Solubilisierungsgrenze (bei L/T zwischen 1.25/1 und 2.5/1) sind die Lipidvesikel bereits einige Stunden nach der Herstellung signifikant größer als in der Nähe des Transfersomen-Optimums'. Solche unerwünschte Folge der Tensidaktivität (siehe z.B. Fromherz, P. in: 'Galstone Disease, Pathopyxiology and Therapeutic Approaches, pp 27-33, Springer, Berlin, 1990) sollte immer berücksichtigt werden. Bei L/T von ca. 1.25/1 setzt die Solubilisierung ein, die zur Entstehung von kleinen, hier nicht mehr erfaßbaren, etwa 5 nm großen Mischmizellen führt.

### Beispiele 76-91:

### Zusammensetzung:

| | |
|---|---|
| 1.627-0.5442 g | Phosphatidylcholin aus Soja-Bohnen (Grade I, S100) |
| 4.373-0.468 g | Na-Cholat, puriss. |
| 60 ml | Phosphatpuffer (physiologisch) |

### Herstellung:

Eine 10 % Suspension von S100 in Phosphatpuffer wird bei Raumtemperatur mit Ultraschall behandelt, bis die mittlere Vesikelgröße ungefähr 350 nm erreicht hat.

Die Suspension wird in drei gleiche Volumenteile geteilt, die 10 %, 1% und 0.2 % Phospholipid enthalten. Aus diesen Volumina werden Aliquote mit je 5 ml Suspension gebildet. Diese werden mit steigenden Mengen von Natriumcholat versetzt (teilweise aus einer konzentrierten Mizellensuspension), die L/T-Verhältnise zwischen 1/5 und 5/1 ergeben. Vor jeder Permeations- und Solubilisierungsmessung werden die Ausgangs-Suspensionen 1 Woche bei 4°C gealtert.

### Vesikel-Permeation durch die Konstriktionen und Solubilisierung:

Um den Permeationswiderstand der Proben zu erfaßen, werden zwei Verfahren verwendet.

Im ersten Testansatz werden die Suspensionen unmittelbar vor jeder Messung auf die Lipidkonzentration von 0,2 % gebracht und anschließend mit einem kleinen Überdruck durch Filter mit 0,1 Mikrometer Porendurchmesser gepreßt. Der Widerstand wird dem Umkehrwert des Volumens, das innerhalb von 5 Minuten durch die Poren dringt, gleichgesetzt.

Im zweiten Testansatz wird der Permeationswiderstand der Proben wie in Beispielen 1-13 ermittelt und jeweils durch Division der Werte mit der Lipidkonzentration normiert.

Die entsprechenden Messdaten zeigen, daß sowohl die Solubilisierungsgrenze als auch die Position des 'Transfersomen-Optimums', ausgedrückt in Form des bevorzugten L/T-Verhältnisses, von der Lipidkonzentration abhängit: im Falle einer 10 % Suspension betragen die entsprechenden Werte um 1/1 und 2.75/1; für die 0,2 % Suspension steigen sie auf 1/4 und 1/1.

### Beispiele 92-98:

### Zusammensetzung:

| | |
|---|---|
| 16.3-5.4 mg | Phosphatidylcholin aus Soja-Bohnen (Grade I, S100) |
| 41.5-5.5 mg | Na-Desoxycholat, puriss. |
| 5 ml | Phosphatpuffer (physiologisch) |

### Herstellung:

Eine 1 % Suspension von desoxycholathaltigen Vesikeln wird wie in den Beispielen 76-91 beschrieben hergestellt.

### Vesikel-Permeation durch die Konstriktionen und Solubilisierung:

Die Messungen dieser Versuchsreihe zeigen, daß die desoxycholathaltigen Vesikel bereits bei L/T um 1/2, d.h. bei einem um den Faktor 2-3 niedrigeren L/T-Verhältnis, solubilisiert und permeationsfähig werden als die S100/Na-Cholat Vesikel.

### Beispiele 99-107:

### Zusammensetzung:

3 mM Suspension von Phosphatidylcholin aus Soja-Bohnen (Grade I, S100) in Phosphatpuffer Na-Cholat, puriss.

### Herstellung:

Eine 3 mM Suspension von S100 in Phosphatpuffer wird bei Raumtemperatur vorhomogenisiert. Zu je 3 ml dieser Suspension werden zunehmende Mengen von Natriumcholat gegeben, damit eine Reihe mit L/T-Verhältnisen zwischen 1/2 und 12/1 entsteht. Nach 3 tägiger Inkubation werden diese Aliquots bei 55°C im Pulsmodus beschallt und gleichzeitig die optische Dichte bei 400 nm aufgezeichnet. Die Analyse der Meßergebnisse mit einem biexponentiellen Modell ergibt zwei charakteristische Vesikularisierungswerte (tau 1 und tau 2), die die temporale Abhängigkeit der Vesikelschalenzahl (tau 1) und der Vesikelgröße (tau 2) charakterisieren.

### Vesikel-Charakteristika und Deformierbarkeit:

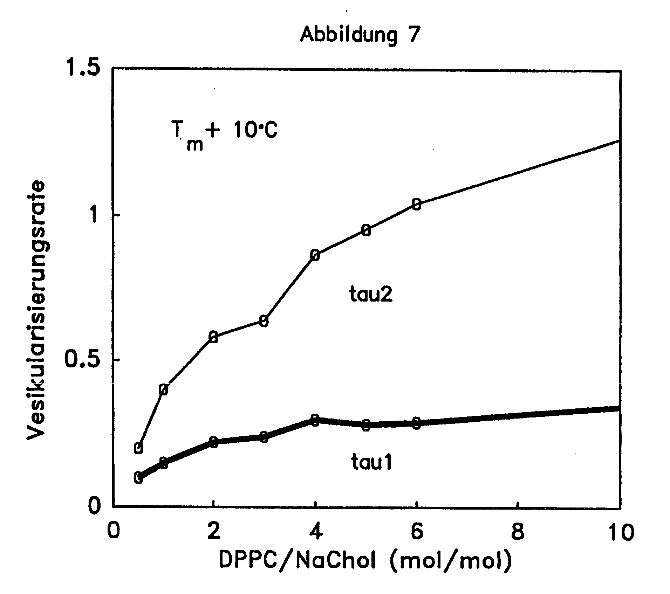

Die in Abb. 7 dargestellten Werte von tau 1 und tau 2 zeigen, daß die mechanische Eigenschaften von Transfersomen, die sich in dem Parameter tau 2 widerspiegeln, eine ähnliche L/T-Abhängigkeit aufweisen wie die Solubilisierung und Permeationsfähigkeit (vgl. Abb. 6). Für die hier untersuchte 0.2 % Suspension bedarf es ungefähr 1 Cholatmoleküls/Lipid, damit die Vesikularisierung (Bildung von geschlossenen, vorwiegend einschaligen Vesikeln) rasch vorangehen kann.

### Beispiele 108-119:

### Zusammensetzung:

| | |
|---|---|
| 121,2-418,3 mg | Phosphatidylcholin aus Soja-Bohnen (Grade I, PC) |
| 378,8-81,7 mg | Triton X-100 |
| 4.5 ml | 0.9 % NaCl Lösung in Wasser |

### Herstellung:

Eine 10 % PC-Suspension in isotonischer Kochsalzlösung wird bei 22°C homogenisiert, bis die mittlere Vesikelgröße ungefähr 400 nm beträgt. Diese Suspension wird in Aliquots von ca. 4,8 ml verteilt. Zu jedem von diesen Aliquots wird solches Volumen von Triton hinzugefügt, daß eine Reihe mit nominalem PC/Triton Verhältnis von 0,25 bis 4 in Schritten von 0,5 entsteht. Alle Suspensionen werden gelegentlich durchmischt und insgesamt 14 Tage bei 4°C gealtert.

### Vesikel-Solubilisierung:

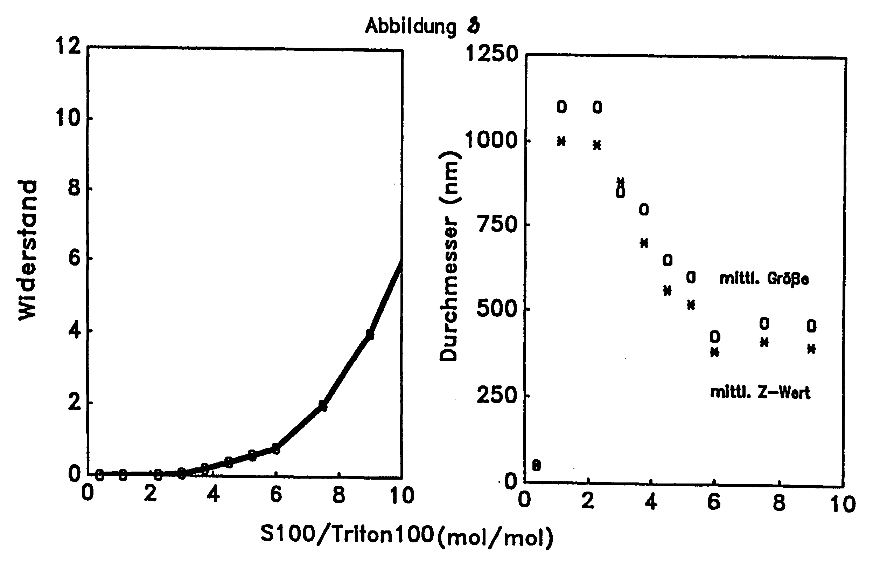

Die optische Dichte (OD (400 nm)) von (1/10 verdünnten) Lipid-Triton-Gemischen, die einen Einblick in die Vesikelsolubilisierung gibt, ist in dem rechten Teil von Abb. 8 dargestellt. Die Solubilisierungsgrenze liegt bei ungefähr 2 Tritonmolekülen je PC-Molekül. Unmittelbar unterhalb dieser Grenze sind die OD(400 nm) und daher die Vesikeldiameter am größten; oberhalb von PC/Triton 2,5/1 ist die Veränderung der optischen Dichte nur noch minimal.

### Vesikel-Permeation und -Charakteristika:

Um die Permeationsfähigkeit von entstandenen Lipidvesikeln und Transfersomen zu erfassen, wurden alle Suspensionen, wie in Beispielen 1-13 beschrieben, durch feinporige (0,22 Mikrometer) Filter gepreßt. Der dafür erforderliche Überdruck steigt graduell mit abnehmender Triton-Konzentration in der Suspension, und beschränkt oberhalb von L/T = 2/1 die Permeationsfähigkeit der Träger zusehends.

Die entsprechenden Ergebnisse sind in linken Hälfte der Abb. 8 zusammengefaßt.

### Beispiele 120-128:

### Zusammensetzung:

| | |
|---|---|
| 403,5-463,1 mg | Dipalmitoylweinsäureester, Na-Salz |
| 96,5-36,9 mg | Laurylsulfat, Na-Salz (SDS) |
| 4,5 ml | Triäthanolamin Puffer, pH 7.5 |

### Herstellung:

In dieser Versuchsreihe wurde ein synthetisches Lipid, das in biologischen Systemen nicht vorkommt, als Grundlage für die Transfersomen eingesetzt. Für die Experimente wurden entsprechende Mengen von Trockenlipid in Glasgefäßen mit je 4,5 ml Puffer gemischt. In diesem Puffer war so viel von Natriumdodecylsulfat (SDS) enthalten, daß das L/T-Verhältnis zwischen 2/1 und 6/1 variierte. Gut durchmischte Suspensionen wurden zuerst 24 Stunden bei Raumtemperatur gelagert und anschließend nochmals gut gemischt.

### Permeationsfähigkeit und Vesikelcharakteristika:

Die Liposomen werden durch ein 0,2 Mikrometer Filter gedrückt. Dabei wird der Permeationswiderstand gemessen. Vesikel mit einem L/T-Verhältnis unterhalb von 4/1 passieren sehr leicht die Membranporen, während die Vesikel mit einem geringeren Tensidgehalt oder Vesikel ohne Zusatz von randaktiven Komponenten nur schwer (erst bei einem Überdruck von mehr als 5 MPa) oder gar nicht (die Membranen platzen) durch die Konstriktionen gelangen.

### Beispiele 129-136:

### Zusammensetzung:

| | |
|---|---|
| 101,6-227 mg | Phosphatidylcholin aus Soja-Bohnen |
| 148,4-22,2 mg | Octyl-glucopyranosid (β-Octylglucosid), puriss. |
| 9,85 ml | Phosphatpuffer, pH 7,3 |
| | Ethanol, absolut |

### Herstellung:

Phosphatidylcholin in Ethanol (50 %) und Octyl-glucopyranosid werden in unterschiedlichen relativen Mengen gemischt, um eine steigende Konzentrationsreihe mit L/T zwischen 1/4 und 2/1 (und einem Endlipidgehalt von 2,5 %) herzustellen. Zu jedem Lipidgemisch werden in einem Glasgefäß 4,5 ml Puffer hinzugefügt. Die Suspension wird auf einem Schüttler bei 25°C 48 Stunden lang gemischt. Ihre Trübung nimmt mit abnehmender Menge von Octylglucosid in der Probe zu. In den stehenden Proben bildet sich ein feiner Niederschlag. Vor Permeationsmessung wird jede Probe gut durchgemischt.

### Vesikel-Permeation und -Charakteristika:

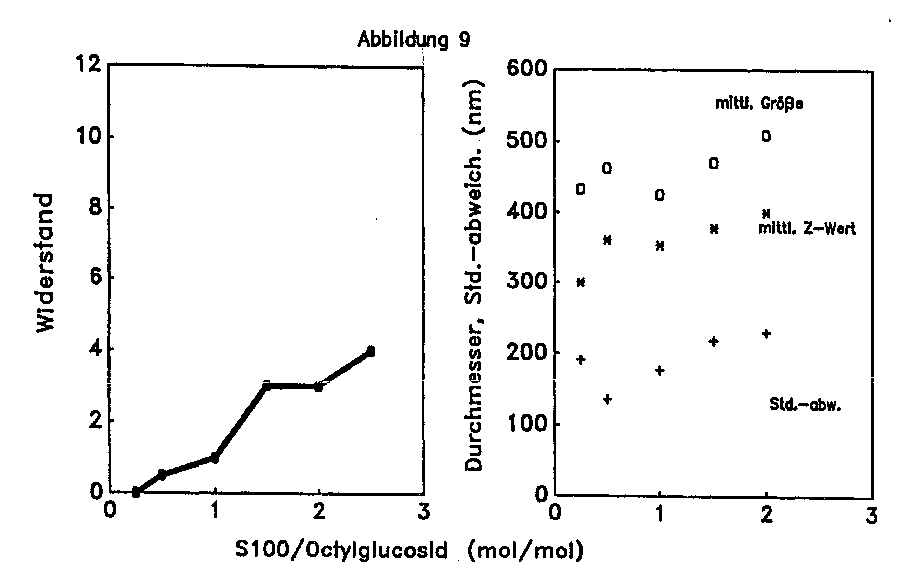

Alle Suspensionen lassen sich mit einem minimalen Überdruck unterhalb von 0,1-0.2 MPa problemlos durch ein Filter mit einem Porendurchmesser von 0,2 Mikrometer durchdrücken; lediglich die beiden Proben mit dem niedrigsten Tensidgehalt weisen einen kleinen Widerstand auf, der auf der renormierten Skala (gemäß Abbildungen 1-5) Werte um 1 und 2,5 annimt. Die Meßergebnisse sind in Abb. 9 präsentiert.

Wird der Porendurchmesser auf 0,05 Mikrometer herabgesetzt, sind nur noch die Suspensionen mit einem L/T-Verhältnis unterhalb von 2/1 filtrierbar.

Unabhängig von der verwendeten Porengröße sind die Präparationen mit einem L/T Verhältnis unterhalb von 2/1 jodoch nicht stabil; nach wenigen Tagen kommt es zu einer Phasentrennung zwischen einer mizellenreichen und einer visikelreichen Phase.

### Beispiele 137-138:

### Zusammensetzung:

| | |
|---|---|
| 43,3 mg, 50 mg | Phosphatidylcholin aus Soja-Bohnen |
| 0,5 mg | Phosphatidylethanolamin-N-Fluorescein |
| 6,7 mg, 0 mg | Cholat, Na-Salz, p.a. |
| 5 ml | Hepes-Puffer, pH 7,3 |

### Herstellung:

Phosphatidylcholin mit 1 %-Zusatz eines fluoreszierenden Lipidmarkers mit oder ohne Desoxycholat werden in 5 ml Puffer aufgenommen. Das Lipid/Tensid-Verhältnis liegt bei 3,5/1 bzw. 1/0. Beide 1 %-Suspensionen werden in einem Glasgefäß 1,5 bzw. 15 Minuten lang ultrabeschallt (25 W, 20°C), bis sie nur noch Vesikel mit mittleren Durchmesser von ca. 100 nm enthalten.

### Spontane Vesikel-Permeation:

Auf je ein Millipore-Filter mit Porendurchmesser von 0,3 Mikrometer in Swinney-Halterung, von der unteren Seite benetzt und zur Hälfte mit Wasser gefüllt, werden durch die obere Öffnung jeweils 50 Mikroliter der Lipidsuspension pipettiert. Durch leichtes Schwenken wird die Probe möglichst gleichmäßig verteilt und für 30 Minuten stehengelassen. Nach vorsichtigem Öffnen der Halterung trocknet der Lipidfilm innerhalb von 60 Minuten aus. Danach wird das Wasser, das sich in der Halterung unter der Membran befindet, abgezogen und fluoreszenzspektrometrisch untersucht (Exzitation 490 nm, Emission 590 nm). (Die gemessene Lichtintensität ist ein Maß für die Permeationsfähigkeit.)

Der durch die tensidhaltigen Transfersomen vermittelte Fluoreszenzmarkertransport führt zu einem Fluoreszenzsignal von 89,5; der Kontrollwert beträgt 44,1. Das zeigt, daß die Transfersomen fähig sind, die eingeschlossenen Stoffe effizient über die Permeabilitätsbarrieren zu transportieren.

### Beispiele 137-139:

Zusammensetzung:

| | |
|---|---|
| 43,5, 45,3, 50 mg | Phosphatidylcholin aus Soja-Bohnen |
| 0,5 mg | Phosphatidylethanolamin-N-Fluorescein |
| 6,5, 4,7, 0 mg | Desoxycholat, Na-Salz, p.a. |
| 5 ml | Hepes-Puffer, pH 7,3 |

### Herstellung und Resultate:

Die Lipidvesikel werden wie in Beispielen 137-138 beschrieben hergestellt und getestet. Die Messungen zeigen, daß die desoxycholathaltigen Transfersomen bereits bei einem charakteristischen Verhältnis L/T=5/1 ähnlich gute Ergebnisse liefern wie cholathaltigen Transfersomen mit L/T=3.5.

### Beispiele 140-142:

### Zusammensetzung::

| | |
|---|---|
| 50 mg; 43,3 mg; 15,9 mg | Phosphatidylcholin aus Soja-Bohnen |
| 0,5 mg | Phosphatidylethanolamin-N-Fluorescein |
| 0 mg; 6,7 mg; 34,1 mg | Cholat, Na-Salz, p.a. |
| 5 ml | Hepes-Puffer, pH 7,3 |

### Herstellung:

Lipidvesikel aus Phosphatidylcholin mit fluoreszierendem Lipidzusatz werden wie in Beispielen 137-138 hergestellt. Für den Versuch werden Suspensionen mit einem Lipid/Tensid-Verhältnis von 1/0, 4/1 und 1/4 verwendet. Die ersten beiden Proben enthalten fluoreszierende Lipid-Vesikel, die letzte Probe eine Mizellensuspension.

### Spontane Penetration in Pflanzenblätter:

Eine frische Zwiebel wird vorsichtig zerpflückt, um einzeln Schalen, die chlorophyllarmen Pflanzenblättern entsprechen, zu gewinnen. Jeweils 25 Mikroliter der fluoreszierenden Suspension werden auf die konkave Innenseite der Zwiebelknollenschalen aufgetragen; sie bilden dort einen konvexen Tropfen mit ca. 0,25 Quadratzentimeter Fläche. (Die tensidhaltigen Träger sind an ihrem besseren Benetzungsvermögen leicht erkennbar.) Nach 90 Minuten wird der (makroskopisch) trockengewordene Lipidfilm mittels Wasserstrahl aus einer Spritzflasche mit jeweils 50 mL abgespült.

Die 'Blattoberfläche' erscheint nach dieser Behandlung im Falle von tensidhaltigen Transfersomen bzw. Mizellen makroskopisch leicht rötlich. Blätter, die mit tensidfreien Vesikeln inkubiert waren, sind von den nichtbehandelten Blättern nicht zu unterscheiden.

Fluoreszenzmikroskopische Untersuchungen durch ein Rotfilter (Anregung durch ein Blaufilter in Auflicht) zeigen, daß die Blätter, die mit Transfersomen bedeckt waren, über die ganze behandelte Fläche intensiv fluoreszieren; an einigen Stellen sind extrem brilliante Aggregate zu erkennen, die wahrscheinlich den nichtentfernten Vesikel-Clustern entsprechen.

Die Fluoreszenz der Blätter, die mit der Tensidlösung behandelt waren, ist an manchen Stellen vergleichbar intensiv, andererorts etwas schwächer als die Fluoreszenz der mit Transfersomen behandelten Blätter.

Die Blätter, die mit normalen Lipidvesikeln behandelt waren, fluoreszieren nicht. Sie sind über weite Teile der Oberfläche nicht von den Blatt-Teilen, die nicht behandelt waren, zu unterscheiden.

Das zeigt, daß Transfersomen im Stande sind, lipophile Substanzen spontan und irreversibel in das Blatt oder seine Oberfläche zu transportieren. In dieser ihrer Eigenschaft übertreffen sie die Präparate mit hochkonzentrierten Tensiden, d. h. anerkannten 'Membranfluidisatoren'.

### Beispiele 143-145:

### Zusammensetzung:

| | |
|---|---|
| 50 mg; 43,5mg; 17,1 mg | Phosphatidylcholin aus Soja-Bohnen |
| 0,5 mg | Phosphatidylethanolamin-N-Fluorescein |
| 0 mg; 4,7 mg; 32,9 mg | Desoxycholat, Na-Salz, p.a. |
| 5 ml | Hepes-Puffer, pH 7,3 |

### Herstellung und Resultate:

Die Herstellung und die Ergebnisse sind im wesentlichen identisch mit denen aus Versuchen 140-142.

### Beispiele 146-148:

### Zusammensetzung:

| | |
|---|---|
| 50 mg; 36,4; 20 mg | Phosphatidylcholin aus Soja-Bohnen |
| 0,5 mg | Phosphatidylethanolamin-N-Fluorescein |
| 0 mg; 13,6 mg; 30 mg | Brij 35 |
| 5 ml | Wasser |

### Herstellung und Resultate:

Die Herstellung und die Ergebnisse sind vergleichbar den Resultaten der Versuche 140-142 und 143-145.

### Beispiele 146-150:

### Zusammensetzung:

| | |
|---|---|
| 84,2 bis 25 mg | Phosphatidylcholin aus Soja-Bohnen 80% |
| 75 kBq | Giberellin A4, 3H-markiert |
| 15,8 bis 75 mg | Polyoxyäthylen (23)-Lauryläther (Brij 35) |
| 1 ml | Wasser |
| | Ethanol, absolut |

### Herstellung:

Ethanolische Lipidlösung (50%) wird mit der entsprechender Menge einer ethanolischen Giberellinlösung vermischt und in 1 ml Wasser bzw. in entsprechende Volumina von Tensidsuspensionen gespritzt, die 10 %-Lipidkonzentration und L/T-Verhältnise von 8/1, 4/1, 2/1, 1/1 und 1/2 gewährleisten. Die Suspension wird mit Ultraschall kurz homogenisiert, damit die mittlere Vesikelgröße immer unter 300 nm ist.

Trägersuspensionen werden über die Oberfläche von jeweils 3 Blättern eines Ficus Benjaminii verteilt; dort trocknen sie 6 Stunden lang. Nach dem anschließenden, intensiven Waschen der Blattoberflächen mit jeweils 5 ml Wasser pro Quadratzentimeter Fläche wird nach dem Entfärben der Blätter mit Peroxid die Radioaktivität in dem Blatthomogenisat szintigraphisch in einem Beta-Zähler bestimmt.

### Wirkstofftransport in Pflanzenblätter:

Die Messungen zeigen, ähnlich wie bei Beispielen 140-142, daß Wirkstoffmoleküle mittels Transfersomen wesentlich effizienter als mit einer Mizellenlösung in die Blattoberfläche getragen werden.

### Beispiele 151-157:

### Zusammensetzung:

| | |
|---|---|
| 32,8-0,64 mg | Phosphatidylcholin aus Soja-Bohnen (reiner als 95 %, PC) |
| 75 kBq | Dipalmitoylphosphatidylcholin, Tritium-markiert |
| 2,2-34,4 mg | Gallensäure, Na-Salz, p.a. |
| 0,32 ml | Phosphatpuffer, pH 7,3 |

### Herstellung:

Jeweils 35 mg Lipid werden mit Tritium-markiertem Dipalmitoylphosphatidylcholin in Chloroform vermischt. Nach dem Vakuumtrocknen werden die Gemische in 0,32 ml Puffer suspergiert; die nominalen Tensid/Lipid-Verhältnise betragen 0; 0,125; 0,167; 0,263; 0,5 und 1 mol/mol. Die Suspensionen werden beschallt, bis sie alle (bis auf die letzte, klare Mizellensuspension) vergleichbar opaleszent sind. (Die erforderlichen Beschallungszeiten nehmen mit dem steigenden T/L-Verhältnis ab.) Vergleichsmessungen mit kalten Suspensionen zeigen, daß die mittlere 'Teilchen'-Größe in den Proben um 100 nm sein muß. Für die Experimente werden 1 Tag alte Suspensionen verwendet.

### Penetration in und durch die Haut:

Auf dem Rücken einer mit Äther narkotisierten, immobilisierten Nacktmaus werden sechs 1x1 cm große Areale markiert. Auf jedes von diesen werden in 3x5 Minuten Abständen jeweils 20 Mikroliter der Trägersuspension aufgetragen. Nach 60 Minuten wird die Maus getötet. Von jedem Hautareal wird eine Probe entnommen, die verkleinert, aufgelöst und entfärbt wird. Die hautassoziierte Radioaktivität wird szintigraphisch bestimmt.

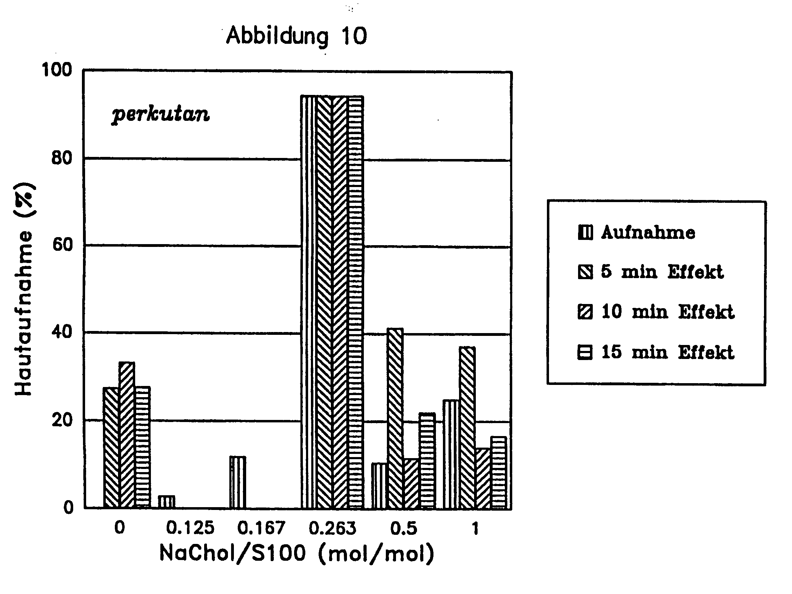

Die entsprechenden Ergebnisse sind in Abb. 10 zusammengefaßt. Als Vergleich ist die normalisierte Wirkung angegeben, die aus unserer Patentanmeldung zur Verwendung von Liposomen zur örtlichen Betäubung übernommen ist. Optimierte Transfersomen sind den nichtoptimalen, aber tensidhaltigen Präparaten klar überlegen.

### Beispiele 158-162:

### Zusammensetzung:

| | |
|---|---|
| 31 mg | Phosphatidylcholin aus Soja-Bohnen (reiner als 95 %, PC) |
| 75 kBq | Dipalmitoylphosphatidylcholin, Tritium-markiert |
| 4 mg | Deoxycholat, Na-Salz, p.a. |
| 0,32 ml | Phosphatpuffer, pH 7,3 |

### Herstellung:

Jeweils 35 mg Lipid (PC und Deoxycholat) werden mit Tritium-markiertem Dipalmitoylphosphatidylcholin in Chloroform vermischt. Das Lipidgemisch wird getrocknet und in 30 Mikroliter warmem, absoluten Ethanol aufgenommen. Diese Lösung wird mit 0,32 ml Puffer (Phosphat 10 mM, 0,9 % NaCl) vermengt; das entspricht L/T = 4/1 . Die entstandene Suspension wird kräftig durchgeschüttelt und anschließend sequentiell durch 0,8; 0,45; 0,22 und 0,1 Mikrometer-Filter gepreßt, um Lipidvesikel mit einem Durchmesser von ca. 800, 400, 200 bzw. 100 nm zu erzeugen (Suspensionen A, B, C, D).

### Penetration in und durch die Haut:

Die Schwänze von je 2 narkotisierten Mäusen werden über einen Zeitraum von 15 min mit jeweils 50 Mikroliter von entsprechenden Vesikelsuspension bestrichen. Zwei Kontrolltiere erhalten eine i.v. Injektion von 0,2 ml 1/10 verdünnter Suspension B. Nach 30, 60, 120, 180, 240 und 360 Minuten werden aus der Schwanzspitze Blutproben entnommen. Die Radioaktivität dieser Proben, die mittels

Betastrahlenszintigraphie besttimmt wird, wiederspiegelt die systemische Konzentration von trägerassoziiertem, radioaktiv markierten Lipid.

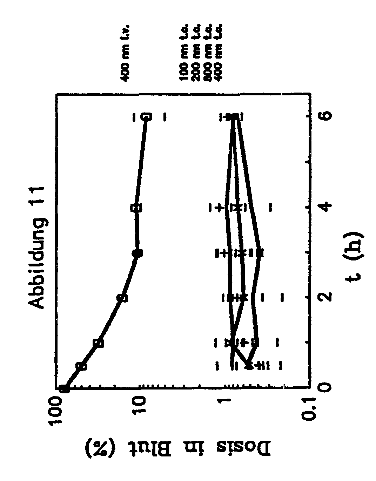

Die Messdaten zeigen (Abb. 11), daß systemisch verabreichte Transfersomen vergleichbar schnell aus dem Blut entfernt werden wie Standardliposomen. Die Trägergröße scheint die spontane Penetration der Haut nicht signifikant zu beeinflußen. Alle in dieser Versuchsreihe untersuchten Transfersomen dringen nach 4 Stunden zu ca. 1 Träger in die Tiefe des Körpers, Tendenz steigend.

### Beispiele 163-165:

### Zusammensetzung:

| | |
|---|---|
| 88 mg | Phosphatidylcholin aus Soja-Bohnen (reiner als 95 %, PC) |
| 75 kBq | Inulin, Tritium markiert |
| 12 mg | Deoxycholat, Na-Salz, p.a. |
| 100 ml | Ethanol, absolut |
| 0,9 ml | Isotonische Kochsalzlösung |

### Herstellung:

100 mg PC in 100 ml warmem Ethanol, oder eine entsprechende PC/Deoxycholat Lösung (L/T = 4,5 ), werden in jeweils 0,9 ml isotonischer Kochsalzlösung aufgenommen (Suspensionen A und B, respektive). Jede Suspension wird beschallt, bis die Vesikelgröße um 150 nm ist.

Zu 38 Mikrolitern von frischer Suspension leerer Liposomen (A) oder Transfersomen (B) werden 12 Mikroliter einer wässrigen Lösung von Tritium-markiertem Inulin pipettiert. Die Gemische werden ansschließend in verschlossenen Gefäßen 60 Minuten im Ultraschallbad bei Raumtemperatur nachbeschallt und 24 Stunden später für die Versuche verwendet.

### Spontane Inulinübertragung durch die Haut:

Auf die (3 Tage vorher) mit Pinzette enthaarten Bäuche von narkotisierten NMRI-Mäusen werden auf ca. 1 cm² Fläche jeweils zweimal 10 Mikroliter der inulinhaltigen Vesikel in 3-5 minütigem Abstand aufgetragen. Nach 15, 30, 60, 120, 180, 240, 300 und 360 Minuten werden jeweils 0,05 ml Blut aus dem Schwanz entnommen und szintigraphisch untersucht. Nach 6 Stunden wird subcutanes Gewebe der Auftragsstelle, sowie die Leber und Milz der Versuchstiere entnommen; nach dem Auflösen und Entfärben werden diese Organe ebenfalls szintigraphiert.

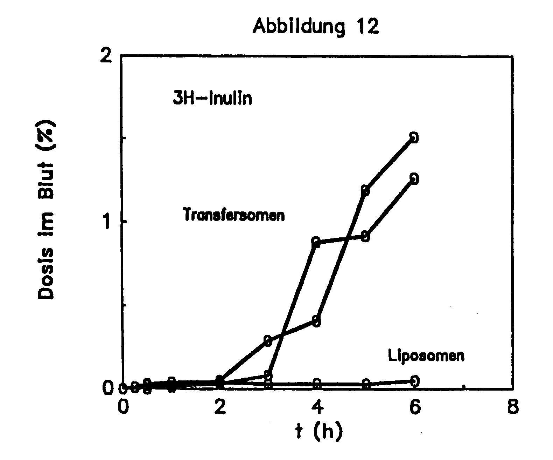

Die Versuchsergebnisse sind in Abb. 12 zusammengefaßt. Sie zeigen, daß normale Liposomen keine perkutane Inulinaufnahme vermitteln. Im Gegensatz dazu gelangen nach 6 Stunden ca. 1,4 % des mittels Transfersomen applizierten Markers ins Blut. Die Übertragung setzt nach etwa 2-3 Stunden ein und ist nach 6 Stunden noch nicht abgeschlossen.

Nach 6 Stunden sind im Falle von Transfersomen durchschnittlich 0,8 % (das entspricht 24,1 % der wiedergefundenen Dosis) in der Haut der Auftragsstelle; 0,9 % werden in der Leber gefunden; in der Milz sind weniger als 0,1 % der absoluten Dosis enthalten. Im Körper (Blut, Milz, Leber) befinden sich also 73,8 % der wiedergefundenen Dosis.

Im Gegensatz dazu sind ungefähr 2 % von den normalen Liposomen an der Auftragsstelle wiederzufinden, während die Dosis in

Leber und Milz unterhalb von 0,1 % liegt. Das entspricht 95,3 % der wiedergefundenen Dosis an der Auftragsstelle und 6,7 % solcher Dosis im Körper der Versuchsmaus.

### Beispiel 166:

### Zusammensetzung:

| | |
|---|---|
| 386 mg | Phosphatidylcholin aus Sojabohnen (reiner als 95 %) |
| 58,5 mg | Natrium-Cholat (L/T = 3,5) |
| 500 ul | Ethanol (96 %) |
| 2,25 ml | 0,9 % NaCl-Lösung (pro Injekt.) |
| 2,25 ml | Actrapid HM 40 (entspricht 90 I.U. rekombinantes Humaninsulin) |

### Herstellung:

Die Herstellung erfolgt im wesentlichen wie in Beispielen 62-75 beschrieben. Zu der Lipidlösung im Ethanol wird ein Gemisch von wässriger Lösung aus Kochsalz und humanem, rekombinanten Insulin (mit 6,75 mg m-Cresol) hinzugefügt. Es entsteht eine trübe Suspension, die über Nacht gealtert wird. Nach 12 Stunden wird diese Suspension mittels Stickstoffgas mit einem Druck von 0,25 MPa unter sterilen Bedingungen durch ein Sterilfilter (Anodisc, Porendurchmesser 0.2 Mikrometer) gepreßt und anschließend abgepackt.

Das nominale Lipid/Tensidverhältnis beträgt 3,5, die berechnete molare Tensidkonzentration in der Lipiddoppelschicht ca. 5/1. Das entspricht 50 % der Solubilisierungskonzentration.

Der mittlere Vesikelradius der fertigen Suspension in dieser Präparation beträgt 97 nm.

### Anwendung:

0,5 ml einer frischen, insulinhaltigen Transfersomen-Suspension werden auf die unvorbehandelte Haut am linken Unterarm einer informierten, freiwilligen, gesunden, männlichen, seit 18 Stunden nüchternen Testperson (37 Jahre) aufgetragen und über ca. 10 cm² verteilt. 5 Minuten später werden noch 300 Mikroliter derselben Suspension zu jeweils einer Hälfte auf den Unter- und Oberarm plaziert. 5-10 Minuten später ist die Suspension am Oberarm (Dosis ca. 2,5 mg/cm²) nicht mehr sichtbar, also vollkommen eingedrungen, während am Unterarm (Dosis ca 7,5 mg/cm²) sind zu diesem Zeitpunkt die Lipidreste noch gut sichtbar.

### Wirkung:

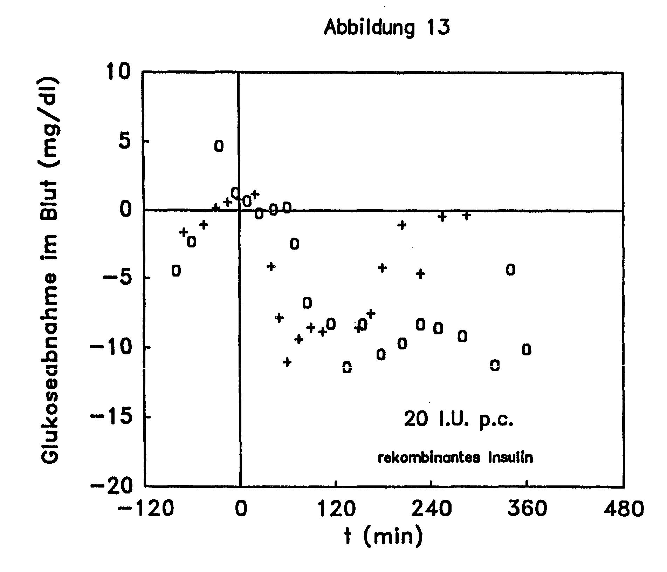

Um die Insulinwirkung zu erfaßen, wird am rechten Handgelenk ca. 2 Stunden vor dem Probeauftrag ein i.v. Katheter positioniert. In Zeitabständen von 15-45 Minuten werden jeweils 1-1,5 ml Blut gezapft; die ersten 0,5-1 ml davon werden verworfen und die restlichen 0,5 ml für den üblichen enzymatischen Glucosetest verwendet. Es werden jeweils drei Bestimmungen mit drei bis vier unabhängigen Proben durchgeführt. Die Messergebnisse sind in der Abbildung 13 zusammengefaßt. Sie zeigen, daß mittels Transfersomen ca. 90 Minuten nach dem Auftrag eine signifikante Blutglucosesenkung eintritt, die ungefähr 2 Stunden dauert und ca. 50 % der Höhe und 200 % der Dauer der Wirkung einer vergleichbaren subkutanen Insulinapplikation erbringt.

### Beispiel 167-172:

### Zusammensetzung:

| | |
|---|---|
| 956 mg | Phosphatidylcholin aus Sojabohnen (+95 %) |
| 0-26 mg | Natrium-Deoxycholat |
| 1 mg | Prostaglandin E1 |
| 1 ml | Ethanol absolut |
| 50 ml | 0,9 % NaCl-Lösung (pro Injekt.) |

### Herstellung:

In ein Glasfläschchen mit 1 mg Prostaglandin wird 1 ml Ethanol pipettiert. Nach Durchmischen wird die Prostaglandinlösung zu dem Trockenlipid in einem anderen Glasgefäß übertragen. Mit der neuen Lipid/Prostaglandinlösung wird das ursprüngliche Fläschchen nochmals gespült und anschließend mit 6 ml einer isotonischer Kochsalzlösung versetzt. Das Prostaglandin-Fläschchen wird mit weiteren 2x2 ml von 0,9 % NaCl gewaschen und dies mit der ursprünglichen Lipidsuspension vermischt. Die Probe wird fünfgeteilt; in die einzelnen Aliquots wird Natrium-Desoxycholat eingewogen und zwar 0; 1,6; 3,25; 6,5 bzw. zweimal 13 mg/ml.

Die resultierenden 10 % Suspensionen werden 24 Stunden gealtert und anschließend, je nach dem Desoxycholatgehalt, ultrabeschallt bzw. manuell durch ein 0,2 Mikrometer-Filter gepreßt. Die Proben mit dem höchsten Tensidgehalt werden entweder mittels Filtration oder mit Ultraschall erzeugt. Zuletzt werden die Suspensionen auf 20 Mikrogramm PGE1/ml verdünnt und in dunklen Spritzflaschen im Kühlschrank aufbewahrt. Der Vesikelradius gleich nach der Herstellung war 85 nm, nach 2 Monaten 100 nm.

### Anwendung und Wirkung:

Jeweils 0,25 ml der Lipidsuspensionen werden auf benachbarte, aber nicht zusammenhängenden Areale der Bauchhaut augetragen. Nach 10 Minuten ist die Hautoberfläche trocken; nach 15 Minuten ist an einigen Applikationsstellen leichte Rötung zu beobachten, die nach Probandenbericht mit einem stumpfen Schmerzgefühl einhergeht. Der Rötungsgrad wurde mit 0, 0, 0, 0-1, 3 und 3 Punkten (auf einer Skala von 1-10) eingestuft.

Dieses Ergebnis zeigt, daß lediglich Transfersomen, nicht aber normale Liposomen oder unoptimierte, detergenshaltigen Vesikel für die Wirkstoffpenetration taugen. Die Herstellungsart ist für diese Anwendung irrelevant.

### Beispiele 173-175:

### Zusammensetzung:

| | |
|---|---|
| 79,4 mg; 88,5 mg | Phosphatdylcholin aus Sojabohnen (+95%) |
| 20,6 mg, 11,5 mg | Natrium-Deoxycholat |
| 10 µg | Hydrocortison |
| 0,1 ml | Ethanol absolut |
| 1 ml | Phosphatpuffer, physiologisch |

### Herstellung:

Lipide und Hydrocortison werden als ca. 50 % ethanolische Lösung gemischt und anschließend mit 0,95 ml Phosphatpuffer versetzt. Die dabei entstehende, sehr heterogene Suspension wird mittels Utraschall (25 W, 3-5 min) nachbehandelt. Proben mit L/T-Verhältnis von 2/1 lassen sich gut, Proben mit L/T = 4/1 dagegen vergleichsweise schlecht homogenisieren.

Proben mit 1 und 2,5 Gew.-% ergeben unabhängig von dem L/T Verhältnis stabile Suspensionen; 10 Gew.-% Wirkstoff lassen sich nicht stabil in Transfersomen der gegebenen Zusammensetzung einarbeiten.

### Beispiele 175-200:

### Zusammensetzung:

| | |
|---|---|
| 1,1 - 2mg | Phosphatidylcholin aus Sojabohnen (+95%=PC) |
| 0 - 32,5 mol% | Tween 80 |
| ph 7,2 | isotoner Phosphatpuffer |

### Herstellung:

In jeweils 25 ml Puffer werden unterschiedliche Mengen von Phospholipid und Tensid eingewogen bzw. einpipettiert, damit eine Konzentrationsreihe mit 0 - 32,5 Mol% Tween 80 bei gleichbleibender 2% Gesamtlipidkonzentration entsteht. Die Proben werden steril abgefüllt und 4 bis 34 Tage gealtert. Anschließend wird ihre optische Dichte bestimmt. Diese ist stark vom Tensidgehalt, aber im Rahmen der Meßbedingungen kaum zeitabhängig.

### Charakterisierung:

Jeweils 23 Proben von je 3 ml aus einzelnen Lipidsuspensionen werden in verschlossenen Gefäßen in einem Utraschallbad beschallt. Nach drei, vier und sechs Stunden wird ihre Trübung gemessen. Dieser Vorgang wird mit einer neuen Versuchsreihe wiederholt, wobei die Positionen von einzelnen Probem systematisch variiert werden; die Trübungsmessung erfolgt wieder nach drei, vier und sechs Stunden. Die entsprechenden, zu einer Konzentration gehörenden Werte werden gemittelt und als Maßstab für die Vesikularisierungsfähigkeit der Probe betrachtet.

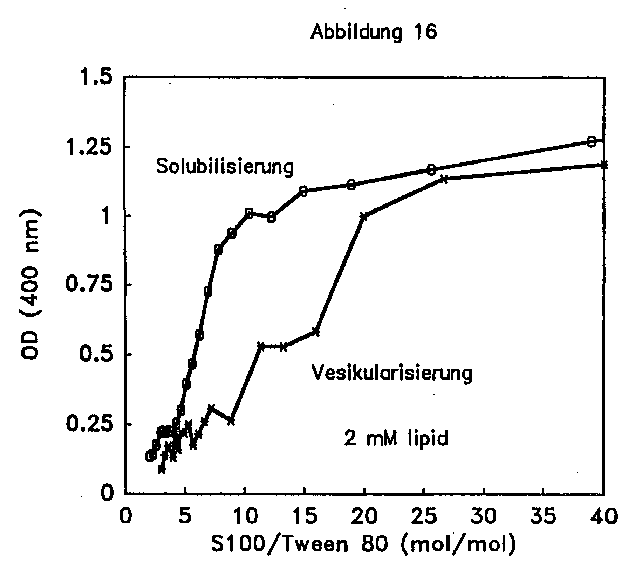

Dieses Verfahren kann als eine Ergänzung bzw. Alternative zur Resistenzmessung, wie sie in Beispielen 40.49 beschrieben ist, betrachtet werden. Abb. 16 zeigt z.B., daß die für eine gute mechanische Deformierbarkeit erforderliche Tensidmenge im Falle von Tween 80 etwa 2- bis 3-fach niedriger ist, als die entsprechende Solubilisierungsmenge. Dieses Ergebnis ist um guten Einklang mit den Resultaten der Permeationsversuche.

### Beispiele 201-215

### Zusammensetzung:

| | |
|---|---|
| 256,4-447 mg | Phosphatidylcholin aus Sojabohnen (+95%=PC) |
| 243,6-53,1 mg | Brij 96 |
| 0,26-0,45 ml | Ethanol, absolut |
| 4,5 ml | Phosphatpuffer, ph 6,5, 10 mM |

### Herstellung:

In die entsprechenden Volumina einer alkoholischen PC-Lösung werden zunehmende Mengen von Brij 96 pipettiert. Dadurch entsteht eine Konzentrationsreihe mit L/T zwischen 1/1 und 1/8. Nach der Zugabe von Puffer entstehen sehr heterogene Liposomen, die mittels Filtration durch einen 0,2 µm Filter homogenisiert werden.

### Permeation und Trägercharakteristika:

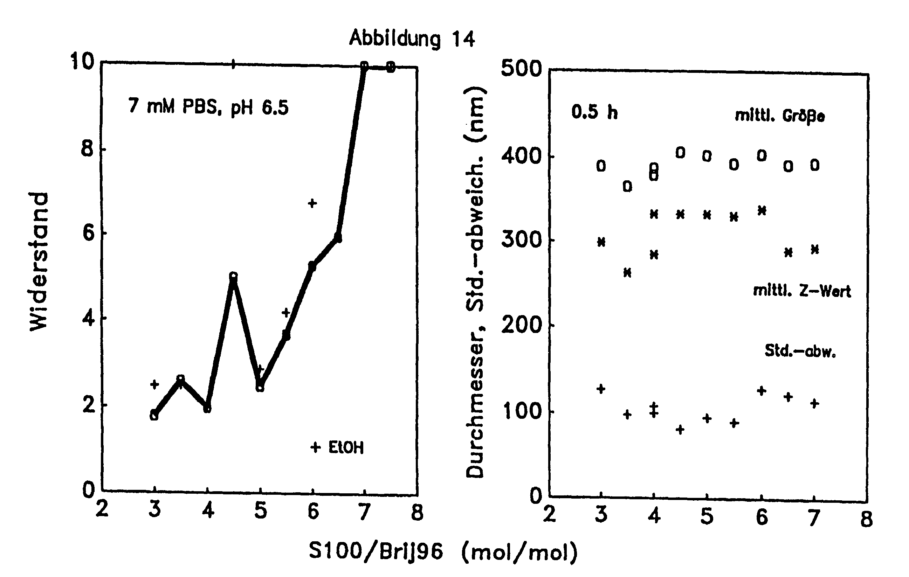

Für die Messung des Permeationswiderstandes wird die bereits beschriebene Methode verwendet. Die entsprechenden Werte sind in dem linken Teil der Abb. 14 als Kreise bzw. Kreuze (zwei unabhängige Versuchsreihen) gezeigt. Der Verlauf der Permeationsresistenz als Funktion des L/T Verhältnisses ist ähnlich wie im Falle von etwaigen Transfersomen und ist in der rechte Hälfte der Abb. 14 dargestellt. Maximale Permeationsfähigkeit wird erst unterhalb von L/T = 3 erreicht.

### Beispiele 216-235

### Zusammensetzung:

| | |
|---|---|
| 202,0-413 mg | Phosphatidylcholin aus Sojabohnen (+95%=PC) |
| 298,0-87,0 mg | Myrj 49 |
| 0,26-0,45 ml | Ethanol, absolut |
| 4,5 ml | Phosphatpuffer, pH 6,5, 10 mM |

### Herstellung und Charakterisierung:

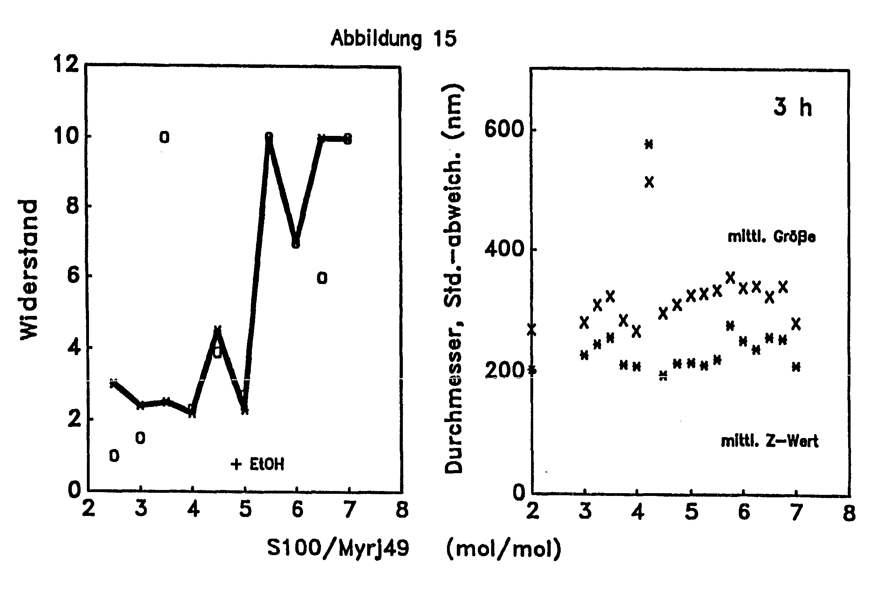

Die Transfersomen werden wie in Beispielen 201-215 beschrieben hergestellt und charakterisiert. Ihre Permeationseigenschaften in Abhängigkeit von der relativen Tensidkonzentration in den Proben ist in der linken Seite der Abb. 15 dargestellt. Die rechte Seite enthält die entsprechenden Gleichgewichtsdaten, die jedoch über die Vesikelfähigkeit zur Permeation und Wirkstoffübertragung keine Auskunft geben können.

### Beispiel 236:

### Zusammensetzung:

| | |
|---|---|
| 144,9 mg | Phosphatidylcholin aus Sojabohnen |
| 24,8 mg | Desoxycholat, Na-Salz |
| 1,45 ml | Actrapid HM 100 (145 I.U.) |
| 0,16 ml | Ethanol, absolut |

### Herstellung.

Beide Lipide werden in entsprechenden Mengen im Ethanol gelöst und mit handelsüblicher Insulinlösung versetzt. Nach 12 Stunden wird die grobe Trägersuspension durch Filtration feinzerteilt und homogenisiert. Der mittlere Vesikeldurchmesser beträgt 225 ± 61 nm. Die nominale Insulin-Konzentration ist 83 I.U. Auf den rechten Unterarm werden über eine Fläche von ca. 10 Quadratzentimeter 0,36 ml (30 I.U.) von Insulin Transfersomen verteilt. Die Blutproben werden alle 10 Minuten über einen heparinisierten Dauerkatheter aus einer Vene am rechten Unterarm entnommen; die ersten 0,5 ml werden jeweils verworfen; die anschließenden 0,5-0,8 ml von jeder Probe werden sedimentiert und sofort eingefroren; mit dem restlichen Volumen wird die Glucosekonzentration bestimmt.

### Wirkung:

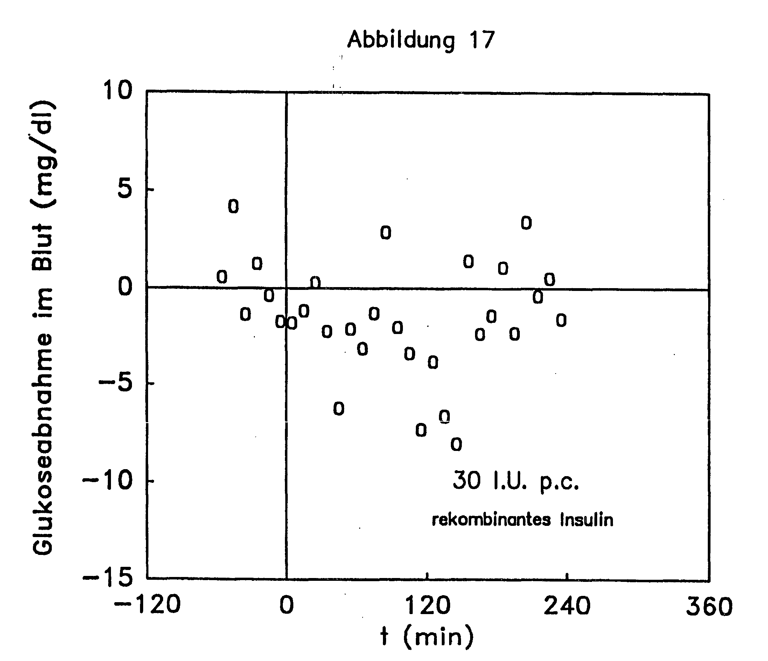

Diese tensidhaltigen Liposomen sind nur wenig im Stande, Insulin über die Haut zu tragen, wie aus Abbildung 17 ersichtlich ist. Je nach Wahl des auszuwertenden Bereiches beträgt die Senkung des Blutglucosespiegels, die sie vermitteln, zwischen 2 und 5 mg/dl für die Dauer von höchstens 30-40 Minuten. Mit vergleichbarer subkutaner Injektion wäre der Effekt um den Faktor von 50-200 höher. Detergensreiche Liposomen, die nicht hinsichtlich ihrer transfersomalen Eigenschaften optimiert sind, sind folglich als Träger für perkutane Applikation wenig geeignet. Der Tensidgehalt solcher Träger kann keine optimale Agenspermeation durch die Haut vermitteln.

Dies zeigt, daß erfindungsgemäße Präparate zwar auch dann (noch) wirksam sein können, selbst wenn sie hinsichtlich des Gehaltes an randaktiver Substanz nicht optimiert sind; die maximalen Vorteile der Erfindung werden aber nur erreicht, wenn der größtmögliche Permeationsfähigkeit gewährleistende Gehalt an randaktiver Substanz erfindungsgemäß ermittelt und eingehalten wird.

Die vorstehend an den Beispielen 166 und 236 schon gezeigte Möglichkeit, Antidiabetica und insbesondere Insulin nichtinvasiv zu applizieren, sofern dafür erfindungsgemäß optimierte Transfersomen eingesetzt werden, wird im folgenden eingehender untersucht.

Bestrebungen, antidiabetische Stoffe in den Körper zu bringen, ohne die übliche Injektionsnadel zu verwenden, bestehen schon lange (siehe z.B. die Übersicht von Lassmann-Vague, (Diabete. Metab. 14,728,1989). So wurde z.B. vorgeschlagen, implantierbare Vorratsbehälter (Wang, P.Y, Biomaterials 10, 197, 1989) oder Pumpen (Walter, H et al., Klin. Wochenschr. 67, 583, 1989) zu benutzen, eine Insulinlösung transnasal (Mishima et al., J. Pharmacobio.-Dynam. 12, 31, 1989), perocular (Chiou et al., J. Ocul. Pharmacol. 5, 81, 1989), peroral in einer Liposomensuspension (Rowland & Woodley, Biosc. Rep. 1, 345, 1981) oder transrectal zu applizieren; wenn die Insulinmoleküle durch die Haut eingebracht werden sollen, wurde die Agenslösung z.B. transcutan mittels Jetinjektion (Siddiqui & Chien, Crit. Rev. Ther. Drug. Carrier. Syst. 3, 195, 1987), mit Hilfe von kleinen Injektoren (Fisken, Lancet 1, 787, 1989), von elektrischen Feldern (Burnette & Ongpipattanakul, J. Pharm. Sci. 76, 765, 1987; Meyer, B.R et al., Amer. J. Med. Sci. 297, 321, 1989) bzw. von chemischen Additiva permeationsmäßig unterstützt.

Alle diese Verfahren haben aber kaum eine Erleichterung für den Diabeteskranken gebracht - vielleicht mit der Ausnahme der Jetinjektion, die jedoch nur eine verfeinerte, technisch sehr aufwendige Form der Injektion ist und daher wenig verbreitet. Der Alltag eines jeden insulinabhängigen Patienten beinhaltet weiterhin das tägliche Injizieren einer Insulinlösung unter die Haut bzw. in das Muskelgewebe (De Meijer, P. et al., Neth. J Med. 34, 210, 1989).

Lipide wurden bisher als Excipienten für die verzögerte Freisetzung von Insulinimplantaten diskutiert (Wang, P.Y Int. J Pharm. 54, 223, 1989) oder, in Form von Liposomen, als Vehikel für die perorale Applikation vorgeschlagen (Patel, 1970), ohne daß jedoch die Ergebnisse reproduzierbar wären (Biochem. Int. 16, 983, 1988). Weitere Arbeiten auf dem Gebiet der insulinhaltigen Liposomen befaßten sich mit methodologischen, nicht therapeutischen Fragen (Wiessner, J. H. und Hwang, K. J. Biochim. Biophys. Acta 689, 490 1982; Sarrach, D. Stud. Biophys. 100, 95, 1984; Sarrach, D. und Lachmann, U. Pharmazie 40, 642, 1985; Weingarten, C. et al. Int. J. Pharm. 26, 251, 1985; Sammins, M.C. et al., J. Pharm. Sci. 75, 838, 1986; Cervato, G. et al., Chem. Phys. Lipids 43, 135, 1987).

Erfindungsgemäß werden die schon vorstehend beschriebenen Transfersomen zur nichtinvasiven Verabreichung von Antidiabetica, insbesondere Insulin, eingesetzt, und zwar in für diesen Zwecke optimierter Ausbildung.

Vorteilhaft ist hierzu mindestens eine Trägersubstanz ein physiologisch verträgliches polares oder nichtpolares Lipid oder eine andere pharmakologisch unbedenkliche amphiphile Substanz; die geeigneten Moleküle sind dadurch gekennzeichnet, daß sie stabile wirkstofftragende Aggregate bilden. Die bevorzugte Aggregatform sind Lipidvesikel, die bevorzugte Membranstruktur ist eine Doppelschicht.

Vorteilhaft wird weiter vorgesehen, daß mindestens eine solche Substanz ein Lipid oder Lipoid aus biologischer Quelle oder ein entsprechendes synthetisches Lipid ist, bzw. eine Abwandlung solcher Lipide, zum Beispiel ein Glycerid, Glycerophospholipid, Sphingolipid, Isoprenoidlipid, Steroid, Sterin oder Sterol, ein schwefel- oder kohlehydrathaltiges Lipid, oder aber ein beliebiges anderes Lipid, das stabile Doppelschichten bildet, z.B. eine halbprotonierte fluide Fettsäure. So werden Lipide aus Ei, Sojabohne, Kokosnuß, Oliven, Saflor, Sonnenblumen, Leinsamen, Walfett, Nachtkerze oder Primel verwendet, mit natürlich belassenen oder, teilweise oder voll hydrogenierten (gehärteten), bzw. ausgetauschten Ketten. Besonders häufig finden die entsprechenden Phosphatidylcholine Anwendung; aber auch Phosphatidylethanolamine, Phosphatidylglycerole, Phosphatidylinositole, Phosphatidsäuren und Phosphatidylserine, sowie Sphingomyeline oder Sphingophospholipide, Glykosphingolipide (z.B. Cerebroside, Ceramidpolyhexoside, Sulfatide, Sphingoplasmalogene), Ganglioside oder andere Glycolipide sind für die Anwendung im Sinne dieser Erfindung gut geeignet. Von synthetischen Lipiden werden vorzugsweise die entsprechenden Dioleoyl-, Dilinoleyl-, Dilinolenyl-, Dilinolenoyl-, Diaracidonyl-, Dimyristoyl-, seltener Dipalmitoyl-, Distearoyl-, phospholipide oder die entsprechenden Sphingosinderivate, Glykolipide oder sonstige Diacyl- bzw. Dialkyl-Lipide verwendet; auch beliebige Kombinationen der erwähnten Substanzen sind geeignet.

Vorteilhaft ist die randaktive Substanz ein nichtionisches, ein zwitterionisches, ein anionisches oder ein kationisches Tensid. Sie kann einen Alkoholrest enthalten. Gerne werden langkettige Fettsäuren oder Fettalkohole, Alkyl-trimethylammonium-Salze, Alkylsulfat-Salze, Cholat-, Deoxycholat-, Glycodeoxycholat-, Taurodeoxycholat-Salze, Dodecyl-dimethylaminoxid, Decanoyl- oder Dodecanoyl-N-methylglucamid (MEGA 10, MEGA 12), N-Dodecyl-N,N-dimethylglycin, 3-(Hexadecyldimethylammonio)-propansulfonat, N-Hexadecylsulfobetain, Nonaethylenglykol-octylphenylether, Nonaethylendodecylether, Octaethylenglykol-isotridecyl-ether, Octaethylen-dodecylether, Polyethylenglykol-20-Sorbitan-Monolaurat (Tween 20), Polyethylenglykol-20-Sorbitan-Monooleat (Tween 80), Polyhydroxyethylen-cetylstearylether (Cetomacrogo, Cremophor O, Eumulgin, C 1000) Polyhydroxyethylen-4-laurylether (Brij 30), Polyhydroxyethylen-23-laurylether (Brij 35), Polyhydroxyethylen-8-stearat (Myrj 45, Cremophor AP), Polyhydroxyethylen-40-stearat (Myrj 52), Polyhydroxyethylen-100-stearat (Myrj 59), polyethoxyliertes Rizinußöl 40 (Cremophor EL), polyethoxyliertes hydriertes Rizinußöl, Sorbitan-monolaurat (Arlacel 20, Span 20), besonders bevorzugt Decanoyl- oder Dodecanoyl-N-methylglucamid, Lauryl- oder Oleoylsulfat-Salze, Natriumdeoxycholat, Natriumglycodeoxycholat, Natriumoleat, Natriumelaidat, Natriumlinoleat, Natriumlaurat, Nonaethylendodecylether, Polyethylenglykol-20-Sorbitan-Monooleat (Tween 80), Polyhydroxyethylen-23-Laurylether (Brij 35), Polyhydroxyethylen-40-Stearat (Myrj 52), Sorbitan-Monolaurat (Arlacel 20, Span 20), usw. verwendet.

Zu den geeignetsten Tensiden dieser Substanzklassen gehören: n-Tetradecyl(=Myristoyl)-glycero-phosphatidsäure, n-Hexadecyl(=Palmityl)-glycero-phosphatidsäure, n-Octadecyl(=Stearyl)-glycero-phosphatidsäure, n-Hexadecylen(=Palmitoleil)-glycero-phosphatidsäure, n-Octadecylen(=Oleil)-glycero-phosphatidsäure, n-Tetradecyl-glycerophosphoglycerol, n-Hexadecyl-glycero-phosphoglycerol, -n-Octadecyl-glycero-phosphoglycerol, n-Hexadecylenglycero-phosphoglycerol, n-Octadecylen-glycero-phosphoglycerol, n-Tetradecyl-glycero-phosphoserin, n-Hexadecylglycero-phosphoserin, -n-Octadecyl-glycero-phosphoserin, n-Hexadecylen-glycero-phosphoserin und n-Octadecylen-glycero-phosphoserin.

Die Gesamtkonzentration der Trägersubstanz beträgt zweckmäßig 0,1 bis 30 Gew.-%. Vorzugsweise beträgt diese Konzentration zwischen 0,1 und 15 %, besonders häufig zwischen 5 und 10 %.

Die Gesamtmenge des randaktiven Stoffes im System beträgt zweckmäßig 0,1 % bis 99 Mol-% der Menge, die für eine Solubilisierung der Träger erforderlich wäre. Häufig liegt das Optimum wirkstoffabhängig in einem Bereich zwischen 1 und 80 Mol.-%, bevorzugt zwischen 10 und 60 Mol.-%; besonders bevorzugt werden Werte zwischen 20 und 50 Mol.-%.

Die Wirkstoffkonzentration liegt für Insulin zumeinst bei 1 bis 500 I.U./ml; vorzugsweise liegt die Konzentration darunter zwischen 20 und 100 I.U./ml. Die Trägerkonzentration liegt dann vorzugsweise im Bereich von 0,1-20 Gew.-%, häufig zwischen 0,5 und 15 Gew.-%, besonders häufig zwischen 2,5 und 10 Gew.-%.

Für die Herstellung werden die Trägersubstanzen, insbesondere Lipide, entweder als solche oder gelöst in einem physiologisch verträglichen, mit Wasser mischbaren Lösungsmittel oder Lösungsvermittler mit einer polaren Lösung kombiniert und so die Trägerbildung eingeleitet.

Vorteilhaft ist, daß die polare Lösung die randaktiven Substanzen enthält. Diese können auch in den Lipiden bzw. deren Lösung enthalten sein.

Die Trägerbildung wird bevorzugt durch Einrühren, mittels Verdampfung aus einer Umkehrphase, durch ein Injektions- oder Dialyseverfahren, durch mechanische Einwirkung, z.B. durch Schütteln, Rühren, Homogenisieren, Ultrabeschallen, Reiben, Frieren bzw. Auftauen, durch Hoch- und Niedrigdruck-Filtration oder sonstige Energiezufuhr herbeigeführt.

Es kann vorteilhaft sein, wenn der Wirkstoffeinschluß nach der Trägerbildung erfolgt.

Bei der Herstellung der Transfersomen durch Filtration wird bevorzugt, daß das Filtermaterial eine Porengröße von 0,1 - 0,8 Mikrometer, insbesondere 0,15-0,3, und besonders bevorzugt 0,22 Mikrometer hat, wobei auch mehrere Filter hintereinander verwendet werden können.

Im Falle einer Transfersomenherstellung mittels Ultraschall werden vorzugsweise Energiedichten von 10-50 kW/Liter/Minute verwendet; in mechanischen Rührwerken sind z.B. typischerweise Umdrehungsbereiche von 1000 bis 5000 pro Minute für die Herstellung von Transfersomen gut geeignet: in Hochdruckhomogenisatoren gewährleisten Drucke von 300-900 Bar nach einer Passage ausreichende Transfersomenhomogenität und -qualität, wobei auch Suspensionen mit 20-30 % Lipid problemlos bearbeitet werden können.

Es ist oft zweckmäßig, die Transfersomen kurz vor der Anwendung aus einem Konzentrat oder Lyophilisat herzustellen.

Kryopreservantien, wie z.B. Oligosaccharide, erleichtern dabei die Transfersomenbildung aus dem Lyophylisat.

Übliche Wirk-, Hilfs-oder Zusatzstoffe, vorzugsweise Stabilisatoren, Konservierungsmittel, Konsistenzbildner, oder Marker können im Erfindungszusammenhang verwendet werden.

Die folgenden Beispiele veranschaulichen die Erfindung, ohne sie zu beschränken. Temperaturen sind in Grad Celsius, Trägergrößen in Nanometer, und sonstige Größen in üblichen SI Einheiten angegeben.

### Beispiel 237:

### Zusammensetzung:

| | |
|---|---|
| 120 mg | Phosphatidylcholin aus Sojabohnen (reiner als 95 %) |
| 20 mg | Natrium-Cholat p.a. (UD = 3,2) |
| 150 µl | Ethanol (96 %) |
| 1,45 ml | Actrapid HM 100 (rekombinantes Humaninsulin 100 I.U./ml) |

### Herstellung:

Die Herstellung erfolgt mit kleinen Abwandlungen wie im Beispiel 166 beschrieben. Der Unterschied besteht darin, daß das Lipid/Insulingemisch bereits einige Minuten nach der Zubereitung mittels einer 1 ml Einmalspritze durch einen 0,22 µm Polycarbonatfilter (Sartorius) handfiltriert wird. Das Endvolumen der Suspension beträgt 1,2 ml; das Lipid/Cholatverhältnis ist nominal 2,8/1, in der Membran ca. 2,4/1. Die Endkonzentration von Insulin entspricht 83 I.U./ml; der Vesikelradius beträgt einen Tag nach der Herstellung im Durchschnitt 94 nm; eine Woche danach 170 nm.

### Anwendung:

Anderthalb Stunden nach Versuchsbeginn werden 240 µl der frischen, sterilen Suspension von insulinhaltigen Transfersomen entnommen (20 I.U.). Diese werden auf die Innenfläche des rechten Unterarms eines männlichen, seit 18 Stunden nüchternen Probanden aufgetragen und zu ca. 0,7 mg/cm² gleichmäßig verteilt. 5 Minuten später ist die Haut makroskopisch trocken; 45 Minuten später ist keine Spur des Auftrages mehr zu sehen.

### Wirkung:

Durch einen i.v. Katheter im linken Unterarm werden in ungleichmäßigen Zeitabständen alle 15 bis 40 Minuten Blutproben entnommen. Die Blutglukosebestimmung erfolgt wie im Beispiel 166 beschrieben.

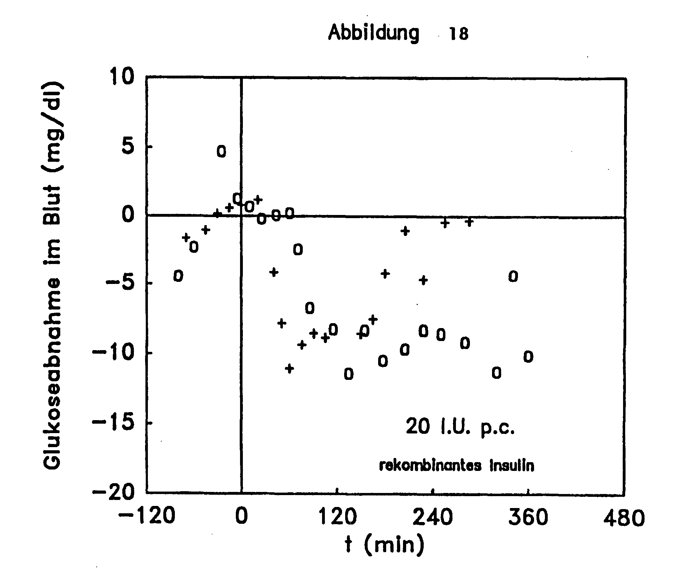

Der zeitliche Ablauf der transfersombedingten Hypoglykemie ist in der Abbildung 18 dargestellt. Der Blutglukosespiegel sinkt nach anderthalb Stunden um 10 mg/ml ab; diese künstliche Hypoglykemie dauert mindestens 4 Stunden an und erreicht somit 70-80 % des Wertes, der mittels herkömmlicher subkutaner Insulinapplikation mit dem Arzneimittel Actrapid erreicht wird. Die Kontrollergebnisse, bei einer solcher s.c. Insulinapplikation (von Transfersomen) erzielt, sind in diesem Bild als Kreuze dargestellt; die Gesamtwirkung entspricht dabei der für die freie Substanz erwarteten.

### Beispiel 238:

### Zusammensetzung:

| | |
|---|---|
| 216 mg | Phosphatidylcholin aus Sojabohnen (487 µl einer 50% Lösung in absolutem Äthanol) |
| 27 mg | Phosphatidylglycerol aus Ei (98 %) |
| 29.45 mg | Ölsäure, puriss. |
| 3 ml | Actrapid HM 100 (rekombinantes Humaninsulin 100 I.U./ml) |
| 40 µl | 1 N NaOH |
| 20 µl | 1 N NaCl |

### Herstellung:

Die Lipide werden gemischt, bis die Lösung klar erscheint. Nach der Zugabe von Actrapid-Lösung, Lauge und Salzlösung entsteht eine trübe Suspension. Nach dem Durchpressen dieser Suspension durch ein Polycarbonatfilter mit Porendurchmesser von 0,2 µm entsteht eine nur wenig opaleszente Suspension. Diese besteht aus Vesikeln(Transfersomen) mit einem mittleren Durchmesser von 320 nm.

### Anwendung:

Die Ausgangskonzentration von Glukose im Blut eines Probanden (70 kg, 37 Jahre, normoglykemisch, 24 Stunden nüchtern) wird über 90 Minuten als Referenz gemessen. Anschließend wird die oben beschriebene Transfersomen-Suspension mit nominal 85 I.U. Insulin/ml, die 12 Stunden bei 4°C gelagert wurde, auf den rechten Unterarm aufgetragen (ca. 330 µl auf 15 cm²); das entspricht einem Auftrag von 28 I.U.

### Wirkung:

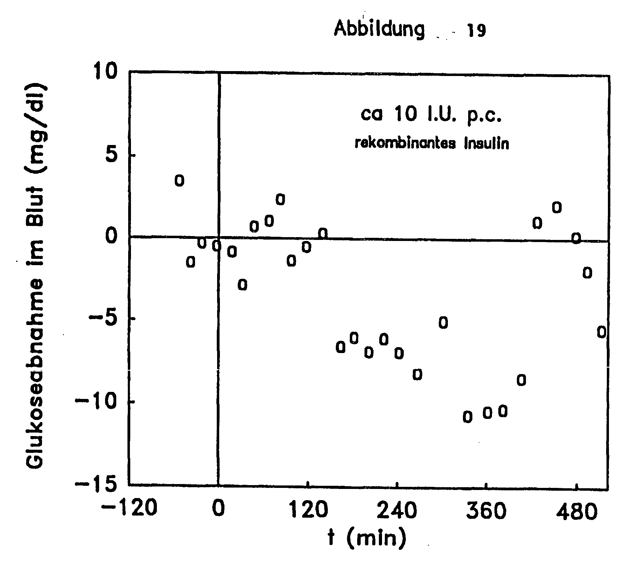

Die Blutproben werden über einen heparinisierten Dauerkatheter aus einer Vene am linken Unterarm entnommen; 0,5 ml von jeder Probe werden sedimentiert und sofort eingefroren; mit dem restlichen Volumen wird die Glucosekonzentration enzymatisch bestimmt. Diese Konzentration fällt nach ca. 2,5 Stunden um ungefähr 8 mg/dl ab und bleibt über 4,4 Stunden herabgesetzt. Das entspricht 75 % des maximal erreichbaren, im Kontrollversuch durch eine s.c. Injektion verursachten Effekts. Die Pharmakokinetik dieser Versuchsreihe ist in der Abb. 19 präsentiert.

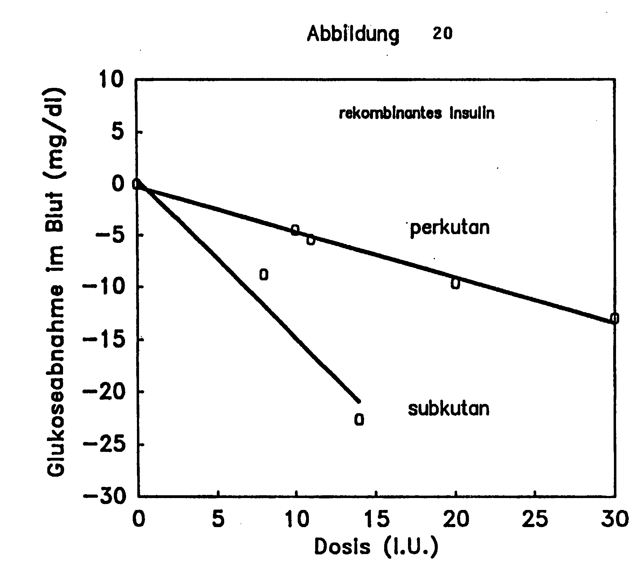

In Abb. 20 sind die Resultate von drei beispielsgemäßen perkutanen Insulinapplikationen mit Transfersomen und von zwei s.c. Injektionen zusammengefaßt.

### Beispiel 239:

### Zusammensetzung:

| | |
|---|---|
| 143 mg | Phosphatidylcholin aus Sojabohnen |
| 18 mg | Phosphatidylglycerol aus Ei (98 %) |
| 19,6 mg | Ölsäure, puriss. |
| 2 ml | Actrapid HM 100 (200 I.U.) |
| 25 µl | 1 N NaOH |

### Herstellung:

Die Lipide werden in ein Glasgefäß eingewogen und mit der handelsüblichen Insulinlösung versetzt. Die entstandene trübe Suspension wird direkt mit einer Titanspitze ultrabeschallt (ca. 5 W, 3x5 Sekunden bei 22°C mit jeweils 60 Sekunden Zeitabstand). Die resultierende, optisch klare Suspension enthält Vesikel mit einem mittleren Radius von 114 ± 17 nm.

### Anwendung und Wirkung:

Die Ergebnisse sind innerhalb der Meßgenauigkeit identisch mit den im Beispiel 238 angeführten.

### Beispiel 240:

### Zusammensetzung:

| | |
|---|---|
| 143 mg | Phosphatidylcholin aus Sojabohnen |
| 18 mg | Phosphatidylglycerol aus Ei (98 %) |
| 20,5 mg | Natrium-Oleat |
| 2 ml | Actrapid HM 100 (200 I.U.) |

### Herstellung:

Die Lipide werden in einem Glasgefäß in 0,15 ml abs. Ethanol aufgelöst und mit der handelsüblichen Insulinlösung versetzt. Ansonsten wird wie in Beispiel 239 verfahren.

### Anwendung und Wirkung:

Auf der Probanden-Unterarmhaut wird auf einer Fläche von ca. 5 cm² ein feines Kunststoffgewebe befestigt. Dieses wird anschließend mit 350 µl der insulinhaltigen Transfersomensuspension beschichtet und offen gelassen.

Die Senkung des Blutglucosespiegels beträgt nach 4 Stunden 7,8 mg/dl und nach 6 Stunden 8,5 mg/dl. Sie ist somit vergleichbar der im Beispiel 238 erzielten.

### Beispiel 241:

Es wird zunächst wie im Beispiel 238 verfahren, wobei jedoch die Zugabe von Kochsalzlösung ausgelassen wird; die trübe, unvorbehandelte Transfersomensuspension wird zweigeteilt. 50 % des Gesamtvoluments werden sterilfiltriert; der Rest wird 15 Sekunden lang bei Raumtemperatur mit 5 W beschallt. Der mittlere Vesikeldurchmesser in beiden Hälften ist ähnlich, 300 nm bzw. 240 nm.

### Beispiel 242:

Es wird wie in Beispielen 238 und 240 verfahren. Transfersomen werden jedoch einmal, zweimal, oder dreimal nacheinander filtriert. Die mittleren Durchmesser betragen 300, 240, und 200 nm.

Die Transfersomen gemäß Beispielen 241 und 242 lassen sich mit vergleichbarem Ergebnis gemäß Beispiel 238 anwenden.

### Beispiel 243:

### Zusammensetzung:

| | |
|---|---|
| 144,9;152 mg | Phosphatidylcholin aus Sojabohnen |
| 24,8;17,6 mg | Desoxycholat, Na-Salz |
| 1,45;1,55 ml | Actrapid HM 100 (145 I.U.) |
| 0,16 ml | Ethanol, absolut |

### Herstellung:

Die Lipide werden in Glasgefäße eingewogen, in Ethanol gelöst und mit der Insulinlösung versetzt. Die entstandene trübe Suspension wird über Nacht gealtert und anschließend nach 12 Stunden durch einen 0.22 Mikrometer Filter gepreßt. Die nominale Insulin-Konzentration beträgt 83 bzw. 84 I.U. Der mittlere Vesikelradius ist in beiden Fällen 112 nm.

### Anwendung und Wirkung:

Die allgemeinen Versuchsbedingungen sind wie in den Beispielen 237-239. Die Transfersomensuspensionen (0.36 ml, entspricht 30 I.U.) werden auf jeweils eine Arminnenseite aufgetragen; die Blutproben werden aus dem anderen Arm durch eine Dauerkanüle entnommen.

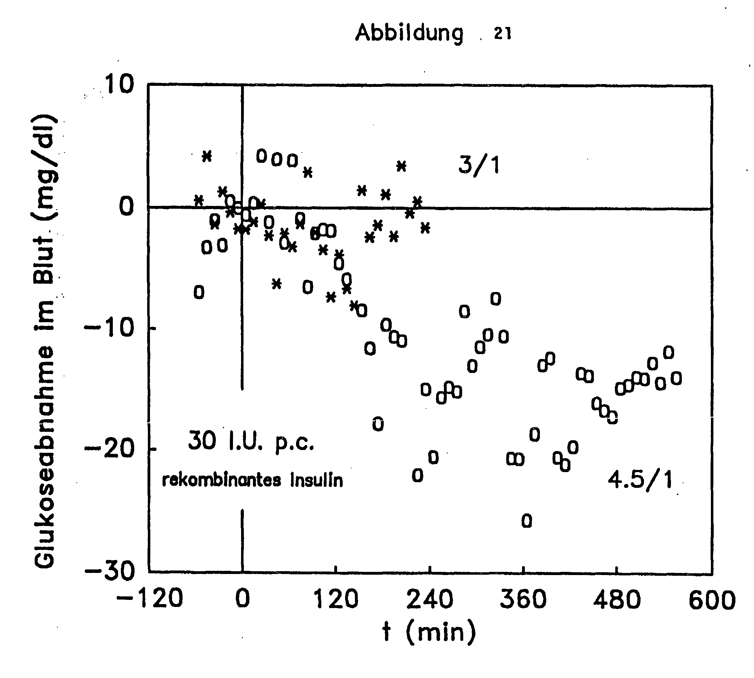

Die Ergebnisse sind in der Abbildung 21 dargestellt. Sie zeigen, daß die Präparation mit einem vergleichsweise hohen Tensidgehalt (Probe 1, L/T=3/1 ) lediglich eine kaum signifikante Senkung im Blutglucosespiegel bewirken kann; die beinahe optimalen Transfersomen dagegen, mit einem um 30% geringeren relativen Tensidgehalt von L/T=4.5/1 , erzeugen eine sehr ausgeprägte 'Hypoglykemie', die über viele Stunden Bestand hat.

Das ist ein weiterer Beweis dafür, daß die Transfersomen den Wirkstoff nach einem ganz anderen, neuen Wirkprinzip durch die Haut tragen als klassische Formulierungen.

Dieses Beispiel zeigt weiterhin, im Anschluß an Beispiel 236, daß im hier untersuchten System zwar auch solche Tensidgehalte verwendbar sind, die vom Optimum entfernt liegen, daß aber besonders vorteilhafte Ergebnisse erzielt werden, wenn der Tensidgehalt bestimmt und gewählt wird, der eine maximale Elastizität und damit Permeationsfähigheit der Transfersomen bei gleichzeitiger (noch) ausreichender Stabilität gegenüber Auflösung, Platzen, Werkstoffverlust usw. ergibt.

## Patentansprüche

1. Verwendung eines Präparats zum Transport von Wirkstoffen durch Permeabilitätebarrieren in Form von mit einer membranartigen Hülle aus einer oder wenigen Lagen amphiphiler Moleküle bzw. mit einer amphiphilen Trägersubstanz versehenen, Flüssigkeitströpfchen,
**dadurch gekennzeichnet, daß** das Präparat einen Gehalt einer randaktiven Substanz aufweist, der bis zu 99 Mol.-% des Gehaltes dieser Substanz entspricht, durch den der Solubilisierungspunkt der Tröpfchen erreicht wird, wobei der Gehalt dem am Solubilisierungspunkt liegenden Gehalt so nahe kommt, daß die Tröpfchen bei noch ausreichender Stabilität maximale Permeationsfähigkeit aufweisen.

2. Verwendung des Präparats nach Anspruch 1,
**dadurch gekennzeichnet, daß** der Gehalt wenigstens 0,1 Mol.-%, insbesondere zwischen 1 und 80 Mol.-%, vorzugsweise zwischen 10 und 60 Mol.-% und besonders bevorzugt zwischen 20 und 50 Mol.-% des die Solubilisierung bewirkenden Gehalts an randaktiver Substanz ausmacht, wobei die Randspannung im Tröpchen vorzugsweise bei etwa 10 Piconewton oder darunter liegt.

3. Verwendung des Präparats nach Anspruch 1 oder 2
**dadurch gekennzeichnet, daß** das Präparat einen Gehalt einer amphiphilen Substanz als Träger bzw. zur Bildung einer membranartigen Hülle um eine Tröpfchenmenge hydrophiler Flüssigkeit aufweimt, wobei der Wirkstoff in der Trägersubstanz, in der Hülle und/oder der Tröpfchenmenge enthalten ist.

4. Verwendung des Präparates nach Anspruch 3
**dadurch gekennzeichnet, daß** das Präparat als amphiphile Substanz eine lipidartige Substanz und als randaktive Substanz vorzugsweise ein Tensid aufweist.

5. Verwendung des Präparats nach einem der Ansprüche 1 bis 4,
**dadurch gekennzeichnet, daß** der Gehalt amphiphiler Substanz zur Applikation auf menschlicher und tierischer Haut zwischen 0,01 und 30 Gew.-% des Präparates, vorzugsweise zwischen 0,1 und 15 Gew.-% und besonders bevorzugt zwischen 5 und 10 Gew.-% beträgt.

6. Verwendung des Präparats nach einem der Ansprüche 1 bis 4,
**dadurch gekennzeichnet, daß** der Gehalt an amphiphiler Substanz zur Applikation bei Pflanzen 0,000001 bis 10 Gew.-%, vorzugsweise zwischen 0,001 und 1 Gew.-% und besonders bevorzugt zwischen 0,01 und 0,1 Gew.-% beträgt.

7. Verwendung des Präparats nach einem der vorstehenden Ansprüche,
**dadurch gekennzeichnet, daß** es als Wirkstoff ein Adrenocorticostaticum, β-Adrenolyticum, Androgen oder Antiandrogen, Antiparasiticum, Anabolicum, Anästheticum oder Analgesicum, Analepticum, Antiallergicum, Antiarrhythmicum, Antiartiroscleroticum, Antiasthmaticum und/oder Bronchospasmolyticum, Antibioticum, Antidrepressivum und/oder Antipsychoticum, Antidiabeticum, Antidotum, Antiemeticum, Antispilepticum, Antifibrinolyticum, Anticonvulsivum, Anticholinergicum, Enzym, Koenzym oder einen entsprechenden Inhibitor, ein Antihistaminicum, Antihypertonicum, einen biologischen Aktivitätsinhibitor, ein Antihypotonicum, Antikoagulans, Antimycoticum, Antimyasthenicum, einen Wirkstoff gegen morbus Parkinson, ein Antiphlogisticum, Antipyreticum, Antirheumaticum, Antisepticum, Atemanalepticum oder Atemstimulanz, Broncholyticum, Cardiotonicum, Chemotherapeuticum, einen Coronardilatator, ein Cytostaticum, Diureticum, einen Ganglienblocker, ein Glucocorticoid, Grippetherapeuticum, Hämostaticum, Hypnoticum, Immumglobulin bzw. -fragment oder eine andere immunologische Substanz, ein bioaktives Kohlehydrat(derivat), ein Kontrazeptivum, ein Migränemittel, ein Mineralcorticoid, einen Morphin-Antagonisten, ein Muskelrelaxans, Narcoticum, Neuraltherapeuticum, ein Nukleotid, Neurolepticum, einen Neurotransmitter oder entsprechenden Antagonisten, ein Peptid(derivat), ein Opthalmicum, (Para)-Sympaticomimeticum oder (Para) Sympathicolyticum, ein Protein(derivat), ein Psoriasis/Neurodermitismittel, Mydriaticum, Psychostimulanz, Rhinologicum, Schlafmittel oder dessen Antagonisten, ein Sedativum, Spasmolyticum, Tuberlostaticum, Urologicum, einen Vasoconstrictor oder -dilator, ein Virustaticum oder ein Wundenheilmittel oder mehrere solcher Agentien enthält.

8. Verwendung des Präparats nach einem der Ansprüche 1 bis 6,
**dadurch gekennzeichnet, daß** der Wirkstoff eine wachstumsbeeinflussende Substanz für Lebewesen ist.

9. Verwendung des Präparats nach einem der Ansprüche 1 bis 6,
**dadurch gekennzeichnet, daß** der Wirkstoff biozide Eigenschaften hat, insbesondere ein Insektizid, Pestizid, Herbizid oder Fungizid ist.

10. Verwendung des Präparats nach einem der Ansprüche 1 bis 6,
**dadurch gekennzeichnet, daß** der Wirkstoff ein Lockstoff, insbesondere ein Pheromon ist.

11. Verfahren zur Herstellung eines Präparats zum Transport von Wirkstoffen durch Permeabilitätsbarrieren in Form von mit einer membranartigen Hülle aus einer oder wenigen Lagen amphiphiler Moleküle bzw. mit einer amphiphilen Trägersubstanz versehenen, Flüssigkeitströpfchen,
**dadurch gekennzeichnet, daß** man den Gehalt an randaktiver Substanz bestimmt, bei dem die Tröpfchen solubiliziert werden und dem Präparat einen diesem Gehalt so nahekommenden Gehalt an randaktiver Substanz zusetzt, daß die Tröpfchen bei noch ausreichender Stabilität maximale Permeationsfähigkeit aufweisen.

12. Verfahren nach Anspruch 11,
**dadurch gekennzeichnet, daß** man Stabilität und Permeationsfähigkeit mittels Filtration , ggf, unter Druck, durch ein feinporiges Filter oder durch anderweitige kontrollierte mechanische Zerkleinerung bestimmt.

13. Verfahren nach Anspruch 11 oder 12, **dadurch gekennzeichnet, daß** der Gehalt an randaktiver Substanz zwischen 0,1 und 99 Mol.-%, insbesondere zwischen 1 und 80 Mol.-%, bevorzugt zwischen 10 und 60 Mol.-% und besonders bevorzugt zwischen 20 und 50 Mol.-% des Gehaltes ausmacht, bei dem der Solubilisierungspunkt der Tröpfchen erreicht wird.

14. Verfahren nach einem der Ansprüche 11 bis 13,
**dadurch gekennzeichnet, daß** das Substanzgemisch zur Erzeugung des Präparates einer Filtration, Ultraschallbehandlung, Rühren, Schütteln oder anderen mechanischen Zerteilungseinwirkungen ausgesetzt wird.

15. Verwendung des Präparats nach einem der Ansprüche 1 bis 10,
**dadurch gekennzeichnet, daß** das Präparat zur nichtinvasiven Verabreichung einen Gehalt an mindestens einem antidiabetischen Wirkstoff, besonders Insulin, aufweist.

16. Verwendung des Präparats nach Anspruch 15
**dadurch gekennzeichnet, daß** es als amphiphile Trägersubstanz ein physiologisch verträgliches polares oder nichtpolares Lipid enthält, wobei die Membranstruktur vorzugsweise eine Doppelschicht ist.

17. Verwendung des Präparats nach Anspruch 16,
**dadurch gekennzeichnet, daß** die amphiphile Substanz ein Lipid oder Lipoid biologischer Herkunft oder ein entsprechendes synthetischen Lipid ist, bzw. eine Abwandlung solcher Lipide, insbesondere ein Glycerid, Glycerophospholipid, Isoprenoidlipid, Sphingolipid, Steroid, Sterin oder Sterol, ein schwefel- oder kohlenhydrathaltiges Lipid, oder aber ein anderes Lipid, das stabile Doppelschichten bildet, vorzugsweise eine halbprotonierte fluide Fettsäure, insbesondere ein Phosphatidylcholin, Phosphatidylethanolamin, Phosphatidylglycerol, Phosphatidylinositol, eine Phosphatidsäure, ein Phosphatidylserin, ein Sphingomyelin oder Sphingophospholipid, Glykosphingolipid (z.B. Cerebrosid, Ceramidpolyhexosid, Sulfatid, Sphingoplasmalogen), Gangliosid oder anderes Glycolipid umfaßt oder ein synthetisches Lipid, vorzugsweise ein Dioleoyl-, Dilinoleyl-, Dilinolenyl-, Dilinolenoyl-, Diarachidoyl-, Dimyristoyl-, Dipalmitoyl-, Distearoyl, phospholipid oder entsprechendes Sphingosinderivat, Glykolipid oder anderes Diacyl- bzw. Dialkyl-Lipid umfaßt.

18. Verwendung des Präparats nach einem der Ansprüche 15 bis 17,
**dadurch gekennzeichnet, daß** es mehrere randaktive Substanzen umfaßt.

19. Verwendung des Präparates nach einem der Ansprüche 15 bis 18,
**dadurch gekennzeichnet, daß** die randaktive Substanz ein nichtionisches, ein zwitterionisches, ein anionisches oder ein kationisches Tensid umfaßt, insbesondere eine langkettige Fettsäure oder einen langkettigen Fettalkohol, ein Alkyl-trimethyl-ammonium-Salz, Alkylsulfat-Salz, Cholat-, Deoxycholat-, Glycodeoxycholat-, Taurodeoxycholat-Salz, Dodecyldimethyl-aminoxid, Decanoyl- oder Dodecanoyl-N-methylglucamid (MEGA 10, MEGA 12), N-Dodecyl-N,N-dimethylglycin, 3-(Hexadecyldimethylammonio)-propansulfonat, N-Hexadecyl-sulfobetain, Nonaethylen-glykoloctylphenylether, Nonaethylen-dodecylether, Octaethylenglykol-isotridecylether, Octaethylen-dodecylether, Polyethylenglykol-20-Sorbitan-Monolaurat (Tween 20), Polyethylenglykol-20-Sorbitan-Monocleat (Tween 80), Polyhydroxyethylen-Cetylstearylether (Cetomacrogo, Cremophor O, Eumulgin, C 1000) Polyhydroxyethylen-4-Laurylether (Brij 30), Polyhydroxyethylen-23-Laurylether (Brij 35), Polyhydroxyethylen-8-Stearat (Myrj 45, Cremophor AP), Polyhydroxyethylen-40-Stearat (Myrj 52), Polyhydroxyethylen-100-Stearat (Myrj 59), polyethoxyliertes Rizinußöl 40 (Cremophor EL), polyethoxyliertes hydriertes Rizinußöl, Sorbitan-Monolaurat (Arlacel 20, Span 20), besonders bevorzugt Decanoyl- oder Dodecanoyl-N-methylglucamid, Lauryl- oder Oleoylsulfat-Salze, Natriumdeoxycholat, Natriumglycodeoxycholat, Natriumoleat, Natriumelaidat, Natriumlinoleat, Natriumlaurat, Nonaethylen-dodecylether, Polyethylenglykol-20-Sorbitan-Monooleat (Tween 80), Polyhydroxyethylen-23-Laurylether (Brij 35), Polyhydroxyethylen-40-Stearat (Myrj 52) und/oder Sorbitan-Monolaurat (Arlacel 20, Span 20) und Lysophospholipide wie n-Octadecylen(≈Oleoyl)-glycerophosphatidsäure, -phosphorylglycerol, oder -phosphorylserin, n-Dilauryk-glycero-phosphatidsäure, -phosphorylglycerol, oder -phosphorylserin, n-Tetradecylglycero-phosphatidsäure, -phosphorylglycerol, oder -phosphorylserin und entsprechende Palmitoeloyl-, Elaidoyl-, Vaccenyl-Lysophospholipide.

20. Verwendung des Präparats nach einem der Ansprüche 15 bis 19,
**dadurch gekennzeichnet, daß** es als Wirkstoff 1 bis 500 I.U. Insulin/ml, vorzugsweise zwischen 20 und 100 I.U./ml enthält und die Konzentration der Trägersubstanz im Präparat im Bereich von 0,1 bis 20 Gew.-%, insbesondere zwischen 0,5 und 15 Gew.-%, besonders bevorzugt zwischen 2,5 und 10 Gew.-% beträgt.

21. Verwendung des Präparats nach einem der Ansprüche 15 bis 20,
**dadurch gekennzeichnet, daß** es als amphiphile Substanz Phosphatidylcholin und/oder Phosphatidylglykol und als randaktive Substanz eine Lysophosphatidsäure oder Lysophosphoglycerol, ein Deoxycholat-, Glycodeoxycholatoder Cholatsalz, ein Laurat, Myristat, Oleat, Palmitoleat, bzw- entsprechendes Phosphat- oder Sulfat-Salz, und/oder ein Tween- oder Myrj-Tensid sowie als Wirkstoff rekombinantes Humaninsulin enthält.

22. Verwendung des Präparats nach einem der Ansprüche 15 bis 21,
**dadurch gekennzeichnet, daß** der Vesikelradius der Präparat-Tröpfchen zwischen etwa 50 und etwa 200 nm, vorzugsweise zwischen etwa 100 und etwa 180 nm liegt.

23. Verfahren zur Herstellung eines Präparates gemäß Anspruch 1 zur nichtinvasiven Verabreichung von antidiabetischen Wirkstoffen, **dadurch gekennzeichnet, daß** man aus wenigstens einer amphiphilen substanz, wenigstens einer hydrophilen Flüssigkeit, wenigstens einer randaktiven Substanz und wenigstens einem antidiabetischen Wirkstoff liposomenartige Tröpfchen erzeugt, die das Präparat bilden.

24. Verfahren nach Anspruch 23,
**dadurch gekennzeichnet, daß** man separat jeweils die randaktive Substanz mit der amphiphilen Substanz und die hydrophile Substanz mit dem Wirkstoff vermischt und ggf. zur Lösung bringt, die Gemische bzw. Lösungen dann zu einer Mischung zusammenführt und in dieser durch Zufuhr von insbesondere mechanischer Energie die Tröpfchenbildung bewirkt.

25. Verfahren nach Anspruch 23 oder 24,
**dadurch gekennzeichnet, daß** die amphiphile Substanz entweder als solche oder gelöst in einem physiologisch verträglichen, mit hydrophilen Flüssigkeiten, insbesondere Wasser mischbaren Lösungsmittel oder Lösungsvermittler mit einer polaren Lösung zusammengegeben wird.

26. Verfahren nach Anspruch 25,
**dadurch gekennzeichnet, daß** die polare Lösung mindestens eine randaktive Substanz enthält.

27. Verfahren nach einem der Ansprüche 23 bis 26,
**dadurch gekennzeichnet, daß** die Tröpfchenbildung durch Einrühren, mittels Verdampfung aus einer Umkehrphase, durch ein Injektions- oder Dialyseverfahren, durch mechanische Beanspruchung wie Schütteln, Rühren, Homogenisieren, Ultrabeschallen, Reiben, Frieren bzw. Auftauen oder Hoch- oder Niedrigdruck-Filtration herbeigeführt wird.

28. Verfahren nach Anspruch 27,
**dadurch gekennzeichnet, daß** die Tröpfchenbildung durch Filtration bewirkt wird und das Filtermaterial eine Porengröße von 0,1 bis 0,8 µm, insbesondere 0,15 bis 0,3 µm und besonders bevorzugt 0,22 µm aufweist, wobei ggf. mehrere Filter hintereinandergeschaltet verwendet werden.

29. Verfahren nach einem der Ansprüche 23 bis 28,
**dadurch gekennzeichnet, daß** der Wirkstoffeinschluß wenigstens teilweise nach der Tröfchenbildung erfolgt.

30. Verfahren nach einem der Ansprüche 23 bis 29,
**dadurch gekennzeichnet, daß** die liposomenartigen Tröpfchen kurz vor der Anwendung aus einem Konzentrat oder Lyophilisat zubereitet werden.

## Claims

1. The use of a preparation for the transport of agents through permeability barriers in the form of minute droplets of a fluid with a membrane-like coating consisting of one or several layers of amphiphilic molecules or supplemented with an amphiphilic carrier substance, **characterized by** the fact that each preparation contains an edge active substance at a concentration that amounts up to 99 mole % of the concentration of this substance required to solubilize the droplet whereby the amount of this substance approaches the solubilization point to an extent that conveys to the droplet maximum permeation capability but simultaneously maintains its sufficient stability.

2. The use of a preparation according to claim 1, wherein the concentration of edge active substance amounts to at least 0.1 mol-%, in particular between 1 and 80 mol-%, preferably between 10 and 60 mol-%, and particularly preferred between 20 and 50 mol-% of the solubilization-inducing concentration of edge active substances, whereby the edge activity of a droplet unit is preferably close to approx. 10 Piconewton or less.

3. The use of a preparation according to claims 1 or 2, **characterized by** the fact that the preparation contains an amount of an amphiphilic substance as a carrier or as a basis for the membrane-like envelope of the droplet forming hydrophilic fluid, the agent being contained in the carrier substance, in the shell, and/or in the droplet material itself.

4. The use of a preparation as claimed in claim 3, wherein said amphiphilic substance is a lipid-like material and said edge active substance is preferably a surfactant.

5. The use of a preparation as claimed in one of claims 1 through 4, wherein the content of said amphiphilic substance for the applications on human or animal skin amounts to 0.01 through 30 weight-% of the preparation mass, preferably between 0.1 and 15 weight-% and particularly preferred between 5 and 10 weight-%.

6. The use of a preparation as claimed in one of claims 1 through 4, wherein the content of the amphiphilic substance in the formulation for application on plants is 0.000001 through 10 weight-%, preferably between 0.001 and 1 weight-% and particularly preferred between 0.01 and 0.1 weight-%.

7. The use of a preparation as claimed in any one of the preceding claims, wherein the agent is an adrenocorticostatic, a β-adrenolytic, an androgen or antiandrogen, antiparasitic, anabolic, anaesthetic or analgesic, analeptic, antiallergic, antiarrhythmic, antiarterosclerotic, antiasthmatic and/or bronchospasmolytic, antibiotic, antidrepressant and/or antipsychotic, antidiabetic, an antidote, antiemetic, antiepileptic, antifibrinolytic, anticonvulsive, an anticholinergic, an enzyme, coenzyme or a corresponding inhibitor, an antihistaminic, antihypertonic, a biological inhibitor of drug activity, an antihypotonic, anticoagulant, antimycotic, antimyasthenic, an agent against Morbus Parkinson, an antiphlogistic, antipyretic, antirheumatic, antiseptic, a respiratory analeptic or a respiratory stimulant, a broncholytic, cardiotonic, chemotherapeutic, a coronary dilatator, a cytostatic, a diuretic, a ganglium-blocker, a glucocorticoid, an antiflew agent, a haemostatic, hypnotic, an immunoglobuline or its fragment or any other immunologically active substance, a bioactive carbohydrate (derivative), a contraceptive, an antimigraine agent, a mineralcorticoid, a morphine-antagonist, a muscle relaxant, a narcotic, a neuraltherapeutic, a nucleotide, a neuroleptic, a neurotransmitter or some of its antagonists, a peptide(derivative), an opthalmic, (para)-sympaticomimetic or (para)sympathicolytic, a protein(derivative), a psoriasis/neurodermitis drug, a mydriatic, a psychostimulant, rhinologic, any sleep-inducing agent or its antagonist, a sedating agent, a spasmolytic, tuberlostatic, urologic, a vasoconstrictor or vasodilatator, a virustatic or any wound-healing substance, or several such agents.

8. The use of a preparation as claimed in one of claims 1 through 6, wherein said agent is a growth modulating substance for living organisms.

9. The use of a preparation as claimed in one of claims 1 through 6, wherein said agent exerts some biocidal activity and particularly is an insecticide, a pesticide, a herbicide or a fungicide.

10. The use of a preparation as claimed in one of claims 1 through 6, wherein an agent is an attractant, in particular from the class of pheromones.

11. A method for manufacturing preparations for the transport of agents through permeability barriers in the form of minute droplets of a fluid in a membrane-like 'envelope' consisting of one or a few layers of amphiphatic molecules, or supplemented with an amphiphilic carrier substance, **characterized by** the fact that the carrier solubilizing concentration of the edge active substance is determined and then an amount of this edge active substance is added to the formulation such that this amount comes as close to the solubilizing concentration as is needed to maximize the droplets permeation capability and maintain sufficient carrier stability.

12. Method as claimed in claim 11, wherein the stability and the permeation capacity of the fluid 'droplet' are determined by means of filtration, if required under pressure, through a fine-pore filter or by means of any other controlled mechanical fragmentation.

13. Method as claimed in claims 11 or 12, wherein the content of said edge active substance is between 0.1 and 99 mol-%, and in particular between 1 and 80 mol-%, preferably between 10 and 60 mol-% and most preferred between 20 and 50 mol-% of the concentration at which solubilization of the carrier is achieved.

14. Method as claimed in one of claims 11 through 13, wherein said mixture of substances required for the formation of a preparation is subjected to filtration, ultrasonication, stirring, agitating or any other mechanical fragmentation.

15. The use of a preparation as claimed in one of claims 1 through 10, wherein said preparation for non-invasive application contains at least one antidiabetic agent, in particular insulin.

16. The use of a preparation as claimed in claim 15, **characterized by** the fact that it contains a physiologically compatible polar or non-polar lipid as an amphiphilic carrier substance, the carrier membrane preferably having a double layer structure.

17. The use of a preparation as claimed in claim 16, wherein the amphiphilic substance is a lipid or a lipoid from any biological source or a corresponding synthetic lipid, or else comprises a modification of such lipids, a glyceride, in particular glycerophospholipid, isoprenoidlipid, sphingolipid, steroid, sterin or sterol, a sulfur- or carbohydrate-containing lipid, or else any other lipid which forms stable double layers, preferably a half-protonated liquid fatty acid, and preferably a phosphatidylcholine, phosphatidylethanolamine, phosphatidylglycerol, phosphatidylinositol, a phosphatidic acid, a phosphatidylserin, a sphingomyelin or sphingophospholipid, glycosphingolipid (e.g. cerebroside, ceramidepolyhexoside, sulfatide, sphingoplasmalogene), a ganglioside or any other glycolipid or a synthetic lipid, preferably a dioleoyl-, dilinoleyl-, dilinolenyl-, dilinolenoyl-, diarachidoyl-, dimyristoyl-, dipalmitoyl, distearoyl, phospholipid or corresponding sphingosinderivative, a glycolipid or any other diacyl- or dialkyl-lipid.

18. The use of a preparation as claimed in one of claims 15 through 17, containing several edge active substances.

19. The use of a preparation as claimed in one of claims 15 through 18, wherein said edge active substance is a nonionic, a zwitterionic, an anionic or a cationic surfactant, in particular a long-chain fatty acid or a long-chain fatty alcohol, an alkyl-trimethyl-ammonium-salt, alkylsulfate-salt, cholate-, deoxycholate-, glycodeoxycholate-, taurodeoxycholatesalt, dodecyl- dimethyl-aminoxide, decanoyl- or dodecanoyl-N-methylglucamide (MEGA 10, MEGA 12), N-dodecyl-N,N-dimethylglycine, 3-(hexadecyldimethylammonio)-propanesulfonate, N-hexadecyl-sulfobetaine, nonaethylene-glycoloctylphenylether, nonaethylenedodecylether, octaethyleneglycol-isotridecylether, octaethylenedodecylether, polyethylene glycol-20-sorbitane-monolaurate (Tween 20), polyethylene glycol-20-sorbitane-monooleate (Tween 80), polyhydroxyethylene-cetylstearyl ether (Cetomacrogo, Cremophor O, Eumulgin, C 1000) polyhydroxyethylene-4-laurylether (Brij 30), polyhydroxyethylene-23-laurylether (Brij 35), polyhydroxyethylene-8-stearate (Myrj 45, Cremophor AP), polyhydroxyethylene-40-stearate (Myrj 52), polyhydroxyethylene-100-stearate (Myrj 59), polyethoxylated castor oil 40 (Cremophor EL), polyethoxylated hydrated castor oil, sorbitanemonolaurate (Arlacel 20, Span 20), particularly preferred decanoyl- or dodecanoyl-N-methylglucamide, lauryl- or oleoylsulfate-salts, sodiumdeoxycholate, sodiumglycodeoxycholate, sodiumoleate, sodiumelaidate, sodiumlinoleate, sodiumlaurate, nonaethylene-dodecyl- ether, polyethylene glycol-20-sorbitane-monooleate (Tween 80), polyhydroxyethylene-23-laurylether (Brij 35), polyhydroxyethylene-40-stearate (Myrj 52) and/or sorbitanemonolaurate (Arlacel 20, Span 20) and lysophospholipid, such as n-octadecylen(=oleoyl)-glycero-phosphatidic acid, -phosphorylglycerol, or -phosphoryl-serine, n-dilauryl-glycerophosphatidic acid, -phosphorylglycerol, or -phosphorylserine, n-tetradecyl- glycero-phosphatidic acid, -phosphorylglycerol, or -phosphorylserine and corresponding palmitoleoyl-, elaidoyl-, vaccenyl-lysophospholipids.

20. The use of a preparation as claimed in one of claims 15 through 19, **characterized by** the fact that it contains 1 through 500 I.U. insulin/ml as agent, preferably between 20 and 100 I.U. insulin/ml and the concentration of the carrier substance in the preparation is in the range of 0.1 through 20 weight-%, in particular between 0.5 and 15 weight-%, particularly preferred between 2.5 and 10 weight-%.

21. The use of a preparation as claimed in one of claims 15 through 20, **characterized by** the fact that a phosphatidylcholine and/or a phosphatidylglycol is used as an amphiphilic substance, and that a lysophosphatidic acid or lysophosphoglycerol, a deoxycholate-, glycodeoxycholate- or cholate salt, a laurate, myristate, oleate, palmitoleate, or a corresponding phosphate- or sulfate-salt, and/or a Tween- or a Myrj-surfactant is used as an edge active substance, recombinant human insulin being the preferred agent.

22. The use of a preparation as claimed in one of claims 15 through 21, wherein the radius of said vesicular droplets in a preparation is between approx. 50 and approx. 200 nm, preferably between approx. 100 and 180 nm.

23. A method for the manufacture of a preparation according to claim 1 for the noninvasive application of antidiabetic agents, wherein said liposome-like droplets are formed from at least one amphiphilic substance, at least one hydrophilic fluid, at least one edge active substance, and at least one antidiabetic agent which together form the preparation.

24. Method as claimed in claim 23, wherein the edge active substance and the amphiphilic substance, and the hydrophilic substance and the agent are separately mixed together and, if required, dissolved in a solution, the resulting mixtures or solutions then being combined as one mixture to induce the formation of carrier particles, particularly by action of mechanical energy.

25. Method as claimed in claims 23 or 24, wherein said amphiphilic substance is either used as such or dissolved in a physiologically compatible solvent which is very frequently miscible with hydrophilic fluids, in particular water, or in a solvation mediating agent together with a polar solution.

26. Method as claimed in claim 25, wherein the polar solution contains at least one edge active substance.

27. Method as claimed in one of claims 23 through 26, **characterized by** the fact that the formation of droplets is induced by substance addition into a fluid phase, evaporation from a reverse phase, using an injection- or dialysis procedure, with the aid of mechanical stress such as shaking, stirring, homogenizing, ultrasonication, shear, freezing and thawing, or high- or low-pressure filtration.

28. Method as claimed in claim 27, **characterized by the fact** that the formation of droplets is caused by filtration, the filtering material having pore diameters of 0.1 through 0.8 µm, in particular with 0.15 through 0.3 µm, especially preferred 0.22 µm, several filters being optionally used in a sequence.

29. Method as claimed in one of claims 23 through 28, wherein inclusion of said agent occurs at least partly after the droplet formation.

30. Method as claimed in one of claims 23 through 29, wherein liposome-like droplets are prepared just before their application from a suitable concentrate or a lyophylisate.

## Revendications

1. Utilisation d'une préparation pour transporter, à travers des barrières de perméabilité, des principes actifs se présentant sous la forme de gouttelettes minuscules comportant une enveloppe en forme de membrane constituée par une couche ou par quelques couches de molécules amphiphiles ou par une substance véhicule amphiphile, **caractérisée en ce que** la préparation a une teneur en substance à activité marginale pouvant atteindre un pourcentage molaire de 99 % de la teneur de cette substance, qui permet d'atteindre le point de solubilisation des gouttelettes, cette teneur se rapprochant suffisamment de la teneur correspondant au point de solubilisation pour que les gouttelettes présentent un pouvoir de pénétration maximal tout en conservant une stabilité suffisante.

2. Utilisation de la préparation selon la revendication 1, **caractérisée en ce que** la teneur, en pourcentage molaire, est égale au moins à 0,1 % et est en particulier comprise entre 1 et 80 %, de préférence entre 10 et 60 % et plus favorablement entre 20 et 50 %, de la teneur en substance à activité marginale qui produit la solubilisation, la contrainte marginale dans les gouttelettes étant, de préférence, égale ou inférieure à 10 pico-Newton.

3. Utilisation de la préparation selon l'une des revendications 1 ou 2, **caractérisée en ce que** la préparation contient une substance amphiphile, en tant que véhicule ou pour la formation d'une enveloppe en forme de membrane autour d'un ensemble de gouttelettes de liquide hydrophile, le principe actif se trouvant dans la substance véhicule, dans l'enveloppe et/ou dans l'ensemble de gouttelettes.

4. Utilisation de la préparation selon la revendication 3, **caractérisée en ce que** la préparation contient, en tant que substance amphiphile, une substance de type lipide et, en tant que substance à activité marginale, de préférence une substance tensio-active.

5. Utilisation de la préparation selon l'une quelconque des revendications 1 à 4, **caractérisée en ce que** la teneur en substance amphiphile pour application sur la peau humaine ou animale est comprise entre 0,01 et 30 % du poids de la préparation, de préférence entre 0,1 et 15 % et plus avantageusement entre 5 et 10 %.

6. Utilisation de la préparation selon l'une quelconque des revendications 1 à 4, **caractérisée en ce que** la teneur en substance amphiphile pour application sur les plantes est comprise, en pourcentage pondéral, entre 0,000001 et 10 %, de préférence entre 0,001 et 1 % et plus avantageusement entre 0,01 et 0,1 %.

7. Utilisation de la préparation selon l'une quelconque des revendications précédentes, **caractérisée en ce qu'**elle contient, en tant que principe actif, un adrénocorticostatique, un β-adrénolytique, un androgène ou un anti-androgène, un antiparasitaire, un anabolisant, un anesthésique ou analgésique, un analeptique, un antiallergique, un antiarythmique, un antiartériosclérotique, un antiasthmatique et/ou bronchospasmolytique, un antibiotique, un antidépresseur et/ou antipsychotique, un antidiabétique, un antidote, un antiémétique, un antiépileptique, un antifibrinolytique, un anticonvulsivant, un anti-cholinergique, une enzyme, une co-enzyme ou un inhibiteur correspondant, un antihistaminique, un antihypertonique, un inhibiteur d'activité biologique, un antihypotonique, un anticoagulant, un antimycotique, un antimyasthénique, une substance contre la maladie de Parkinson, un antiphlogistique, un antipyrétique, un antirhumatisant, un antiseptique, un analeptique respiratoire ou stimulant respiratoire, un broncholytique, un cardiotonique, un chémothérapeutique, un dilatateur coronarien, un cytostatique, un diurétique, un gangliobloqueur, un glucocorticoïde, un antigrippe, un hémostatique, un hypnotique, une immunoglobuline ou fragment d'immunoglobuline ou autre substance immunologique, un glucide bioactif (ou dérivé), un contraceptif, un antimigraineux, un minéralocorticoïde, des antagonistes morphiniques, un myorelaxant, un narcotique, un médicament neurologique, un nucléotide, un neuroleptique, un neurotransmetteur ou antagonistes correspondants, un peptide (ou dérivé), un ophtalmique, un (para)-sympaticomimétique ou (para-)sympaticolytique, une protéine (ou dérivé), une substance Psoriasis/Neurodermique, un mydriatique, un psychostimulant, un rhinologique, un somnifère ou ses antagonistes, un sédatif, un spasmolytique, un tuberlostatique, un urologique, un vasoconstricteur ou vasodilatateur, un virustatique ou une substance pour soigner les plaies, ou bien plusieurs de ces agents.

8. Utilisation de la préparation selon l'une quelconque des revendications 1 à 6, **caractérisée en ce que** le principe actif est une substance ayant une influence sur la croissance des êtres vivants.

9. Utilisation de la préparation selon l'une quelconque des revendications 1 à 6, **caractérisée en ce que** le principe actif a des propriétés biocides et est, en particulier, un insecticide, un pesticide, un herbicide ou un fongicide.

10. Utilisation de la préparation selon l'une quelconque des revendications 1 à 6, **caractérisée en ce que** le principe actif est une substance attirante, en particulier une phérormone.

11. Procédé pour la fabrication d'une préparation servant à transporter, à travers des barrières de perméabilité, des principes actifs se présentant sous la forme de gouttelettes minuscules comportant une enveloppe en forme de membrane constituée par une couche ou par quelques couches de molécules amphiphiles ou par une substance véhicule amphiphile, **caractérisé en ce que** l'on détermine la teneur en substance à activité marginale pour laquelle les gouttelettes se solubilisent et **en ce que** l'on ajoute à la préparation la quantité de substance à activité marginale permettant de se rapprocher suffisamment de cette teneur, de telle sorte que les gouttelettes présentent un pouvoir de pénétration maximal tout en conservant une stabilité suffisante.

12. Procédé selon la revendication 11, **caractérisée en ce que** l'on détermine la stabilité et le pouvoir de pénétration par filtration, le cas échéant sous pression, au moyen d'un filtre microporeux ou autre procédé mécanique de fragmentation contrôlée.

13. Procédé selon l'une ou l'autre des revendications 11 et 12, **caractérisée en ce que** la teneur en substance à activité marginale, en pourcentage molaire, est comprise entre 0,1 et 99 %, de préférence entre 1 et 80 %, plus favorablement entre 10 et 60 % et plus favorablement encore entre 20 et 50 % de la teneur qui permet d'atteindre le point de solubilisation des gouttelettes.

14. Procédé selon l'une quelconque des revendications 11 à 13, **caractérisée en ce que** le mélange de substances servant à fabriquer la préparation est soumis à une filtration, à un traitement aux ultrasons, à un brassage, à une agitation ou à d'autres actions mécaniques de fragmentation.

15. Utilisation de la préparation selon l'une quelconque des revendications 1 à 10, **caractérisée en ce que** la préparation, pour l'administration non invasive, contient une certaine teneur de substance antidiabétique, en particulier de l'insuline.

16. Utilisation de la préparation selon la revendication 15, **caractérisée en ce qu'**elle contient, en tant que substance véhicule amphiphile, un lipide polaire ou non polaire, physiologiquement compatible, la membrane ayant de préférence une structure à double couche.

17. Utilisation de la préparation selon la revendication 16, **caractérisée en ce que** la substance amphiphile peut être un lipide ou un lipoïde d'origine biologique ou bien un lipide de synthèse correspondant ou bien une substance obtenue par modification de ces lipides, en particulier un glycéride, un glycérophospholipide, un isoprénoïdelipide, un sphingolipide, un stéroïde, une stérine ou stérol, un lipide contenant du soufre ou un glucide, ou bien tout autre lipide susceptible de former des doubles couches stables, de préférence des acides gras fluides à semi-protonation, en particulier une choline de phosphatidyle, une éthanolamine de phosphatidyle, un glycérol de phosphatidyle, un inositol de phosphatidyle, un acide de phosphatide, une sérine de phosphatidyle, une sphingomyéline ou un sphingophospholipide, un glucosphingolipide (par exemple, un cérébroside, un céramidpolyhexoside, un sulfatide, un sphingoplasmalogène), un ganglioside ou autre glucolipide, ou bien un lipide de synthèse, de préférence un dioleoyl-, un dilinoleyl-, un dilinolényl-, un dilinolénoyl-, un diarachidoyl-, un dimyristoyl-, un dipalmitoyl-, un distéaroyl, un phospholipide ou un dérivé correspondant de sphingosine, un glucolipide ou autre lipide de diacyle ou de dialkyle.

18. Utilisation de la préparation selon l'une quelconque des revendications 15 à 17, **caractérisée en ce qu'**elle comprend plusieurs substances à activité marginale.

19. Utilisation de la préparation selon l'une quelconque des revendications 15 à 18, **caractérisée en ce que** la substance à activité marginale peut être une substance tensio-active non ionique, zwitterionique, anionique ou cationique, en particulier un acide gras à chaîne longue ou un alcool gras à chaîne longue, un sel d'alkyle-triméthyle-ammonium, un sel de sulfate d'alkyle, un sel de cholate, de déoxycholate, de glycodéoxycholate ou de taurodéoxycholate, ou un aminoxyde de dodécyle-diméthyle, un N-méthylglucamide de décanoyl ou de dodécanoyl (MEGA 10, MEGA 12), une glycine de N-dodécyle-N,N-diméthyle, un sulfonate de 3-(hexadécyldiméthylammonio)-propane, une sulfobétaïne de N-hexadécyle, un glycol-octylphényléther de nonaéthylène, un dodécyléther de nonaéthylène, un isotridécyléther d'octaéthylèneglycol, un dodécyléther d'octaéthylène, un monolaurate de polyéthylèneglycol-20-sorbitane (Tween 20), un monooléate de polyéthylèneglycol-20-sorbitane (Tween 80), un cétylstéaryléther de polyhydroxyéthylène (Cetomacrogo, Cremophor 0, Eumulgin, C 1000), un 4-lauryléther de polyhydroxyéthylène (Brij 30), un 23-lauryléther de polyhydroxyéthylène (Brij 35), un 8-stéarate de polyhydroxyéthylène (Myrj 45, Cremophor AP), un 40-stéarate de polyhydroxyéthylène (Myrj 52), un 100-stéarate de polyhydroxyéthylène (Myrj 59), une huile de ricin 40 polyéthoxylée (Cremophor EL), une huile de ricin 40 hydratée polyéthoxylée, un monolaurate de sorbitane (Arlacel 20, Span 20), et plus avantageusement, un N-méthylglucamide de décanoyl ou de dodécanoyl, des sels de sulfate de lauryle ou d'oléoyl, un déoxycholate de sodium, un glycodéoxycholate de sodium, un oléate de sodium, un élaidate de sodium, un linoléate de sodium, un laurate de sodium, un dodécyléther de nonaéthylène, un monooléate de polyéthylèneglycol--20-sorbitane (Tween 80), un 23-lauryléther de polyhydroxyéthylène (Brij 35), un 40-stéarate de polyhydroxyéthylène (Myrj 52) et/ou un monolaurate de sorbitane (Arlacel 20, Span 20) et un lysophospholipide tel qu'un acide glycéro-phosphatide de n-octadécylène (= oléoyl), un phosphorylglycérol de n-octadécylène ou une phosphorylsérine de n-octadécylène, un acide glycéro-phosphatide de n-dilaurique, un phosphorylglycérol de n-dilaurique ou une phosphorylsérine de n-dilaurique, un acide glycéro-phosphatide de n-tétradécyle, un phosphorylglycérol de n-tétradécyle, une phosphorylsérine de n-tétradécyle et les lysophospholipides de palmitoéloyl, d'élaidoyl ou de vaccényle correspondants.

20. Utilisation de la préparation selon l'une quelconque des revendications 15 à 19, **caractérisée en ce qu'**elle contient, en tant que principe actif, une quantité d'insuline comprise entre 1 et 500 I.U./ml, de préférence entre 20 et 100 I.U./ml, et **en ce que** la concentration de la substance véhicule dans la préparation, en pourcentage pondéral, est comprise entre 0,1 et 20 %, de préférence entre 0,5 et 15 % et plus favorablement entre 2,5 et 10 %.

21. Utilisation de la préparation selon l'une quelconque des revendications 15 à 20, **caractérisée en ce qu'**elle contient, en tant que substance amphiphile, une choline de phosphatidyle et/ou un glycol de phosphatidyle et, en tant que substance à activité marginale, un acide de lysophosphatide ou un lysophosphoglycérol, un sel de déoxycholate, de glycodéoxycholate ou de cholate, un laurate, un myristate, un oléate, un palmitoléate ou un sel de phosphate ou de sulfate correspondant et/ou une substance tensio-active Tween ou Myrj et, en tant que principe actif, de l'insuline humaine recombinante.

22. Utilisation de la préparation selon l'une quelconque des revendications 15 à 21, **caractérisée en ce que** le rayon de l'enveloppe des gouttelettes de préparation se situe entre 50 nm environ et 200 nm environ, de préférence entre 100 nm environ et 180 nm environ.

23. Procédé pour la fabrication d'une préparation conformément à la revendication 1 pour l'administration non invasive de substances antidiabétiques, **caractérisé en ce que**, à partir d'au moins une substance amphiphile, d'au moins un liquide hydrophile, d'au moins une substance à activité marginale et d'au moins un principe actif antidiabétique, des gouttelettes de type liposome sont obtenues et constituent la préparation.

24. Procédé selon la revendication 23, **caractérisée en ce que** l'on mélange, d'une part la substance à activité marginale avec la substance amphiphile et d'autre part la substance hydrophile avec le principe actif, le cas échéant en les mettant en solution, les mélanges ou les solutions étant alors réunis pour former un mélange dans lequel les gouttelettes sont formées, en particulier par apport d'une énergie mécanique.

25. Procédé selon l'une ou l'autre des revendications 23 et 24, **caractérisé en ce que** la substance amphiphile est mélangée, en tant que telle ou en solution, dans un solvant ou un médiateur de dissolution physiologiquement compatible et miscible avec des liquides hydrophiles, en particulier avec l'eau, avec une solution polaire.

26. Procédé selon la revendication 25, **caractérisé en ce que** la solution polaire contient au moins une substance à activité marginale.

27. Procédé selon l'une quelconque des revendications 23 à 26, **caractérisé en ce que** la formation de gouttelettes est obtenue par brassage, par vaporisation à partir d'une phase réversible, par un procédé d'injection ou de dialyse, par une action mécanique telle qu'une agitation, un brassage, une homogénéisation, un traitement aux ultrasons, un frottement, une congélation, ou une condensation, ou bien par filtration à haute ou à basse pression.

28. Procédé selon la revendication 27, **caractérisé en ce que** la formation de gouttelettes est obtenue par filtration, le matériau filtrant ayant une taille de pore comprise entre 0,1 et 0,8 µm, en particulier entre 0,15 et 0,3 µm, et plus favorablement égale à 0,22 µm, plusieurs filtres pouvant éventuellement être disposés en série.

29. Procédé selon l'une quelconque des revendications 23 à 28, **caractérisé en ce que** l'apport en principe actif est effectué, au moins partiellement, après la formation des gouttelettes.

30. Procédé selon l'une quelconque des revendications 23 à 29, **caractérisé en ce que** les gouttelettes de type liposome sont préparées juste avant leur utilisation, à partir d'un produit concentré ou lyophilisé.
